# EUROPEAN PATENT APPLICATION

(11) **EP 2 192 197 A1**
(43) Date of publication of application: **02.06.2010**
(21) Application number: 08170119.5
(22) Date of filing: 27.11.2008
(51) Int. Cl.: C12Q 1/68

(54) **Predicting clinical response to treatment with a soluble tnf-antagonist or tnf, or a tnf receptor agonist**

(71) Applicant: Vereniging voor Christelijk Hoger Onderwijs, Wetenschappelijk Onderzoek en Patiëntenzorg, 1081 HV Amsterdam (NL)
(72) Inventor: The designation of the inventor has not yet been filed
(74) Representative: Habets, Winand

(57) **Abstract**

The invention relates to methods for predicting a clinical response to a therapy with a soluble TNF antagonist, TNF or a TNF receptor agonist and a kit for use in said methods.

## Description

The invention relates to methods for predicting a clinical response to a treatment with a soluble TNF antagonist, with TNF or a TNF receptor agonist using expression levels of genes of the Type I INF pathway and a kit for use in said methods. In another aspect, the invention relates to a method for evaluating a pharmacological effect of a treatment with a soluble TNF antagonist, TNF or a TNF receptor agonist.
Anti-tumour necrosis factor- (TNF-) therapies represent an important advancement in therapy for RA. However, there remains a proportion of patients who do not improve despite therapy. Although TNF antagonists have been shown to be highly efficient and safe for the treatment of RA patients, clinical complications have been reported for a subgroup of the treated patients. These include the reactivation of tuberculosis (Gomez-Reino A&R 48, 2003), the induction of anti-dsDNA autoantibodies (15;16) and development of symptoms of SLE (15). Increased anti-dsDNA autoantibodies have been observed in approximately 15% and SLE-like symptoms in about 0.2% of treated RA patients. The SLE symptoms are reversible upon discontinuation of anti-TNF treatment. These drugs are expensive and have the potential of serious toxicity. Therefore, it would be ideal to predict the patients who will respond, so that the use of these drugs can be targeted. Methods of predicting a TNF response known in the art are based on observational, habitual or demographic variables (age, sex, disease activity score (DAS28) or health assessment questionnaire (HAQ) scores (Hydrich et al. Rheum 2006: 1558-1565). There is a need is for improved methods using personalised biological parameters. Administration of TNF or a TNF receptor agonist has been implied as a therapy for Type I diabetes (Ban L, et al, PNAS 2008;105(36):13644-9). It is desirable to predict whether a patient will respond to a therapy with TNF or a receptor agonist.

The invention relates to a method for prognosticating a clinical response of a patient to a treatment with a soluble TNF-antagonist, TNF or TNF receptor agonist, said method comprising determining the level of an IFN-I type response in at least two samples of said individual, wherein a first of said samples has not been exposed to a soluble TNF-antagonist, TNF or TNF receptor agonist and wherein at least a second of said samples has been exposed to said soluble TNF-antagonist, TNF or TNF receptor agonist prior to determining said level, said method further comprising comparing said levels and prognosticating said clinical response from said comparison.

The term "patient" refers to any subject (preferably human) afflicted with a disease likely to benefit from a treatment with soluble TNF-antagonist, TNF or TNF receptor agonist, in particular a TNF -related disease. Two types of TNF related diseases exist. One type is a disease wherein high expression of TNF is a problem. In another type of a TNF related disease, low expression of TNF is a problem. Problems with high expression of TNF can be treated with a TNF antagonist. Problems with low expression of TNF can be treated with TNF or a TNF receptor agonist. In a preferred embodiment, said TNF-related disease comprises a disease which is likely to benefit from a treatment with a soluble TNF-antagonist. Preferably, said TNF -related disease comprises an autoimmune disorder, an infectious disease, transplant rejection or graft-versus-host disease, malignancy, a pulmonary disorder, an intestinal disorder, a cardiac disorder, sepsis, a spondyloarthropathy, a metabolic disorder, anemia, pain, a hepatic disorder, a skin disorder, a nail disorder, and vasculitis. In one embodiment, the autoimmune disorder is selected from the group consisting of rheumatoid arthritis, rheumatoid spondylitis, osteoarthritis, gouty arthritis, allergy, multiple sclerosis, autoimmune diabetes, autoimmune uveitis, and nephrotic syndrome. In another embodiment, said TNF -related disease is selected from the group consisting of inflammatory bone disorders, bone resorption disease, alcoholic hepatitis, viral hepatitis, fulminant hepatitis, coagulation disturbances, burns, reperfusion injury, keloid formation, scar tissue formation, pyrexia, periodontal disease, obesity, and radiation toxicity. In still another embodiment, said TNF -related disease is selected from the group consisting of Behcet's disease, ankylosing spondylitis, asthma, chronic obstructive pulmonary disorder (COPD), idiopathic pulmonary fibrosis (IPF), restenosis, diabetes, anemia, pain, a Crohn's disease-related disorder, juvenile rheumatoid arthritis (JRA), a hepatitis C virus infection, psoriatic arthritis, and chronic plaque psoriasis.

In one embodiment of the invention, the TNF related disease is Crohn's disease. In another embodiment, the disease is ulcerative colitis. In still another embodiment, the disease is psoriasis. In still another embodiment, the disease is psoriasis in combination with psoriatic arthritis (PsA).

In another preferred embodiment, said TNF-related disease comprises a disease which is likely to benefit from a treatment with TNF or TNF receptor agonist. Preferably, said disease comprises type I diabetes.

In a preferred embodiment, said patient is an individual suffering from at risk or suffering from Rheumatoid Arthritis (RA)" With the term an individual suffering from at risk or suffering from Rheumatoid Arthritis (RA) is meant an individual who is diagnosed with RA or is suspected by a doctor of suffering from RA or of developing the symptoms of RA within 10 years. Predominant symptoms of RA comprise pain, stiffness, and swelling of peripheral joints. The clinical manifestation of the disorder is very variable, ranging from mild, self-limiting arthritis to rapidly progressive multi-system inflammation with profound morbidity and mortality (Lee & Weinblatt 2001; Sweeney & Firestein 2004). RA symptoms may also comprise joint damage, which typically occurs early in the course of rheumatoid arthritis; 30 percent of patients have radiographic evidence of bony erosions at the time of diagnosis, and this proportion increases to 60 percent by two years (van der Heijde 1995; BrJ. Rheumatol., vol. 34 Suppl 2, pp. 74-78). Typically, RA is a polyarthritis, which involves many joints (six or more), although for example in the early stages of the disease, only one or a few joints might be afflicted. Virtually all peripheral joints can be affected by the disease; however, the most commonly involved joints are those of the hands, feet and knees (Smolen et al. 1995; Arthritis Rheum., vol. 38, no. 1, pp. 38-43). In addition, RA can affect the spine, and atlanto-axial joint involvement is common in longer- standing disease, and constitutes a directly joint-related cause of mortality. Extra-articular involvement is another hallmark of RA, and this can range from rheumatoid nodules to life-threatening vasculitis (Smolen & Steiner 2003; Nat.Rev.Drug Discov., vol. 2, no. 6, pp. 473-488). RA can be classified using history, physical examination, laboratory and radiographic findings and this is usually performed according to criteria as described in Arnett FC, et al.: Arthritis Rheum 31:315, 1988. Preferably, said individual has a DAS28 score of 3.2 or higher. More preferably, said DAS28 score is 4.6 or higher. Most preferably, said DAS28 score is 5.1 or higher.

Patients who are resistant to methotrexate (MTX), usually considered first-line therapy for the treatment of RA, are a further preferred group of patients for whom the method of the invention can be particularly useful.

More generally, patients who already receive a basic treatment for their TNF -related disease, e.g. with MTX, azathioprine or leflunomide, are particularly good candidates for the test method of the invention.

With the term "clinical response" is meant the clinical result of a treatment with a soluble TNF-antagonist, TNF or TNF receptor agonist. Said clinical response can be a positive or a negative clinical response. With a positive clinical response is meant that the severity of symptoms or the number of symptoms is reduced as a result of a treatment with said soluble TNF-antagonist, TNF or TNF receptor agonist. Preferably, said clinical result is the result of a treatment with a soluble TNF antagonist. When the disease is RA, it is preferred that a positive clinical response comprises at least reduction of swelling of joints. Preferably, an assessment of a clinical response is based on standardized and preferably validated clinical response criteria such as provided by the guidelines of organisations such as the National Institute for Health and Clinical Excellence (NICE), EULAR and/or ACR. Preferred clinical response criteria comprise DAS, DAS28 or the EULAR criteria or a combination thereof. In a preferred embodiment determination of a clinical response is based on an assessment using the DAS28 criteria. An advantage of the DAS28 is that it very sensitive to small effects of a therapy. Therefore, the method is very accurate when using DAS28 criteria.
Preferably, a positive clinical response is defined as a reduction in DAS28 score of at least 1.2 compared to the score of said individual prior to treatment with a soluble TNF antagonist. More preferably, a clinical response is based on assessment using EULAR criteria and DAS28 criteria.

Even more preferably, clinical response criteria are combined with demographic data, other clinical information or information about relevant habits. Demographic data comprise gender and/or age. Clinical information may comprise any relevant clinical observation or data. Preferred clinical information comprises CRP, ESR, ACPA titre, IgM RF titre, disease duration and medication. Information about relevant habits may be any relevant information. Preferred information comprises information about smoking habits.

The term "TNF" refers to Tumor Necrosis Factor, which is a naturally occurring cytokine, which plays a central role in the inflammatory response and in immune injury. TNF is also known as cachexin or cachectin and tumor necrosis factor-alpha. It is formed by the cleavage of a precursor transmembrane protein, forming soluble molecules which aggregate to form trimolecular complexes. These complexes then bind to receptors found on a variety of cells. Binding produces an array of pro-inflammatory effects, including release of other pro-inflammatory cytokines, including IL-6, IL-8, and IL-1; release of matrix metalloproteinases; and up regulation of the expression of endothelial adhesion molecules, further amplifying the inflammatory and immune cascade by attracting leukocytes into extravascular tissues.
The term "soluble TNF antagonist" refers to molecules, such as proteins or small molecules, which can significantly reduce TNF concentration.

Preferably said soluble TNF-antagonist comprises anti-TNF antibodies, e.g. infliximab, adalimumab, CDP571 or D2E7. Recombinant TNF-receptor based proteins have also been developed (e.g. etanercept, a recombinant fusion protein consisting of two soluble TNF receptors joined by the Fc fragment of a human IgG1 molecule). A pegylated soluble TNF type 1 receptor can also be used as a TNF blocking agent. Additionally, thalidomide has been demonstrated to be a potent soluble TNF antagonist. Soluble TNF antagonist thus further include phosphodiesterase 4 (IV) inhibitor thalidomide analogues and other phosphodiesterase IV inhibitors.

Preferred soluble TNF antagonists of the invention include, for example, infliximab, etanercept, certolizumab pegol (CDP870), golimumab and adalimumab. More preferably, said soluble TNF antagonist comprises infliximab.

With the term "sample" is meant any suitable sample comprising proteins or nucleotides. Preferred suitable samples include whole blood, saliva, faecal material, buccal smears, skin, and biopsies of specific organ tissues, such as muscle or nerve tissue and hair follicle, because these samples comprise relevant expression products. Preferably, said cell sample is a blood sample, because a blood sample is easy obtainable and comprises large amounts of relevant expression products.

With the term "IFN-I type response" is meant a response comprising the expression an expression product of a gene involved in the IFN-I pathway. With the level of an IFN-I type response is meant the amount of expression product of any gene involved in the IFN-I response pathway.

An "expression product" of a gene is RNA produced from said genes or a protein produced from said RNA. The levels of the expression products may be determined separately for each different expression product or as a single measurement for more different expression products simultaneously. Preferably, the determination of the level of the expression products is performed for each different expression product separately, resulting in a separate measurement of the level of the expression product for each different expression product. This enables a more accurate comparison of expression levels of expression products with the expression levels of the same expression products in a control.

Determination of the level of the expression products according to methods of the invention may comprise the measurement of the amount of nucleic acids or of proteins. In a preferred embodiment of the invention, determination of the level of the expression products comprises determination of the amount of RNA, preferably mRNA. A level can be the absolute level or a relative level compared to the level of another mRNA. mRNA can be isolated from the samples by methods well known to those skilled in the art as described, e.g., in Ausubel et al., Current Protocols in Molecular Biology, Vol. 1; pp.4.1.1 -4.2.9 and 4.5.1-4.5.3, John Wiley & Sons, Inc. (1996). Methods for detecting the amount of mRNA are well known in the art and include, but are not limited to, northern blotting, reverse transcription PCR, real time quantitative PCR and other hybridization methods. The amount of mRNA is preferably determined by contacting the mRNAs with at least one sequence-specific oligonucleotide which hybridises to said mRNA. In a preferred embodiment said mRNA is determined with two sequence-specific oligonucleotides which hybridise to different sections of said mRNA. The sequence-specific oligonucleotides are preferably of sufficient length to specifically hybridize only to the RNA or to a cDNA prepared from said mRNA. As used herein, the term "oligonucleotide" refers to a single-stranded nucleic acid. Generally the sequence-specific oligonucleotides will be at least 15 to 20 nucleotides in length, although in some cases longer probes of at least 20 to 25 nucleotides will be desirable. Said sequence-specific oligonucleotides may also comprise non-specific nucleic acids. Such non-specific nucleic acids can be used for structural purposes, for example as an anchor to immobilise the oligonucleotides. The sequence-specific oligonucleotide can be labelled with one or more labelling moieties to permit detection of the hybridized probe/target polynucleotide complexes. Labelling moieties can include compositions that can be detected by spectroscopic, biochemical, photochemical, bioelectronic, immunochemical, and electrical optical or chemical means. Examples of labelling moieties include, but are not limited to, radioisotopes, e.g., 32P, 33P, 35S, chemiluminescent compounds, labelled binding proteins, heavy metal atoms, spectroscopic markers such as fluorescent markers and dyes, linked enzymes, mass spectrometry tags, and magnetic labels. Oligonucleotide arrays for mRNA or expression monitoring can be prepared and used according to techniques which are well known to those skilled in the art as described, e.g., in Lockhart et al., Nature Biotechnology, Vol. 14, pp. 1675-1680 (1996); McGaII et al., Proc. Natl. Acad. ScI. USA, Vol. 93, pp. 13555-13460 (1996); and U.S. Patent No. 6,040,138.

A preferred method for determining the amount of mRNA involves hybridization of labelled mRNA to an ordered array of sequence-specific oligonucleotides. Such a method allows the simultaneously determination of the mRNA amounts. The sequence-specific oligonucleotides utilized in this hybridization method typically are bound to a solid support. Examples of solid supports include, but are not limited to, membranes, filters, slides, paper, nylon, wafers, fibers, magnetic or nonmagnetic beads, gels, tubing, polymers, polyvinyl chloride dishes, etc.

According to a preferred embodiment of the invention the determining the level(s) of the expression products is performed by measuring the amount of protein. The term "protein" as used herein may be used synonymously with the term "polypeptide" or may refer to, in addition, a complex of two or more polypeptides which may be linked by bonds other than peptide bonds, for example, such polypeptides making up the protein may be linked by disulfide bonds. The term "protein" may also comprehend a family of polypeptides having identical amino acid sequences but different post-translational modifications, particularly as may be added when such proteins are expressed in eukaryotic hosts. These proteins can be either in their native form or they may be immunologically detectable fragments of the proteins resulting, for example, from proteolytic breakdown. By "immunologically detectable" is meant that the protein fragments contain an epitope which is specifically recognized by e.g. mass spectrometry or antibody reagents as described below. Proteins levels can be determined by methods known to the skilled person, comprising but not limited to: mass spectrometry, Western blotting, immunoassays, protein expression assay, protein microarray etc.

A preferred embodiment of the invention provides a protein microarray (Templin et al. 2004; Comb.Chem.High Throughput Screen., vol. 7, no. 3, pp. 223-229) for simultaneous binding and quantification of the at least two biomarker proteins according to the invention. The protein microarray consists of molecules (capture agents) bound to a defined spot position on a support material. The array is then exposed to a complex protein sample. Capture agents such as antibodies are able to bind the protein of interest from the biological sample. The binding of the specific analyte proteins to the individual spots can then be monitored by quantifying the signal generated by each spot (MacBeath 2002; Nat. Genet, vol. 32 Suppl, pp. 526-532; Zhu & Snyder 2003; Curr.Opin.Chem.Biol., vol. 7, no. 1 , pp. 55-63). Protein microarrays can be classified into two major categories according to their applications. These are defined as protein expression microarrays, and protein function microarrays (Kodadek 2001 ; Chem.Biol., vol. 8, no. 2, pp. 105-115). Protein expression microarrays mainly serve as an analytic tool, and can be used to detect and quantify proteins, antigen or antibodies in a biological fluid or sample. Protein function microarrays on the other hand can be used to study protein-protein, enzyme-substrate and small molecule-protein interactions (Huang 2003; Front Biosci., vol. 8, p. d559-d576). Protein microarrays also come in many structural forms. These include two-dimensional microarrays constructed on a planar surface, and three-dimensional microarrays which use a Flow- through support.

Types of protein microarray set-ups: reverse phase arrays (RPAs) and forward phase arrays (FPAs) (Liotta et al. 2003; Cancer Cell, vol. 3, no. 4, pp. 317-325). In RPAs a small amount of a tissue or cell sample is immobilized on each array spot, such that an array is composed of different patient samples or cellular lysates. In the RPA format, each array is incubated with one detection protein (e.g., antibody), and a single analyte endpoint is measured and directly compared across multiple samples. In FPAs capture agents, usually an antibody or antigen, are immobilized onto the surface and act as a capture molecule. Each spot contains one type of immobilized antibody or capture protein. Each array is incubated with one test sample, and multiple analytes are measured at once.

One of the most common forms of FPAs is an antibody microarray. Antibody microarrays can be produced in two forms, either by a sandwich assay or by direct labelling approach. The sandwich assay approach utilizes two different antibodies that recognize two different epitopes on the target protein. One antibody is immobilized on a solid support and captures its target molecule from the biological sample. Using the appropriate detection system, the labelled second antibody detects the bound targets. The main advantage of the sandwich assay is its high specificity and sensitivity (Templin, Stoll, Bachmann, & Joos 2004; Comb.Chem.High Throughput.Screen., vol. 7, no. 3, pp. 223-229). High sensitivity is achieved by a dramatic reduction of background yielding a high signal-to noise ratio. In addition, only minimal amounts of labelled detection antibodies are applied in contrast to the direct labelling approach were a huge amount of labelled proteins are present in a sample. The sandwich immunoassay format can also be easily amenable to the field of microarray technology, and such immunoassays can be applied to the protein microarray format to quantify proteins in conditioned media and/or patient sera (Huang et al. 2001 ; Clin.Chem.Lab Med., vol. 39, no. 3, pp. 209-214; Schweitzer et al. 2002; Nat Biotechnol., vol. 20, no. 4, pp. 359-365).

In the direct labelling approach, all proteins in a sample are labelled with a fluorophore. Labelled proteins that bind to the protein microarray such as to an antibody microarray are then directly detected by fluorescence. An adaptation of the direct labelling approach is described by Haab and co-workers (Haab, Dunham, & Brown 2001; Genome Biol., vol. 2, no. 2, p). In this approach, proteins from two different biological samples are labelled with either Cy3 or Cy5 fluorophores. These two labelled samples are then equally mixed together and applied to an antibody microarray. This approach, for example, allows comparisons to be made between diseased and healthy, or treated and untreated samples. Direct labelling has several advantages, one of which is that the direct labelling method only requires one specific antibody to perform an assay.

Miniaturized and multiplexed immunoassays may also used to screen a biological sample for the presence or absence of proteins such as antibodies (Joos et al. 2000; Electrophoresis, vol. 21 , no. 13, pp. 2641-2650; Robinson et al. 2002; Nat.Med., vol. 8, no. 3, pp. 295-301).

In a preferred embodiment of the invention, the detection or capture agents such as the antibodies are immobilized on a solid support, such as for example on a polystyrene surface. In another preferred embodiment, the detection or capture agents are spotted or immobilized in duplicate, triplicate or quadruplicate onto the bottom of one well of a 96 well plate.

It is required that a first of said samples has not been exposed to a soluble TNF-antagonist, TNF or a TNF receptor agonist. Said first sample of said samples is used as a control sample. The level of an IFN-I type response of this is determined and compared to the level of an IFN-I type response of said at least a second of said samples from said individual. It is preferred that first sample of said samples and at least a second of said samples are of the same tissue.

It is required that said at least a second of said samples has been exposed to a soluble TNF-antagonist, TNF or TNF receptor agonist. A sample which is of an individual who had received said soluble TNF-antagonist, TNF or TNF receptor agonist as a treatment can be used or a cell sample from an untreated individual can be used wherein said cell sample has been provided with a soluble TNF-antagonist, TNF or TNF receptor agonist in vitro. It is preferred that all samples have been provided with the same soluble TNF-antagonist, TNF or TNF receptor agonist. However, it is possible to perform a method according to the invention wherein said at least a second of said samples has been provided with TNF or a TNF receptor agonist in vitro, whereas said first of said samples has been provided with a TNF antagonist. This method is also preferred. An advantage thereof is that an in vitro culture with TNF or a TNF agonist is technically easier to perform. When a cell sample is used from an individual who had been treated with a soluble TNF-antagonist, TNF or a TNF receptor agonist, it is preferred to use a sample that is collected at some time after said individual had been exposed to said soluble TNF-antagonist, TNF or TNF receptor agonist to allow said soluble TNF-antagonist, TNF or TNF receptor agonist to interact with said sample and to allow INF type I genes to respond to said soluble TNF-antagonist, TNF or TNF receptor agonist. Preferably, a cell sample is used which is collected between 1 and 4 months after the first exposure to said soluble TNF-antagonist, TNF or TNF receptor agonist. More preferably, a cell sample which is collected between at 2, 3 or 4 months after exposure is used, because at said time points, differences between good and poor responders are greater. Expression of genes involved in the type I IFN pathway reaches its peak between 1 and 2 months after starting a treatment with a soluble TNF-antagonist, TNF or TNF receptor agonist. It is preferred that at least two cell samples are collected between 1 and 4 months after exposure to soluble TNF-antagonist, TNF or TNF receptor agonist is used, because more samples from different time points increases the accuracy of the method. Most preferably, a cell sample collected at 1, 2, 3 and 4 months after exposure to soluble TNF-antagonist, TNF or TNF receptor agonist is used.

When using a cell sample from an individual who did not receive an a treatment with said soluble TNF-antagonist, TNF or TNF receptor agonist, said cell sample is preferably exposed to a soluble TNF-antagonist, TNF or TNF receptor agonist under in vitro conditions. For in-vitro culturing conditions, said cell sample is preferably a blood sample. Preferably, said conditions comprise culturing cells. Culturing procedures for different cell types are well known in the art and a skilled person will be able to select a suitable procedure for the selected cell types.

A method of the invention is also suited to prognosticate the clinical response of an individual to said soluble TNF-antagonist, TNF or TNF receptor agonist, prior to starting a treatment of said individual there upon. To this end said at least two samples are cultured in vitro, in the presence or absence of a soluble TNF-antagonist, TNF or TNF receptor agonist. It is understood that if the method is performed using in-vitro exposure of a sample, said first and said second samples may have been collected as a single sample which is split into a first and a second sample. An advantage thereof is that such method can be used to determine the prospect of a positive clinical response in individuals before the start of a TNF therapy. It is preferred that said TNF-antagonist, TNF or TNF receptor agonist is allowed to interact with said cells and to allow genes involved in the type I IFN pathway to respond to TNF-antagonist, TNF or TNF receptor agonist before measuring expression levels of said genes. Preferably, a preferred moment for measuring said expression levels is when the response of said genes is at its peak. A skilled person will be able to establish the most suitable moment to do this by culturing a cell sample taken from an individual prior to therapy with a TNF-antagonist, TNF or TNF receptor agonist. Preferably, culturing conditions comprise culturing in the presence of a TNF-antagonist, TNF or TNF receptor agonist for 24 to 48 hours. Preferably, said samples comprise blood cells. Preferably, said sample comprises whole blood.

In a method according to the invention, an individual has a decreased prospect of a positive clinical response to a treatment with a soluble TNF-antagonist if expression levels of the expression products of a said treatment induced IFN genes of said second sample are not significantly different from, or preferably higher compared to the levels of the same expression products of said first sample. A decreased prospect of a positive clinical response to a treatment with soluble TNF-antagonist is indicated if the in-vitro TNF induced expression of IFN response genes is not significantly different or is increased compared to levels of the same products of said first sample.

In the present invention we show that some RA patients display a significant treatment-induced increased expression of type I IFN-response genes. However, in another group of treated RA patients a rather stable expression of IFN-response genes is observed after Infliximab treatment. Especially RA patients that showed a poor clinical response to treatment showed an increase in IFN-response gene expression levels after treatment with a soluble TNF antagonist.

Pronounced differences in IFN levels were already present prior to treatment. Therefore it can not be excluded that the *in vivo* differences in IFN expression in treated *vs.* untreated SOJIA patients, as described by Palucka and co-workers, may be related to inter-individual differences rather than the result of treatment effect. In the current study the heterogeneous expression level of type I IFN-response genes before treatment was unrelated to its change after TNF blockade.

In patients suffering from Sjögren Syndrome (SS) an increase in type I IFN activity after three months of Etanercept treatment was previously demonstrated (19). While our results are partly in contrast with those published in SS this discrepancy may be explained by several different factors. Their read-out system for type I IFN activity is different from ours since they used an indirect reporter-cell assay to measure type I IFN activity in plasma while we measured transcription of type I IFN-response genes. Another important explanation may be that all SS patients fail to respond to TNF blockade while in RA a major part of the patients do respond to treatment. Indeed, our data showed that most RA patients with a treatment-induced increase in type I IFN-response gene expression levels have a poor clinical response to treatment.
The present invention confirms the existence of both regulatory routes since some RA patients actively increase and others decrease their type I IFN-response genes upon TNF blockade.

In summary, this study shows that there is a large variation in the change of IFN-response gene expression levels after TNF blockade between RA patients. Interestingly, treatment-induced upregulation of IFN-response genes is associated with a poor clinical response to Infliximab treatment. Monitoring of the IFN-response genes OAS1, LGALS3BP, Mx2, OAS2 and SERPING1 before and after the start of TNF blockade is useful for early determination of clinical response to treatment. Consequently, TNF or a TNF receptor agonist induces the opposite effect of that observed upon treatment with a soluble TNF antagonist. Hence, in-vitro TNF induced downregulation of IFN response genes or an expression of IFN response genes which is not significantly different is associated with a good clinical response to a soluble TNF-antagonist.

An expression level is classified as different when said expression level of said expression product of said first sample is statistically significantly increased or decreased in said individual compared to the level of the same expression product found in said first sample. The term "significantly" or "statistically significant" refers to statistical significance and generally means a two standard deviation (SD) above normal, or higher, or below, or lower concentration of the expression product. In preferred embodiments, said difference is classified as statistically significant if the expression level is at least a 20 percent increased or decreased compared to expression level of the same expression product in control individuals. Preferably, the increase or decrease is at least 20, 25, 30, 35, 40, 45, 50, 75, 100, 150, 200 or 250 percent. Most preferably, said increase or decrease is at least 100 percent.

More preferred is a method, wherein said IFN-I type response level is determined by determining in said at least two samples the level of an expression product of at least one gene of Table 2. An advantage thereof is that these genes are more predictive. More preferably, 2, 3, 4, 5, 6, 7, 8, 9, 10, 11, 12, 13, 14, 15, 20, 25, 30 or 34 genes of Table 2 are used, because the inclusion of more genes of this table improves the accuracy of the method.

More preferred is a method, wherein one level of said IFN-I type response is determined by determining the level of an expression product of at least OAS1, LGALS3BP, RSAD2, IFI44L, MX2, OAS2 and/or SERPING1. An advantage thereof is that these genes have a good predictive power.

In a preferred embodiment of the invention, said IFN-I type response is determined by determining the level of an expression product of at least OAS1 and LGALS3BP. An advantage thereof is that these use of these genes results in a good predictive power.

In another preferred embodiment of the invention, said IFN-I type response is determined by determining the level of an expression product of at least OAS1, RSAD2 and IFI44L. These genes have a good predictive power.

Even more preferred is an embodiment, wherein said IFN-I type response is determined by further determining the level of an expression product of at least MX2, OAS2 and/or SERPING1. An advantage thereof is that the combined use leads to an improved predictive power.
Another preferred embodiment is a method wherein said IFN-I type response is determined by determining the level of an expression product of at least OAS1, LGALS3BP, OAS1, RSAD2 and IFI44L. More preferably, said IFN-I type response is determined by determining the level of an expression product of at least OAS1, LGALS3BP, Mx2, OAS2 and SERPING1.

More preferably said IFN-I type response is determined by determining the level of an expression product of at least the 15 validation genes listed in Table 2. This further improves the predictive power of the method. Most preferred is a method wherein at least the 34 genes listed in Table 2 are used.
In another aspect, the invention relates to a method for prognosticating a clinical response of a patient to a treatment with a soluble TNF-antagonist, TNF or TNF receptor agonist, said method comprising determining the level of the expression products of the genes listed in Table 6 in at least two samples of said individual, wherein a first of said samples has not been exposed to a soluble TNF-antagonist, TNF or TNF receptor agonist and wherein at least a second of said samples has been exposed to a TNF-antagonist, TNF or TNF receptor agonist prior to determining said level, said method further comprising comparing said levels and prognosticating said clinical response from said comparison. Preferably, said soluble TNF-antagonist, TNF or TNF receptor agonist comprises a soluble TNF-antagonist.
In another aspect, the invention relates to a method for prognosticating a clinical reponse of a patient to a treatment with a soluble TNF-antagonist, TNF or TNF receptor agonist, said method comprising determining the level of the expression products at least one gene of Table 6 in at least two samples of said individual, wherein a first of said samples has not been exposed to a soluble TNF-antagonist, TNF or TNF receptor agonist and wherein at least a second of said samples has been exposed to TNF-antagonist, TNF or TNF receptor agonist prior to determining said level, said method further comprising comparing said levels and prognosticating said clinical reponse from said comparison.
More preferred is a method wherein said at least one gene comprises 2, 3, 4, 5, 6, 7, 8, 9, 10, 11, 12, 13, 14, 15, 16, 17, 18, 19, 20 or 21 genes of Table 6.

More preferred is a method wherein said at least two samples comprise cell samples. An advantage thereof is that cells samples comprise nucleic acids, which can advantageously be used for determining said levels of an IFN-I type response, said level of expression product of said genes and/or said at least one gene listed in Table 6.

More preferred is a method wherein second sample is of an individual between 1 and 4 months after the first exposure of said individual to said soluble TNF-antagonist, TNF or TNF receptor agonist. An advantage thereof is that within this period, said IFN-I type response level or said level of the expression products of Table 6 differs significantly compared to said first sample.

In another aspect the invention relates to a method for treatment of a patient with a soluble TNF-antagonist, TNF or a TNF receptor agonist, comprising determining a prognosis for a clinical response to a treatment with said soluble TNF-antagonist, TNF or TNF receptor agonist, further comprising treating said individual with said soluble TNF-antagonist, TNF or TNF receptor, if said individual has been prognosticated as a good responder.

In another aspect, the invention relates to use of a soluble TNF-antagonist, TNF or TNF receptor agonist for the preparation of a medicament for the treatment of a patient, wherein prior to said treatment a prognosis for a clinical response to said soluble TNF-antagonist, TNF or TNF receptor agonist was determined with any of the methods described above.

In another aspect, the invention relates to an improved pharmacodynamic marker (PD marker) for evaluating a pharmacological effect of a treatment with a TNF-antagonist, TNF or TNF receptor agonist. Good PD markers are needed to improve the prediction of the efficacy and safety of a treatment with a TNF-antagonist, TNF or TNF receptor agonist at the individual patient level. These quantitative PD markers should reflect features of drug exposure and drug response with respect to modulation of the molecular target, the cognate biochemical pathways and/or downstream biological effects. The availability of quantitative PD markers provides a rational basis for decision making during e.g. treatment optimization. A PD marker currently described for anti-TNF treatment is serum CRP levels (Elliott et al., Lancet 1994; 344(8930):1105-1110). Other PD markers include cytokine levels for IL-1RA and IL-6 which are described to be downregulated early after TNF blockade. The change in IL-6 levels is associated with CRP reduction (Elliott et al. J Immunol 1999; 163(3):1521-1528). However, CRP levels are not specifically increased in RA making its use as PD marker for anti-TNF treatment difficult. Moreover, most of the described PD markers are assessed by using mean levels of patient groups while most of these markers are not affected in each individual patient.

The invention provides a method for evaluating a pharmacological effect of a treatment of a patient with a TNF-antagonist, TNF or TNF receptor agonist said method comprising determining the level of an expression product of at least one gene of Table S2 in at least two samples of said individual, wherein a first of said samples has not been exposed to a TNF-antagonist, TNF or a TNF receptor agonist and wherein at least a second of said samples has been exposed to said TNF-antagonist, TNF or TNF receptor agonist prior to determining said level. The method is preferably used to determine whether the dose of a TNF-antagonist, TNF or a TNF receptor agonist which a patient receives is sufficiently high to achieve an effect or a clinical response. Whether a clinical response can be achieved depends also on other factors. The method can also be used to determine whether the dose of a TNF-antagonist, TNF or a TNF receptor agonist which a patient receives is not too high and might therefore cause side effects. With the term "pharmacological effect" is meant a biochemical or physiological effect of a TNF-antagonist, TNF or a TNF receptor agonist. Preferably, such pharmacological effect is specific for a treatment with a TNF-antagonist, TNF or a TNF receptor agonist. Preferably, such pharmacological effect reflects the relationship between an effective dose and the clinical response. Preferably, said effective dose is the dose as measured in the blood level. With the term "evaluating" is meant that results of a pharmacological effect is determined and used for decision making steps regarding further treatment. Preferably, evaluating comprises evaluating the dose, the efficacy and/or the safety of said TNF-antagonist, TNF or TNF receptor agonist. Preferably, the expression products of at least 2, 3, 4, 5, 6, 7, 8, 9, 10, 15, 25, 50, 100, 150, 200, 250, 300, 350 or 400 genes of Table S2 are used in the method. Other terms used are explained above. Preferably, the expression products of said genes comprises the genes, wherein the level of said expression product is higher than 1.5, 1.6, 1.7, 1.8, 1.9, 2.0, 2.1, 2.2, 2.4, 2.6 or 3.0, or lower than 0.68, 0.67, 0.66, 0.65, 0.64, 0.62 or 0.60 (see column "fold change", Table S2). Values higher than 1 in "fold change" as listed in table S2 are indicative of an upregulation after treatment. Upregulation of a gene having a "fold change" higher than 1 as listed in table S2 is indicative of an effective dose of a TNF-antagonist. Values lower than 1 in "fold change" in table S2 are indicative of a downregulation after treatment. Downregulation of a gene having a "fold change" lower than 1 is indicative of an effective dose of a TNF-antagonist. In another preferred embodiment, said at least one gene comprises preferably all of the following genes: P13 Peptidase inhibitor 3, skin-derived (SKALP); PLXNC1 , Plexin C1; JMJD1B Jumonji domain containing 1B; GFPT1, Glutamine-fructose-6-phosphate transaminase 1; MARCKS, Myristoylated alanine-rich protein kinase C substrate; F13A1, Coagulation factor XIII, A1 polypeptide; CENTD3, Centaurin, delta 3; CHI3L1, Chitinase 3-like 1 (cartilage glycoprotein-39); Cyclin-dependent kinase 5, regulatory subunit 1; CDK5R1 (p35); ABR, Active BCR-related gene and SMPDL3A alias Sphingomyelin phosphodiesterase, acid-like 3A. Said genes are upregulated after treatment. See Table S2 for further details on the mentioned genes. Preferred is a method wherein at least said second of said samples has been exposed to a TNF antagonist prior to determining said level Another preferred is a method wherein at least said second of said samples has been exposed to a TNF or TNF receptor agonist prior to determining said level.

Another preferred embodiment comprises the following genes: neutrophil cytosolic factor 1 [Macaca mulatta]; Immunoglobulin lambda locus; MGC29506, Hypothetical protein MGC29506; MCM5, Minichromosome maintenance complex component 5; SPTY2D1, SPT2, Suppressor of Ty, domain containing 1 (S. cerevisiae); InaD-like (Drosophila); IFITM3, Interferon induced transmembrane protein 3 (1-8U); RPSA, Ribosomal protein SA; EEF1G; Eukaryotic translation elongation factor 1 gamma; ZNF600, Zinc finger protein 600; RPLPO-like, Similar to ribosomal protein P0; HLA-C, Major histocompatibility complex, class I, C; IGJ, Immunoglobulin J polypeptide, linker protein for immunoglobulin alpha and mu polypeptides; RNASET2, Ribonuclease T2; RPS15A, Ribosomal protein S15a and CD14 alias CD14 molecule. Said genes are downregulated after treatment.

An advantage of this method is that said level of at least one gene of Table S2 reflects a specific response to a therapy with a TNF-antagonist, TNF or a TNF receptor agonist. Another advantage is that said response is present in all patients, unrelated to clinical response. Therefore, said response reflects drug activity and can be used to monitor drug efficacy at the individual patient level. Drug efficacy is the ability of a drug to produce the desired therapeutic effect.

In a preferred embodiment, said at least one gene comprises Peptidase Inhibitor 3 (PI/SKALP). An advantage of using said gene is that this gene is most significantly down-regulated after treatment with a TNF-antagonist.

In another aspect, the invention relates to a method for treatment of a patient with a TNF-antagonist, TNF or a TNF receptor agonist, wherein the dose of said TNF-antagonist, TNF or TNF receptor agonist of said treatment is based on results obtained by a method for evaluating a pharmacological effect of a treatment of a patient with a TNF-antagonist, TNF or a TNF receptor agonist said method comprising determining the level of a pharmacogenomic response of at least one gene of Table S2 in at least two samples of said individual, wherein a first of said samples has not been exposed to a TNF-antagonist, TNF or a TNF receptor agonist and wherein at least a second of said samples has been exposed to a TNF-antagonist, TNF or a TNF receptor agonist prior to determining said level. The term "based on" means that results of said method are taken into account in establishing the dose of said TNF-antagonist, TNF or TNF receptor agonist most suited for the individual patient. Preferred is a method wherein a patient is treated with a TNF-antagonist and wherein said method for evaluating a pharmacological response is based on results obtained by a method for evaluating a pharmacological effect wherein said at least a second of said samples has been exposed to TNF or a TNF receptor agonist prior to determining said level.

In another aspect, the invention relates to use of a TNF-antagonist, TNF or a TNF receptor agonist for the preparation of a medicament for the treatment of an patient, wherein said wherein said treatment is evaluated based on a method for evaluating a pharmacological effect of a treatment of a patient with a TNF-antagonist, TNF or a TNF receptor agonist said method comprising determining the level of a pharmacogenomic response of at least one gene of Table S2 in at least two samples of said individual, wherein a first of said samples has not been exposed to a TNF-antagonist, TNF or a TNF receptor agonist and wherein at least a second of said samples has been exposed to said TNF-antagonist, TNF or TNF receptor agonist prior to determining said level. Another preferred embodiment is the use of a TNF antagonist for the preparation of a medicament for the treatment of an patient and wherein said method for evaluating a pharmacological response is based on results obtained by a method for evaluating a pharmacological effect wherein said at least a second of said samples has been exposed to TNF or a TNF receptor agonist prior to determining said level.

In another aspect, the invention relates to a kit suitable for use in the above method, comprising up to 34 reagents, sequence specific oligonucleotides and/or capture agents for detecting up to 34 of the gene products of the genes listed in Table 2.

In another aspect, the invention relates to a kit suitable for use in the above method, comprising up to 21 reagents, sequence specific oligonucleotides and/or capture agents for detecting up to 21 of the gene products of the genes listed in Table 6.

### Figure legends

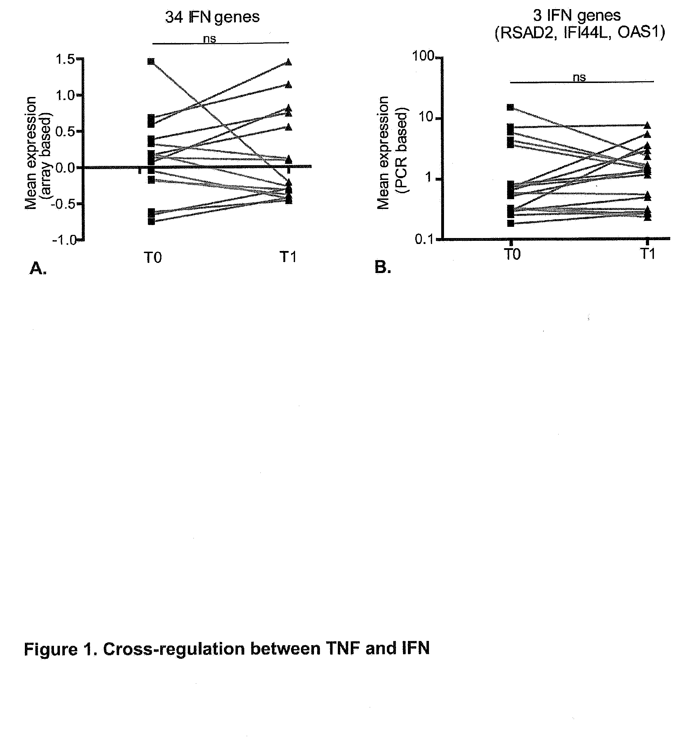

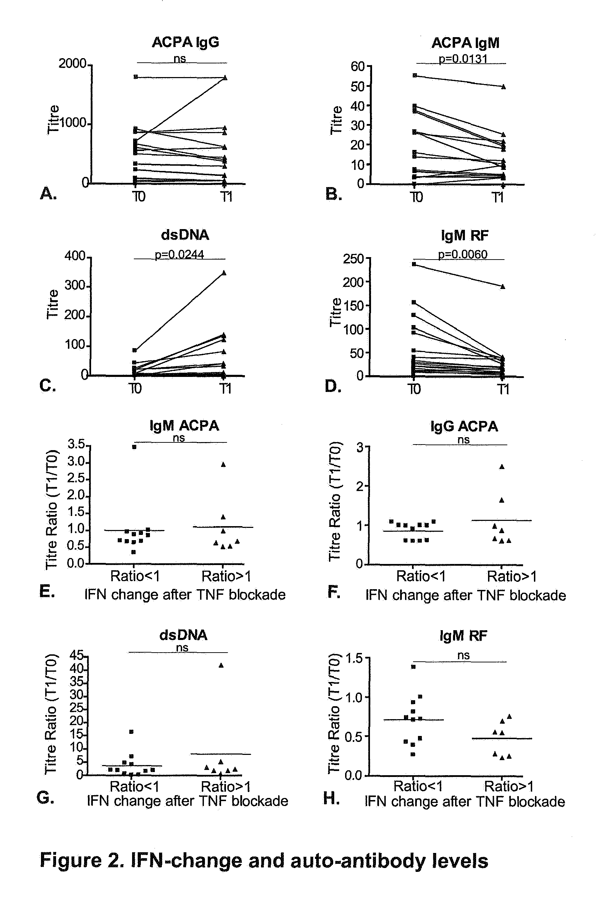

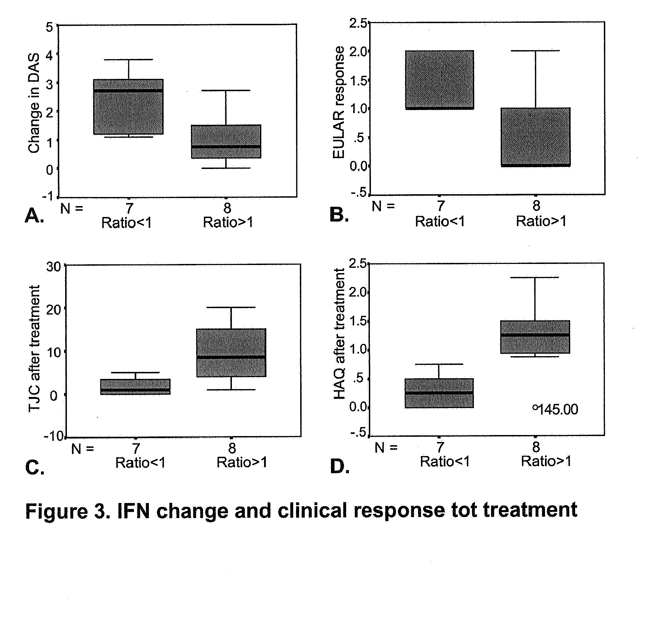

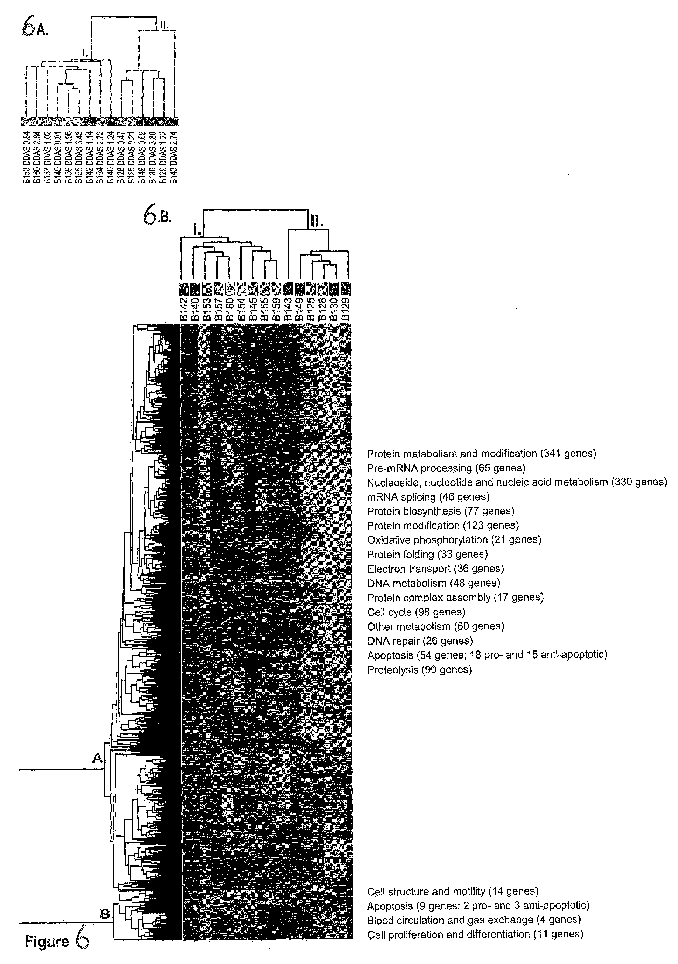

**Figure 1****. Differential cross regulation between TNF and IFN**
   The expression levels of 34 type I IFN-response genes were determined by cDNA microarray analysis in peripheral blood cells of 15 patients before (T0) and one month after (T1) anti-TNF treatment. Subsequently, for each patient the expression levels were averaged and baseline levels were compared to post-treatment levels (A). The patients whose IFN levels are induced after TNF blockade are indicated by red lines and patients with a downregulation by green lines. The expression levels of three (RSAD2, IFI44L and OAS1) IFN-response genes were measured by quantitative real-time PCR in an independent group of 18 patients. The expression levels of the three genes were averaged and baseline levels were compared to post-treatment levels (B). ns: not significant
**Figure 2****.** Relation between treatment-induced changes in IFN and autoantibody levels
   Figures A-D show the levels in ACPA IgG (A), ACPA IgM (B), dsDNA (C) and IgM RF (D) before and after treatment (n=18). Paired t-test was used to compare pre-treatment with post-treatment levels. In figures E-H the treatment-induced changes in autoantibody levels (IgM ACPA [E], IgG ACPA [F], dsDNA [G], IgM RF [H]) are compared between the two different response groups, stratified by increased or decreased mean expression levels of three IFN-response genes after treatment.
   ^{*} p<0.05; ns: not significant
**Figure 3****.** Relation between treatment-induced changes in IFN and clinical response
   Patients were divided into two groups based on their up- or downregulation of 34 IFN-response genes after treatment (as demonstrated in Figure 1A) and compared to each other with respect to clinical response to treatment. Patients with a downregulation in IFN-response genes displayed a significant (p<0.05) better clinical response to treatment as assessed by change in DAS (A), EULAR response (B), lower TJC (Tender Joint Count) (C) and lower HAQ (D) scores after treatment. RQ= Relative Quantity.

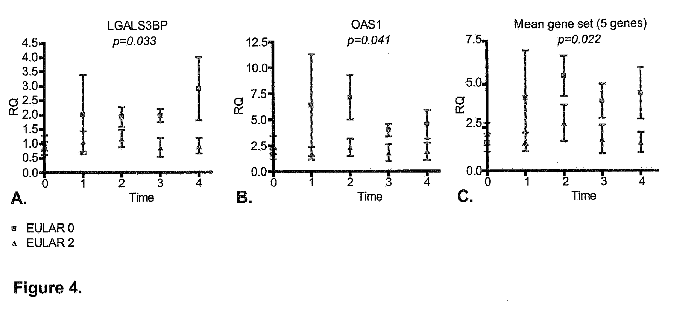

**Figure 4****.** Poor clinical response to TNF blockade is accompanied with upregulation of IFN
   For five EULARO poor responder (red colour) and five EULAR2 good responder (green colour) patients the expression levels of 15 IFN-response genes were measured by quantitative real-time PCR (Biomark) at baseline, one, two, three and four months after treatment. From two poor and one good responder patients the three months time points are missing. Expression levels for the two different clinical response groups were averaged and levels were compared using two-way ANOVA test. The change in expression levels of two genes, LGALS3BP (A) and OAS1 (B) were significantly different between the two clinical response groups. The combination of five genes (LGALS3BP, OAS1, Mx2, SERPING1 and OAS2) showed the best significant difference between the two clinical response groups.

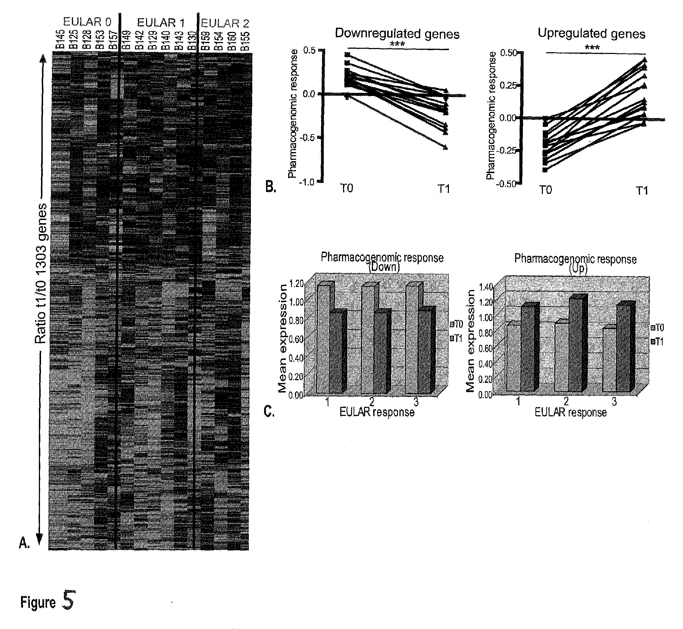

**Figure 5****.** Pharmacogenomic response to anti-TNF treatment
   One-way (supervised) hierarchical cluster analyses using the 1623 pharmacogenomic response genes whose expression levels were significantly (FDR<5%) changed after one month of TNF blockade. Genes were only included if both baseline and post-treatment data were present in at least 80% of the patients, resulting in a total selection of 1303 genes. Up-regulated genes after therapy are indicated by a red colour, down-regulated by a green colour and genes that show no differences in expression after therapy are indicated in black. Figure 1B shows the individual pre- *vs.* post-treatment comparison of the mean expression values of the pharmacogenomic response genes. Figure 1C shows that the extent of pharmacogenomic response as well as the baseline and post-treatment levels are not related to clinical response. *** p<0.001
**Figure 6****.** Inter-individual differences in treatment-induced changes in gene expression
   Gene expression ratio's (biological response) that differed at least two-fold after treatment in at least 4 patients were two-way hierarchically clustered. The resulting 440 genes divided the RA patients into two different patient groups (I and II) based on their difference in pharmacological response (A). SAM analysis revealed 2777 significantly (FDR<5%) differential changed genes between groups I and II. Cluster analysis was used to visualize these significant pharmacological differences in gene expression changes and PANTHER classification analysis was applied to determine the biological relevance of the pharmacological differences (B). Up-regulated genes after therapy are indicated by a red colour, down-regulated by a green colour and genes that show no differences in expression after therapy are indicated in black. At the top the clinical response is given in colours: Red, EULAR 0 poor response; blue, EULAR 1 moderate response; green, EULAR 2 good response.

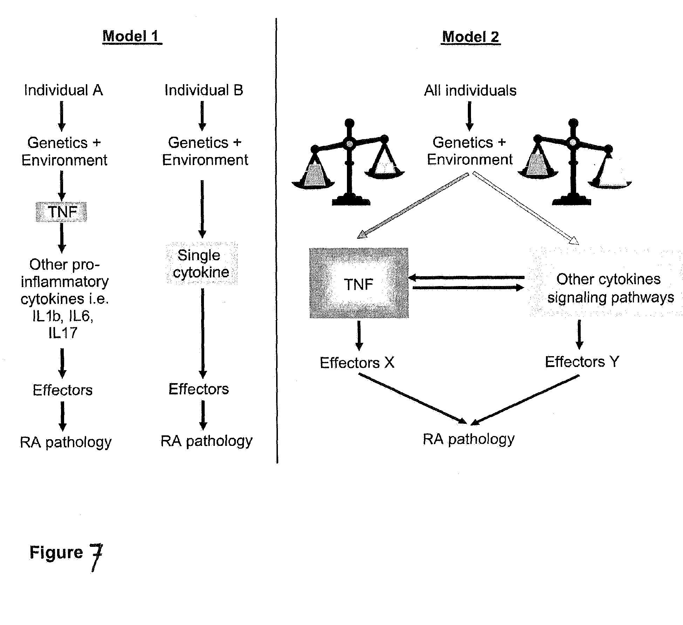

**Figure 7****.** Models describing the possible role of TNF in disease pathogenesis of RA
   Primary research has clearly revealed the importance of TNF in the pathogenesis of RA. Initially, TNF was presented as the most dominant pro-inflammatory cytokine driving downstream activation of other pro-inflammatory cytokines such as IL-6 and IL-1B. However, since not all RA patients showed a good response to TNF blockade these trials also indicated that the model (Figure 7; model 1, individual A) describing a TNF mediated pro-inflammatory signalling cascade leading to disease does not fit for all patients. Subsequent studies revealed significant roles for other cytokines as IL-18, RANKL, IL-17, IL-32, etc. in the pathogenesis of RA implying that in some patients other cytokines than TNF may play a dominant role (Figure 7; model 1, individual B). Because our pharmacogenomic data showed that all RA patients studied have an active TNF response profile prior to treatment which is affected by TNF blockade in all patients irrespective of clinical response, model 1 can be considered as invalid. Our data favour the existence of parallel cytokine networks operating simultaneously which facilitate the bypass of TNF neutralization (Figure 7; model 2).

### EXAMPLES

### Example 1: Predicting a clinical response in RA patients treated with Infliximab

### Materials & Methods

### Patients

Consecutive patients with RA according to the ACR criteria were enrolled in the study at the outpatient clinic of the Academic Medical Center (AMC) in Amsterdam over a period of one year. Inclusion criteria were: 18-85 years of age, a failure of at least two disease modifying anti-rheumatic drugs (DMARDs) including methotrexate (MTX), and active disease (DAS28 ≥ 3.2). Patients with a history of an acute inflammatory joint disease of different origin or previous use of a TNF blocking agent were excluded. Patients were on stable maximally tolerable MTX treatment. Whole blood samples (2.5 ml) were obtained using PAXgene tubes (PreAnalytix, GmbH, Germany) from 33 RA patients prior to initiation of anti-TNF therapy with Infliximab (3 mg/kg intravenously at baseline, at week 2, 6, and subsequently every 8 weeks). After 1, 2, 3 and 4 months of treatment another PAXgene tube was obtained. All patients gave written informed consent and the study protocol was approved by the Medical Ethics Committee (AMC). After 16 weeks of treatment the clinical response to treatment was assessed using both the EULAR criteria (24;25) as well as the reduction in DAS28 of at least 1.2 (26). An overview of the patients' characteristics is given in Table 1.

### Blood sampling for RNA isolation

2.5 ml blood was drawn in PAXgene blood RNA isolation tubes (PreAnalytix, GmbH, Germany) and stored at -20°C. Tubes were thawed for 2 hours at room temperature prior to RNA isolation. Next, total RNA was isolated using the PAXgene RNA isolation kit according to the manufacturer's instructions including a DNAse (Qiagen, Venlo, Netherlands) step to remove genomic DNA. Quantity and purity of the RNA was tested using the Nanodrop spectrophotometer (Nanodrop Technologies, Wilmington, Delaware USA).

### Microarray data

We used 43K cDNA microarrays from the Stanford Functional Genomics Facility (http://microarray.org/sfgf/) printed on aminosilane-coated slides containing ∼20.000 unique genes. Only one batch of arrays was used for all experiments. First DNA spots were UV-cross linked to the slide using 150-300 mJoules. Prior to sample hybridisation, slides were pre-hybridised at 42 degrees Celsius for 15 minutes in a solution containing 40% ultra-pure formamide (Invitrogen, Breda, Netherlands), 5% SSC (Biochemika, Sigma), 0.1% SDS (Fluka Chemie, GmbH, Switzerland) and 50 g/ml BSA (Panvera, Madison, USA). After pre-hybridisation slides were briefly rinsed in MilliQ water, thoroughly washed in boiling water and 95% ethanol and air-dried. Sample preparation and microarray hybridisation was performed as described previously (27) apart from the different post processing and pre-hybridisation described above.
Data storage and filtering was performed using the Stanford Microarray Database (28) (http://genome-www5.stanford.edu//) as described previously (29). Raw data can be downloaded from the publicly accessible Stanford database website.

### IFN-response gene set

Previously, we showed that a prominent cluster of highly correlated type I IFN-response genes were upregulated in a subgroup of biological-naïve RA patients compared to healthy controls (6). A gene set consisting of 34 type I IFN-response genes was obtained from this data. A smaller IFN gene set consisting of 15 genes was selected for validation analysis using Fluidigm's BioMark^{™} Real-Time PCR System. Detailed information of the gene lists is given in Table 2.

### Quantitative real-time PCR

RNA (0.5 µg) was reverse transcribed into cDNA using the Revertaid H-minus cDNA synthesis kit (MBI Fermentas, St. Leon-Rot, Germany) according to the manufacturer's instructions. Quantitative real-time PCR was performed using an ABI Prism 7900HT Sequence detection system (Applied Biosystems, Foster City, CA, USA) using SybrGreen (Applied Biosystems). Primers were designed using Primer Express software and guidelines (Applied Biosystems) and used primer sequences are listed in supplementary Table S1. To calculate arbitrary values of mRNA levels and to correct for differences in primer efficiencies a standard curve was constructed. Expression levels of target genes were expressed relative to 18SRNA.

### Fluidigm's BioMarh^{™} Real-Time PCR System

The Bi̊oMark^{™} 48.48 Dynamic Array (Fluidigm Corporation, South San Francisco, CA, USA) for real-time quantitati̊ve PCR was used to simultaneously measure the expression of 15 IFN-response genes (See Table 2) in 47 samples (plus 1 NTC) in triplicate. This experiment was performed at the outsourcing company ServiceXS (Leiden, Netherlands). Used pre-designed Taqman Gene Expression Assays are listed in supplementary Table S1. Expression levels of target genes were expressed relative to 18SRNA.

### Statistical analysis

Data was analyzed using software programs Graphpad Prism 4 (Graphpad Software, Inc., La Jolla, CA) and Statistical Package for Social Sciences version 14.0 (SPSS; Chicago, IL). First data was checked for normal (Gaussian) distribution. Two-group comparisons were analyzed using the independent sample T test, the Mann-Whitney U test or two-way ANOVA analysis where appropriate. Data were considered significant with p-values less than 0.05.

### Results

### Differential effect of TNF blockade on type I IFN signature

We studied the effect of TNF blockade on the transcription of type I IFN-response genes in peripheral blood cells of 15 RA patients before and one month after anti-TNF (Infliximab) treatment. Therefore, we determined the gene expression profiles of peripheral blood cells of 15 RA patients before and one month after anti-TNF (Infliximab) treatment. For each patient the expression levels of 34 IFN-response genes (Table 2) were averaged and baseline values were compared to post-treatment levels (Figure 1A). some RA patients showed a significant (*p*=0.0009) increased expression of the type I IFN-response genes after one month of TNF blockade. However, in other RA patients the expression levels of type I IFN-response genes were slightly decreased (*p*=0.08).
To confirm these results in an independent cohort of 18 patients by quantitative real-time PCR, genes were selected that showed the best correlation (R>0.9) with the mean expression value of the 34 type I IFN-response genes used in the previous analysis. This resulted in a smaller gene set of three genes (RSAD2, IFI44L and OAS1) and the mean expression levels of these genes were measured by quantitative real-time PCR for all 18 RA patients before and one month after Infliximab therapy. Again some patients showed a significant (p=0.002) increased mean expression of these three IFN-response genes after TNF blockade, whereas other patients displayed similar or decreased levels (Figure 1B). These results confirm the microarray results by showing the lack of consistent regulation between TNF and type I IFN genes. Collectively, these results demonstrate the heterogeneous response to TNF blockade with respect to changes in type I IFN-response gene expression levels.
The type I IFN-response gene expression levels were highly heterogeneous prior to treatment. We hypothesized that the observed differences in anti-TNF induced changes in IFN expression may be related to the IFN-response gene expression profile prior to treatment. To test this hypothesis the relationship between the baseline IFN expression levels and its change after TNF blockade was tested. In the 15 patients the mean expression of the 34 genes measured by microarray analysis the baseline levels did not correlate with their corresponding change after treatment (Pearson R=-0.42, p=0.12; data not shown). This was confirmed in the validation group of 18 patients using the mean expression levels of the three IFN-response genes (RSAD2, IFI44L and OAS1) measured by quantitative real-time PCR, although a trend towards significance was observed (Spearman R=-0.44, *p*=0.064; data not shown). These results evidently demonstrate that the cross regulation between TNF and IFN in RA is not as clear as previously described for SLE and SS. After one month of TNF blockade a subgroup of RA patients show significant increased expression of type I IFN-response genes whereas in another group the expression levels remain similar or slightly decrease. Moreover, the type I IFN gene expression profile prior to treatment is not associated with its change upon TNF blockade.

### No association between IFN induction and change in autoantibodies levels

Next, we investigated if there was a relation between the anti-TNF induced changes in type I IFN-response gene expression levels after one month of treatment and the subsequent production of autoantibodies. ACPA, RF and dsDNA-autoantibody levels, determined before and 24 weeks after TNF blockade, were available for 18 of the 33 patients studied. For each of these 18 patients the mean expression values of the three IFN-response genes (RSAD2, IFI44L and OAS1) was measured by quantitative real-time PCR were calculated before and one month after anti-TNF treatment. Patients were divided into two groups based on their treatment-induced change in type I IFN-response gene expression level in two groups, ratio>1 and ratio<1. The ratio is defined as the expression level of the IFN response genes 1 month post therapy divided by the the gene expression level before the treatment. Although some patients revealed an increase in anti-dsDNA antibodies levels after 24 weeks of treatment, we did not find an association between anti-dsDNA-autoantibody levels (at T=0 and T=1) or change in autoantibody levels (ratio T=1/T=0), with the patients who had an increased IFN response profile (Figure 2 and data not shown). In addition, whereas some patients demonstrated decreased ACPA and/or RF levels after treatment, this change was also unrelated to changes in type I IFN-response gene expression levels. These data do not support a role for an increased type I IFN-response activity in alteration of autoantibody titres in RA.

### TNF/IFN cross regulation and clinical response to treatment

Finally, we investigated if the treatment-induced changes in type I IFN-response gene expression levels were associated with clinical response to treatment. Therefore, the patients (n=15) were divided into two groups based on induction or reduction of the 34 IFN-response genes as demonstrated in Figure 1A. Subsequently, clinical parameters were compared between the two groups. Interestingly, the patients who showed an increase in type I IFN-response gene expression levels had a poor clinical response to treatment. This was reflected by lower changes in disease activity scores (DAS, P=0.013)), higher disease activity after treatment with higher tender joint counts (TJC, P=0.015) and a higher health assessment questionnaire score (HAQ, P=0.008). Accordingly, all patients without an anti-TNF induced increase in type I IFN gene activity had a good or moderate response to treatment as assessed by EULAR response criteria (P=0.018, Figure 3).
To confirm and further extend the observed association between treatment-induced change in type I IFN-induced gene expression levels and clinical response to treatment, five EULAR good and five EULAR poor responders were selected and the expression levels of 15 IFN-response genes (selected from the 34 gene set used above, see Table 2) were measured at baseline, 1, 2, 3 and 4 months after treatment by quantitative real-time PCR. The expression levels were averaged for the individual patients and the treatment-induced changes in IFN-response gene expression levels over time were compared between the two response groups using two-way ANOVA. Overall, the IFN response genes showed an upregulation in the poor responder group, which was most prominent at two months after the start of therapy (data not shown). At the individual gene level the increased expression between in poor versus good responders reached significance for the OAS1 and LGALS3BP genes (Figure 4A and 4B). For three other IFN-response genes (Mx2, OAS2 and SERPING1) a trend (∼p=0.06, data not shown) was observed towards increased expression in the non-responder patients. Combining the five genes (OAS1, LGALS3BP, Mx2, OAS2 and SERPING1) together into one gene set improved the significance (Figure 4C). These data confirm the microarray data, and demonstrate that poor clinical response to Infliximab treatment is associated with increased treatment-induced type I IFN-response gene activity.

### Example 2: Evaluation of a pharmacological effect of a treatment of RA patients with Infliximab

### Materials & Methods

### Patients

Consecutive patients with RA according to the ACR criteria were enrolled in the study at the outpatient clinic of the Academic Medical Center (AMC) in Amsterdam over a period of one year. Inclusion criteria were: 18-85 years of age, a failure of at least two disease modifying anti-rheumatic drugs (DMARDs) including methotrexate (MTX), and active disease (DAS28 ≥ 3.2). Patients with a history of an acute inflammatory joint disease of different origin or previous use of a TNF blocking agent were excluded. Patients were on stable maximally tolerable MTX treatment. Whole blood samples (2.5 ml) were obtained using PAXgene tubes (PreAnalytix, GmbH, Germany) from 33 RA patients prior to initiation of anti-TNFa therapy with Infliximab (3 mg/kg intravenously at baseline, at week 2, 6, and subsequently every 8 weeks). After one month of treatment a second PAXgene tube was obtained. All patients gave written informed consent and the study protocol was approved by the Medical Ethics Committee (AMC). From 15 randomly chosen patients gene expression profiles were determined. The remaining 18 patients were used as a validation group to confirm gene expression levels by quantitative real-time PCR. An overview of the patients' characteristics is given in Table 5.
After 16 weeks of treatment the clinical response to treatment was assessed using both the EULAR criteria (22;23) as well as the reduction in DAS28 of at least 1.2 (24).

### Blood sampling for RNA isolation

2.5 ml blood was drawn in PAXgene blood RNA isolation tubes (PreAnalytix, GmbH, Germany) and stored at -20°C. Tubes were thawed for 2 hours at room temperature prior to RNA isolation. Next, total RNA was isolated using the PAXgene RNA isolation kit according to the manufacturer's instructions including a DNAse (Qiagen, Venlo, Netherlands) step to remove genomic DNA. Quantity and purity of the RNA was tested using the Nanodrop spectrophotometer (Nanodrop Technologies, Wilmington, DE).

### Sample hybridisation for microarray analysis

We used 43K cDNA microarrays from the Stanford Functional Genomics Facility (http://microarray.org/sfgf/) printed on aminosilane-coated slides containing ∼20.000 unique genes. Only one batch of arrays was used for all experiments. First DNA spots were UV-cross linked to the slide using 150-300 mJoules. Prior to sample hybridisation, slides were pre-hybridised at 42 degrees Celsius for 15 minutes in a solution containing 40% ultra-pure formamide (Invitrogen, Breda, Netherlands), 5% SSC (Biochemika, Sigma), 0.1% SDS (Fluka Chemie, GmbH, Switzerland) and 50 g/ml BSA (Panvera, Madison, WI). After pre-hybridisation slides were briefly rinsed in MilliQ water, thoroughly washed in boiling water and 95% ethanol and air-dried. Sample preparation and microarray hybridisation was performed as described previously (25) apart from the different post-processing and pre-hybridisation described above.

### Microarray data analysis

Data storage and filtering was performed using the Stanford Microarray Database (26) (http://genome-www5.stanford.edu//) as described previously (27). Raw data can be downloaded from the publicly accessible Stanford database website. Statistical Analysis of Microarrays (28) (SAM) was used to determine significantly differential expressed genes. A gene was considered as significantly differential expressed if the False Discovery Rate (FDR) was equal to or less than 5%. Cluster analysis (29) was used to define clusters of co-ordinately changed genes after which the data was visualized using Treeview. To interpret our data after SAM analysis, we applied PANTHER (Protein ANalysis THrough Evolutionary Relationships) Classification System (Applied Biosystems, Foster City, CA) at http://PANTHER.appliedbiosystem.com (30;31). This analysis uses the binomial statistics tool to compare the list of significantly up- or down-regulated genes to a reference list to statistically determine over- or under- representation of PANTHER classification categories such as biological processes. A Bonferroni correction was applied to correct for multiple testing and after classification analysis a significant p-value (p<0.05) indicates that a given category may be of biological interest.

### Real-time PCR

RNA (0.5 µg) was reverse transcribed into cDNA using the Revertaid H-minus cDNA synthesis kit (MBI Fermentas, St. Leon-Rot, Germany) according to the manufacturer's instructions. Quantitative real-time PCR was performed using an ABI Prism 7900HT Sequence detection system (Applied Biosystems) using SybrGreen (Applied Biosystems). Primers were designed using Primer Express software and guidelines (Applied Biosystems). To calculate arbitrary values of mRNA levels and to correct for differences in primer efficiencies a standard curve was constructed. Expression levels of target genes were expressed relative to 18SRNA.

### Statistical analysis

Data was analyzed using the software programs Graphpad Prism 4 (Graphpad Software, Inc., La Jolla, CA) and Statistical Package for Social Sciences version 14.0 (SPSS; Chicago, IL). First data was checked for normal distribution using the Kolmogorov-Smirnov Z test. Two-group comparison of normally distributed data was analyzed using the non-parametric independent sample t-test including Levene's test for equality of variances. Not normally distributed data was analyzed using the two-independent-samples Mann-Whitney U test. Differences were considered statistically significant with p-values less than 0.05.

### Results

### Pharmacogenomic response to Infliximab treatment

The bioactivity of TNF in RA patients can be measured retrospectively by analyzing the change in gene transcription after TNF blockade. In this explorative study we set out to determine the transcriptional response to anti-TNF treatment in RA using cDNA microarray analysis of PB cells. The gene expression profiles of 15 RA patients before (t=0) and one month after (t=1) anti-TNF treatment (Infliximab) were analyzed. To determine the significant pharmacological response to treatment we performed a paired analysis using significance analysis of microarrays (SAM). Using a false discovery rate (FDR) of 5% this analysis revealed a large number of significantly changed genes: 692 genes were significantly up-regulated whereas 931 genes were significantly down-regulated after one month of anti-TNF treatment (for total gene list see supplementary Table S1). Accordingly, these 1623 genes encompass the pharmacogenomic anti-TNF response set and are referred to as such in this article.
The most significantly down-regulated gene was Peptidase Inhibitor 3 (PI3/SKALP), which expression level was down-regulated 1.84 times after treatment. This significant down-regulation was confirmed by quantitative real-time PCR in the validation cohort of in total 18 RA patients receiving treatment (p=0.02; data not shown). To verify that the gene expression changes were anti-TNF specific, two different gene sets involved in TNFR1 and TNFR2 signalling (Biocarta) were obtained. For all genes in both gene sets the average value in expression level for each patient was calculated and pre-treatment levels were compared with post-treatment levels. This revealed a significant down-regulation of both gene sets after TNF-blockade (paired t-test p<0.05, data not shown) confirming that both TNFR signalling pathways are affected by Infliximab.
To unravel the biological function of the pharmacogenomic response genes we applied PANTHER's classification analysis. The down-regulated genes could be classified into several biological pathways such as inflammation, angiogenesis, and B and T cell activation (Table 4) indicating that blocking TNF dampens these immune related pathways. The up-regulated genes consisted of many genes with unknown function and could therefore not be classified into pathways.
Collectively, these data demonstrate the existence of a universal pharmacogenomic response gene set reflecting the pharmacodynamic changes in PB after neutralization of TNF.

### Pharmacogenomic response profile is common in all patients

The identification of a pharmacogenomic response gene set allows evaluation of the presence or absence of an anti-TNF response for each individual patient. This knowledge will provide information regarding the presence or absence of TNF bioactivity in relation to clinical response. Therefore, we calculated for each patient the treatment-induced change (ratio T=1/T=0) in transcript level for each gene in the pharmacogenomic response gene set and visualized the response values between patients using supervised one-way hierarchical cluster analysis (Figure 5). This analysis revealed significant changes in the expression of the pharmacogenomic response set in all RA patients tested, irrespective of clinical response.
However, this analysis does not give much information about the possible inter-individual difference in the extent of pharmacological response. Hence, for each individual patient a mean expression value for both the up- and down-regulated regulated pharmacogenomic response genes was calculated and pre-treatment values were compared with post-treatment values (Figure 5B). These results revealed that all patients treated displayed a similar magnitude of pharmacogenomic response.
Next we wanted to analyse if inter-individual differences present at baseline or after treatment may be related to clinical response to treatment. Therefore, for each clinical response group ( poor, moderate and good responders) the average expression levels for both the up- and down-regulated pharmacogenomic response genes were calculated and the different response groups were compared with each other (Figure 5C). Baseline, pharmacodynamic change as well as remaining expression levels for the genes encompassing the above described pharmacogenomic anti-TNF response set were similar between the different clinical response groups. Collectively, these data could not reveal consistent differences between clinical responders and non-responders in the pharmacogenomic response.
These results imply that all RA patients have an active TNF response program prior to treatment reflected by a common pharmacological response towards anti-TNF irrespective to clinical response to treatment.

### Heterogeneity in pharmacological response

Although all patients showed a common pharmacogenomic response, not all patients exhibited a good clinical response to treatment. This suggests that other pathways might be differentially changed after anti-TNF treatment possibly related to clinical response. To delineate the existence of subtle inter-individual differences in the pharmacodynamic effects of Infliximab treatment we next investigated the presence of genes that are differentially affected between patients and were therefore missed in the previous group-based paired analysis. We searched for genes that showed at least a two-fold change after treatment in at least 4 patients. This resulted in the selection of 440 genes, which were subjected to unsupervised two-way hierarchically clustering and visualized by Treeview (Figure 6A). This analysis divided the RA patients into two different patient groups (I and II) based on their difference in pharmacological response. Usage of different criteria for the gene selection generally resulted in an identical subdivision, indicative for the robustness of this cluster-based subclassification of patients (data not shown). Application of SAM analysis between all the expressed genes in the two pharmacological response groups revealed 2777 significantly (FDR<5%) differentially changed genes: 249 genes with ratio values higher in patients from group II and 2528 genes with lower ratio values. Cluster analysis was used to visualize these pharmacological differences in gene expression changes and PANTHER classification analysis was applied to determine the biological relevance of the pharmacological differences (Figure 6B). This analysis revealed that the up-regulated genes in group I are involved in processes like oxidative phosphorylation, DNA repair, (pro-) apoptosis, protein metabolism, modification and biosynthesis, whereas genes up-regulated in group II are involved in (anti-)apoptosis and cell proliferation and differentiation.

### Clinical differences between patients of groups I and II

Subsequently, the clinical differences between patients of the two different pharmacological response groups were investigated. Interestingly, the patients from group I had a significantly lower disease activity score (DAS28) before start of treatment compared to patients from group II. Although both groups contained responder and non-responder patients according to a change of DAS28 after 16 weeks of at least 1.2 (24), only group I contained patients with good responder scores as assessed by EULAR. Consistent with higher levels of disease activity at baseline in group II, patients in this group had significantly higher thrombocyte counts compared to group I (32). There were no differences in ACPA and RF titres, disease duration, change in DAS after 16 weeks, medication, treatment dose, BSE and CRP levels but group I did contain more males than group II.

### Discussion

The current study demonstrates the pharmacological effect of TNF blockade on the peripheral blood transcriptome profile. Four weeks after the start of TNF blockade significant changes in the expression levels of approximately 1600 genes were found. Pathway level analysis of these significant changes in gene expression levels revealed an overall dampening of immune response after TNF blockade. According to its pro-inflammatory cytokine activity we observed a down-regulation of genes involved in inflammation, angiogenesis, T and B cell activation. Interestingly, we observed this down-modulation of inflammation in every RA patient treated irrespective of clinical response, implying that all RA patients had an active TNF response program prior to treatment contributing to disease.
Despite the presence of an active TNF response program in all RA patients treated, not all patients responded well to Infliximab treatment suggesting that other gene transcripts may be differentially changed between patients. Indeed, a careful analysis on the subtle inter-individual differences in response revealed beyond the common pharmacogenomic response the presence of two significantly differential pharmacodynamic response patterns. These two responses were characterized by a differential treatment-induced change in expression levels of genes involved in processes like oxidative phosphorylation, DNA repair, protein biosynthesis, cell proliferation and apoptosis.
The differential pharmacodynamic response pattern separated the patients into two groups. Interestingly, only one response group contained good responders to treatment as assessed by EULAR indicating that the differential pharmacodynamic response patterns may be related to clinical response to treatment.
Primary research has clearly revealed the importance of TNF in the pathogenesis of RA (3). Initially, TNF was presented as the most dominant pro-inflammatory cytokine driving downstream activation of other pro-inflammatory cytokines such as IL-6 and IL-1β (36). Consequently, clinical trials started with monoclonal antibodies aimed at TNF neutralization which showed reduction of clinical signs in approximately two-third of the patients treated (4). However, since not all RA patients showed a good response to TNF blockade these trials also indicated that the model (Figure 7; model 1, individual A) describing a TNF mediated pro-inflammatory signalling cascade leading to disease does not fit for all patients. Subsequent studies revealed significant roles for other cytokines as IL-18, RANKL, IL-17, IL-32, etc. in the pathogenesis of RA (17) implying that in some patients other cytokines than TNF may play a dominant role (Figure 7; model 1, individual B). Because our pharmacogenomic data showed that all RA patients studied have an active TNF response profile prior to treatment which is affected by TNF blockade in all patients irrespective of clinical response, model 1 can be considered as invalid. Our data favour the existence of parallel cytokine networks operating simultaneously which facilitate the bypass of TNF neutralization (17) (Figure 7; model 2).
Overall, our data clearly show that all RA patients tested have an active TNF response program which is affected after TNF blockade irrespective of clinical response to treatment.

**Table 1. Baseline patients' characteristics**

| | | **Array samples** | **Validation group (n=18)** |
|---|---|---|---|
| | | **(n=15)** | |
| Age | | 51 (39-55) | 58 (51-69)* |
| Gender (female/male) | | 7/8 | 14/4 |

| Disease Characteristics: | | | |
|---|---|---|---|
| | DAS28 | 5.6 (4.6-7.0) | 5.7 (5.0-6.6) |
| | CRP (mg/dl) | 8 (6-22) | 13 (5-44) |
| | ESR | 25 (12-41) | 32 (16-47) |
| | ACPA titre | 100 (15-595) | 541 (121-1805) |
| | IgM RF titre | 28 (14-133) | 67 (22-182) |
| | Disease duration (months) | 77 (29-240) | 65 (36-1992) |
| | Erosions | n=13 | n=15 |

| Medication: | | | |
|---|---|---|---|
| | MTX dose (mg/week) | 25 (20-30) | 21 (15-25) |
| | Prednisone | n=2 | n=5 |
| | NSAID | n=7 | n=12 |

| | | | |
|---|---|---|---|
| * Values are listed as median (interquartile range 25-75) unless indicated otherwise; *p*=0.045 t-test between array and validation group; DAS28, 28-joint Disease Activity Score; CRP, C-reactive protein; ESR, erythrocyte sedimentation rate; ACPA, anti-citrullinated peptide antibodies; RF, Rheumatoid Factor; MTX, methotrexate; NSAID, Non-Steroidal Anti-Inflammatory Drugs | | | |

**Table 2 IFN-response gene sets used in study**

| IFN set | Validatio | Symbol | Name |
|---|---|---|---|
| (34 | n (15 | | |
| genes) | genes) | | |
| X | * | RSAD2 | Radical S-adenosyl methionine domain containing 2 (alias cig5) |
| X | * | IFI44L | Interferon-induced protein 44-like |
| X | * | OAS1 | 2',5'-oligoadenylate synthetase 1, 40/46kDa |
| X | | AA142842 | none |
| X | | AA075725 | none |
| X | * | ISG15 | ISG15 ubiquitin-like modifier |
| X | * | SAMD9L | Sterile alpha motif domain containing 9-like |
| X | * | EPSTI1 | Epithelial stromal interaction 1 (breast) |
| X | * | IFIT1 | Interferon-induced protein with tetratricopeptide repeats 1 |
| X | | PARP14 | Poly (ADP-ribose) polymerase family, member 14 |
| X | * | IFI6 | Interferon, alpha-inducible protein 6 |
| X | * | SERPING | Serpin peptidase inhibitor, clade G (C1 inhibitor), |
| | | 1 | member 1 |
| X | * | IFI35 | Interferon-induced protein 35 |
| X | * | IRF2 | Interferon regulatory factor 2 |
| X | * | OAS2 | 2'-5'-oligoadenylate synthetase 2, 69/71kDa |
| X | | AI347124 | none |
| X | | EIF2AK2 | Eukaryotic translation initiation factor 2-alpha kinase 2 |
| X | | RTP4 | Receptor (chemosensory) transporter protein 4 |
| X | | Hs. 128576 | CDNA FLJ90394 fis, clone NT2RP2005632 |
| X | | Hs.97872 | Transcribed locus |
| X | | IFIT2 | Interferon-induced protein with tetratricopeptide repeats 2 |
| X | | MX1 | Myxovirus (influenza virus) resistance 1 |
| X | | PLSCR1 | Phospholipid scramblase 1 |
| X | | IRF7 | Interferon regulatory factor 7 |
| X | * | MX2 | Myxovirus (influenza virus) resistance 2 (mouse) |
| X | | TNFAIP6 | Tumor necrosis factor, alpha-induced protein 6 |
| X | | RNF213 | Ring finger protein 213 |
| X | | UBE2L6 | Ubiquitin-conjugating enzyme E2L 6 |
| X | * | LGALS3B | Lectin, galactoside-binding, soluble, 3 binding |
| | | P | protein |
| X | | IFI16 | Interferon, gamma-inducible protein 16 |
| X | * | IFITM1 | Interferon induced transmembrane protein 1 (9-27) |
| X | | ATF3 | Activating transcription factor 3 |
| X | | TAP1 | Transporter 1, ATP-binding cassette, sub-family B (MDR/TAP) |
| X | | IL1RN | Interleukin 1 receptor antagonist |

| | | | |
|---|---|---|---|
| *"X" means that the gene in this row has been selected in the "IFN set";* "*" *means that the gene in this row has been selected in the "validation set"* | | | |

**Table 3. Baseline patients' characteristics**

| | | **Array** | **Validation group** |
|---|---|---|---|
| | | **samples** | **(n=18)** |
| | | **(n=15)** | |
| Age | | 51 (39-55) | 58 (51-69)* |
| Gender (female/male) | | 7/8 | 14/4 |

| Disease Characteristics: | | | |
|---|---|---|---|
| | DAS28 | 5.6 (4.6-7.0) | 5.7 (5.0-6.6) |
| | CRP (mg/dl) | 8 (6-22) | 13 (5-44) |
| | ESR | 25 (12-41) | 32 (16-47) |
| | ACPA titre | 100 (15-595) | 541 (121-1805) |
| | IgM RF titre | 28 (14-133) | 67 (22-182) |
| | Disease duration (months) | 77 (29-240) | 65 (36-1992) |
| | Erosions | n=13 | n=15 |

| Medication: | | | |
|---|---|---|---|
| | MTX dose (mg/week) | 25 (20-30) | 21 (15-25) |
| | Prednisone | n=2 | n=5 |
| | NSAID | n=7 | n=12 |

| | | | |
|---|---|---|---|
| * Values are listed as median (interquartile range 25-75) unless indicated otherwise; * p=0.045 t-test between array and validation group; DAS28, 28-joint Disease Activity Score; CRP, C-reactive protein; ESR, erythrocyte sedimentation rate; ACPA, anti-citrullinated peptide antibodies; RF, Rheumatoid Factor; MTX, methotrexate; NSAID, Non-Steroidal Anti-Inflammatory Drugs | | | |

**Table 4. PANTHER pathway-level analysis of significantly differential expressed genes**

| **Pathways genes:** | **down-regulated** | **Ref.** | **FDR<5** | **Expecte** | **p-value** |
|---|---|---|---|---|---|
| | | **list** | **%** | **d** | |
| Angiogenesis | | 229 | 21 | 6.11 | 2.32E-04 |
| Inflammation mediated by | | 315 | 25 | 8.41 | 3.18E-04 |
| chemokine and cytokine signalling | | | | | |
| pathway | | | | | |
| B cell activation | | 86 | 12 | 2.3 | 7.18E-04 |
| Integrin signalling pathway | | 227 | 20 | 6.06 | 7.30E-04 |
| T cell activation | | 111 | 13 | 2.96 | 1.86E-03 |
| FGF signalling pathway | | 148 | 15 | 3.95 | 2.23E-03 |
| PDGF signalling pathway | | 189 | 17 | 5.05 | 2.82E-03 |
| TGF-beta signalling pathway | | 154 | 15 | 4.11 | 3.50E-03 |
| Ras Pathway | | 91 | 11 | 2.43 | 6.39E-03 |
| EGF receptor signalling pathway | | 150 | 14 | 4 | 1.01E-02 |
| Endothelin signalling pathway | | 98 | 11 | 2.62 | 1.22E-02 |
| Alzheimer | disease-amyloid | 77 | 9 | 2.06 | 3.93E-02 |
| secretase | | | | | |
| pathway | | | | | |

| | | | | | |
|---|---|---|---|---|---|
| FDR, False Discovery Rate; Ref. list, NCBI homo sapiens reference gene list | | | | | |

**Table 5. Demographic and clinical features of the patients in response groups I and II***

| | **Group I** | **Group II** |
|---|---|---|
| Total patients | n=9 | n=6 |
| Gender | 3 female: 6 male | 4 female: 2 male |
| Age | 52 (41 - 61.5) | 45.5 (33.5 - 53) |
| Disease duration | 98 (29 - 256) | 73.5 (29 - 213) |
| (months) | | |
| MTX dose (mg/week) | 30 (17.5 - 30) | 22.5 (16.875 - 25) |
| Treatment dose | 219 (192 - 270) | 231 (183.75 - 283.5) |
| Weight T=0 | 73 (64 - 90) | 77 (61.25 - 94.5) |
| DAS28 T=0 | 4.6 (4.1 - 6.25) | 6.9 (5.48 - 7.33) * |
| DAS28 T=1 | 4.1 (3.15 - 4.9) | 4.7 (4.2 - 6.15) |
| Change in DAS28 | 1.2 (0.9 - 2.75) | 0.95 (0.425 - 2.975) |
| (at 16 weeks) | | |
| EULAR 0-1-2(n) | 3-2-4 | 2-4-0 |
| IgM RF titre | 19 (7.5 - 82.5) | 128 (13.75 - 428.25) |
| ACPA titre | 130 (6 - 991.5) | 76 (39.75 - 503) |
| Thrombocytes T=0 | 292 (225.5 - 324.5) | 358.5 (339.25 - 399.5) ** |
| Leucocytes T=0 | 6.8 (5.15 - 8) | 6.3 (4.475 - 8.375) |
| HB T=0 | 8.7 (7.55 - 8.9) | 7.85 (6.15 - 8.825) |
| Tender joint count | 9 (5 - 14) | 16 (10 - 21.25) |
| Swollen joint count | 9 (6 - 15) | 14.5 (9.25 - 16.25) |
| ESR T=0 | 24 (7 - 31.5) | 40 (13.5 - 61.75) |
| CRP T=0 (mg/dl) | 8 (5.5 - 17.5) | 10.5 (5.25 - 36.5) |

| | | |
|---|---|---|
| * Values are listed as median (interquartile range 25-75) unless indicated otherwise; * means p<0.05 and ** p<0.01 T-test group I *us.* II RF, rheumatoid factor; ACPA, anti-citrullinated peptide antibodies; CRP, C-reactive protein; DAS28, 28-joint Disease Activity Score; ESR, erythrocyte sedimentation rate; MTX, methotrexate; HB, haemoglobin | | |

**Table 6: A list of Genes predictive of TNF response**

| **Gene array information** | **GeneID** | **Entrez GeneID** | **UGRepAcc** |
|---|---|---|---|
| Hs.632994 ∥ ∥ MRNA differentially expressed in malignant melanoma, clone MM | | | |
| D3 (1) | Hs.632994 | | BM802949 |
| Hs.591198 ∥ EVI2A ∥ Ecotropic viral integration site 2A (1) | Hs.591198 | 2123 | NM_001003927 |
| Hs.123464 ∥ P2RY5 ∥ **Purinergic receptor P2Y, G-protein coupled, 5 (2) | Hs.123464 | 10161 | BC045651 |
| Hs.478150 ∥ PDCD10 ∥ Programmed cell death 10 (1) | Hs.478150 | 11235 | BC002506 |
| Hs.508644 ∥ FLJ10154 ∥ Hypothetical protein FLJ10154 (1) | Hs.508644 | 55082 | CR936748 |
| Hs.657723 ∥ ∥ **Transcribed locus, weakly similar to NP_055314.1 coupled | | | |
| purinergic receptor P2Y10 [Homo sapiens] (1) | Hs.657723 | | |
| Hs.466507 ∥ LSR ∥ Lipolysis stimulated lipoprotein receptor (1) | Hs.466507 | 51599 | AK126834 |
| Hs.633884 ∥ ∥ Transcribed locus, moderately similar to XP_001096337.1 similar | | | |
| to 60 kDa heat shock protein, mitochondrial precursor (Hsp60) (60 kDa | | | |
| chaperonin) (CPN60) (Heat shock protein 60) (HSP-60) (Mitochondria (1) | Hs.633884 | | BQ050293 |
| Hs.503315 ∥ PRKRIR ∥ Protein-kinase, interferon-inducible double stranded RNA | | | |
| dependent inhibitor, repressor of (P58 repressor) (2) | Hs.503315 | 5612 | BX641144 |
| ∥ ∥ ∥ ∥ AA502960 ∥ ∥ ∥ ∥ 107285 | IMAGE:940687 | | AA502960 |
| Hs.351475 ∥ POLR2K ∥ Polymerase (RNA) II (DNA directed) polypeptide K, | | | |
| 7.0kDa (1) | Hs.351475 | 5440 | B1758413 |
| Hs.400295 ∥ RPL30 ∥ Ribosomal protein L30 (1) | Hs.400295 | 6156 | AK128768 |
| Hs.534346 ∥ RPS7 ∥ Ribosomal protein S7 (1) | Hs.534346 | 6201 | BM478334 |
| Hs.631580 ∥ SAE2 ∥ SUMO1 activating enzyme subunit 2 (1) | Hs.631580 | 10054 | AK124730 |
| Hs.519930 ∥ C6orf62 ∥ Chromosome 6 open reading frame 62 (2) | Hs.519930 | 81688 | AL136632 |
| Hs.33922 ∥ C1orf156 ∥ Chromosome 1 open reading frame 156 (1) | Hs.33922 | 92342 | CR609500 |
| Hs.532019 ∥ MRPL1 ∥ Mitochondrial ribosomal protein L1 (1) | Hs.532019 | 65008 | NM_020236 |
| Hs.215766 ∥ GTPBP4 ∥ GTP binding protein 4 (1) | Hs.215766 | 23560 | AK126769 |
| Hs.458412 ∥ TRIM52 Tripartite motif-containing 52 (1) | Hs.458412 | | BM811518 |
| Hs.232375 ∥ ACAT1 ∥ Acetyl-Coenzyme A acetyltransferase 1 (acetoacetyl | | | |
| Coenzyme A thiolase) (1) | Hs.232375 | 38 | NM_000019 |
| Hs.156625 ∥ C1orf80 ∥ **Chromosome 1 open reading frame 80 (1) | Hs.156625 | 64853 | AK289865 |

**Table S1**

| **Symbol** | **product size (bp)** | **Forward primer sequence** | **Reverse primer sequence** | | |
|---|---|---|---|---|---|
| 18SRNA | 84 | CCGAGTAAGTGCGGGTCATAA | CCATCCAATCGGTAGTAGCG | | |
| RSAD2 | 90 | GTGGTTCCAGAATTATGGTGAGTATTT | CCACGGCCAATAAGGACATT | | |
| OAS1 | 175 | TGCGCTCAGCTTCGTACTGA | GGTGGAGAACTCGCCCTCTT | | |
| IFI44L | 77 | CCGAGCGGTATAGGATATATTCTGTT | TGCTCCTTCTGCCCCATCTA | | |

| **Pre-designed Taqman Gene Expression Assays used for Fluidigm's BioMark™ Real-Time PCR System:** | | | | | |
|---|---|---|---|---|---|
| **Symbol** | **Assay ID** | **Gene Name** | | | |
| IFI6 | Hs00242571_m1 | interferon, alpha-inducible protein 6 | | | |
| IFIT1 | Hs00356631_g1 | interferon-induced protein with tetratricopeptide repeats 1 | | | |
| IFITM1 | Hs00705137_s1 | interferon induced transmembrane protein 1 (9-27) | | | |
| IRF2 | Hs01082884_m1 | interferon regulatory factor 2 | | | |
| ISG15 | Hs01921425_s1 | ISG15 ubiquitin-like modifier | | | |
| OAS2 | Hs00942643_m1 | 2'-5'-oligoadenylate synthetase 2, 69/71kDa | | | |
| RSAD2 | Hs00369813_m1 | radical S-adenosyl methionine domain containing 2 | | | |
| SAMD9L | Hs00541567_s1 | sterile alpha motif domain containing 9-like | | | |
| SERPING1 | Hs00934329_m1 | serpin peptidase inhibitor, clade G (C1 inhibitor), member 1, (angioedema, hereditary) | | | |
| EPSTI1 | Hs00264424_m1 | epithelial stromal interaction 1 (breast) | | | |
| IPI35 | Hs00413458_m1 | interferon-induced protein 35 | | | |
| IFI44L | Hs00199115_m1 | interferon-induced protein 44-like | | | |
| Mx2 | Hs00159418_m1 | myxovirus (influenza virus) resistance 2 (mouse) | | | |
| OAS1 | Hs00242943_m1 | 2',5'-oligoadenylate synthetase 1, 40/46kDa | | | |
| 18SRNA | Hs99999901_s1 | Eukaryotic 18S rRNA;18S ribosomal RNA | | | |
| LGALS3BP | Hs01003086_m1 | lectin, galactoside-binding, soluble, 3 binding protein | | | |
| Supplementary Table S2. SAM paired analysis n=15 t=0 vs. t=1 | | | | | |
| | | | | | |
| Positive genes (692); increased after treatment | | | | | |

| Gene Name | Gene ID | Fold Change | q-value(%) | Entrez GeneID | UGRepAcc |
|---|---|---|---|---|---|
| \|\| \|\| \|\| \|\| N23865 \|\| \|\| \|\| \|\| 99644 | 254545 | 14.65 | 0.00 | | N23865 |
| Hs.648940 \|\| \|\| Transcribed locus, strongly similar to XP_001084055.1 neutrophil cytosolic factor 1 [Macaca mulatta] (1) | Hs.648940 | 8.91 | 0.00 | | AL577485 |
| Hs.449585 \|\| IGL@ \|\| Immunoglobulin lambda locus (5) | Hs.449585 | 4.13 | 0.00 | 3535 | AK057174 |
| Hs.409563 \|\| MGC29506 \|\| Hypothetical protein MGC29506 (1) | Hs.409563 | 3.44 | 0.00 | 51237 | AK292706 |
| Hs.517582 \|\| MCM5 \|\| Minichromosome maintenance complex component 5 (1) | Hs.517582 | 3.41 | 0.00 | 4174 | NM_006739 |
| \|\| \|\| \|\| \|\| AA229609 \|\| \|\| \|\| \|\| 112984 | 1010136 | 3.08 | 1.43 | | AA229609 |
| Hs.659235 \|\| SPTY2D1 \|\| SPT2, Suppressor of Ty, domain containing 1 (S. cerevisiae) (1) | Hs.659235 | 2.67 | 0.50 | | BX648114 |
| Hs.478125 \|\| INADL \|\| InaD-like (Drosophila) (1) | Hs.478125 | 2.62 | 1.75 | 10207 | NM_176877 |
| Hs.374650 \|\| IFITM3 \|\| Interferon induced transmembrane protein 3 (1-8U) (1) | Hs.374650 | 2.44 | 1.67 | 10410 | BF965170 |
| Hs.449909 \|\| RPSA \|\| Ribosomal protein SA (1) | Hs.449909 | 2.36 | 4.44 | 3921 | BM554167 |
| \|\| \|\| \|\| \|\| AA912032 \|\| \|\| \|\| \|\| 223522 | 1486028 | 2.30 | 1.67 | | AA912032 |
| Hs.444467 \|\| EEF1G \|\| Eukaryotic translation elongation factor 1 gamma (1) | Hs.444467 | 2.29 | 0.00 | | BM473292 |
| Hs.166312 \|\| ZNF600 \|\| Zinc finger protein 600 (1) | Hs.166312 | 2.28 | 0.00 | | AA995911 |
| Hs.448226 \|\| RPLPO-like \|\| Similar to ribosomal protein P0 (1) | Hs.448226 | 2.23 | 0.95 | | BM456985 |
| Hs.658976 \|\| \|\| Transcribed locus (1) | Hs.658976 | 2.23 | 0.00 | | AV762905 |
| Hs.449621 \|\| HLA-C \|\| Major histocompatibility complex, class I, C (4) | Hs.449621 | 2.19 | 0.50 | 3107 | BM923616 |
| Hs.643431 \|\| IGJ \|\| Immunoglobulin J polypeptide, linker protein for immunoglobulin alpha and mu polypeptides (2) | Hs.643431 | 2.16 | 4.88 | | CB955785 |
| Hs.529989 \|\| RNASET2 \|\| Ribonuclease T2 (1) | Hs.529989 | 2.14 | 2.15 | 8635 | AK001769 |
| Hs.129621 \|\| \|\| Transcribed locus (1) | Hs.129621 | 2.07 | 0.00 | | AL703996 |
| Hs.633610 \|\| \|\| Transcribed locus (1) | Hs.633610 | 2.05 | 0.50 | | BF435244 |
| Hs.370504 \|\| RPS15A \|\| Ribosomal protein S15a (1) | Hs.370504 | 2.04 | 4.01 | 6210 | BM907705 |
| Hs.163867 \|\| CD14 \|\| CD14 molecule (1) | Hs.163867 | 2.02 | 2.15 | 929 | NM_000591 |
| \|\| \|\| \|\| \|\| AA226543 \|\| \|\| \|\| \|\| 99284 | 1009241 | 2.00 | 2.02 | | AA225521 |
| Hs.201641 \|\| BASP1 \|\| Brain abundant, membrane attached signal protein 1 (1) | Hs.201641 | 1.99 | 2.15 | 10409 | NM_006317 |
| Hs.655579 \|\| \|\| Transcribed locus, strongly similar to XP_001113299.1 similar to ribosomal protein S27 [Macaca mulatta] (2) | Hs.655579 | 1.98 | 3.52 | | BF030952 |
| Hs.602613 \|\| \|\| Transcribed locus (1) | Hs.602613 | 1.97 | 1.75 | | AI733050 |
| Hs.276770 \|\| CD52 \|\| CD52 molecule (2) | Hs.276770 | 1.97 | 2.98 | 1043 | BQ276596 |
| Hs.524498 \|\| PA2G4 \|\| Proliferation-associated 2G4, 38kDa (1) | Hs.524498 | 1.96 | 0.00 | 5036 | NM_006191 |
| Hs.348935 \|\| IGLL1 \|\| Immunoglobulin lambda-like polypeptide 1 (2) | Hs.348935 | 1.92 | 2.15 | 3543 | NM_020070 |
| \|\| \|\| \|\| \|\| AA228922 \|\| \|\| \|\| \|\| 118717 | 1011375 | 1.90 | 2.15 | | AA228922 |
| Hs. 163113 \|\| C17orf62 \|\| Chromosome 17 open reading frame 62 (1) | Hs.163113 | 1.90 | 2.61 | 79415 | AK090484 |
| Hs.694002 \|\| \|\| **Transcribed locus (1) | Hs.694002 | 1.87 | 4.01 | | |
| Hs.663239 \|\| \|\| Transcribed locus (1) | Hs.663239 | 1.86 | 2.98 | | AA477708 |
| Hs.655103 \|\| FKBP11 \|\| FK506 binding protein 11, 19 kDa (1) | Hs.655103 | 1.85 | 0.95 | 51303 | AB209018 |
| Hs.655168 \|\| SFXN4 \|\| Sideroflexin 4 (1) | Hs.655168 | 1.84 | 0.95 | 119559 | BC050475 |
| \|\| \|\| \|\| \|\| AA229572 \|\| \|\| \|\| \|\| 116615 | 1010089 | 1.83 | 0.72 | | AA229572 |
| Hs.563509 \|\| AP1S1 \|\| Adaptor-related protein complex 1, sigma 1 subunit (1) | Hs.563509 | 1.83 | 3.52 | 1174 | NM_001283 |
| Hs.631567 \|\| CD79A \|\| CD79a molecule, immunoglobulin-associated alpha (1) | Hs.631567 | 1.83 | 0.00 | 973 | AK223371 |
| Hs.559841 \|\| \|\| Transcribed locus (1) | Hs.559841 | 1.82 | 0.89 | | BF432991 |
| Hs.522572 \|\| SFRS17A \|\| Splicing factor, arginine/serine-rich 17A (1) | Hs.522572 | 1.82 | 1.75 | 8227 | L03426 |
| Hs.501728 \|\| RHOG \|\| Ras homolog gene family, member G (rho G) (1) | Hs.501728 | 1.80 | 0.00 | 391 | BM912141 |
| Hs.463797 \|\| MRTO4 \|\| MRNA turnover 4 homolog (S. cerevisiae) (1) | Hs.463797 | 1.80 | 2.98 | 51154 | AK126387 |
| Hs.5662 \|\| GNB2L1 \|\| Guanine nucleotide binding protein (G protein), beta polypeptide 2-like 1 (1) | Hs.5662 | 1.80 | 0.00 | 10399 | BF312441 |
| Hs.655652 \|\| LILRB2 \|\| **Leukocyte immunoglobulin-like receptor, subfamily B (with TM and ITIM domains), member 2 (1) | Hs.655652 | 1.79 | 1.75 | 10288 | NM_005874 |
| Hs.515369 \|\| TYROBP \|\| TYRO protein tyrosine kinase binding protein (1) | Hs.515369 | 1.79 | 2.61 | 7305 | B1838734 |
| Hs.533262 \|\| ANAPC2 \|\| Anaphase promoting complex subunit 2 (1) | Hs.533262 | 1.79 | 0.89 | 29882 | NM_013366 |
| Hs.311640 \|\| RPS27A \|\| **Ribosomal protein S27a (1) | Hs.311640 | 1.78 | 2.61 | 6233 | BU588489 |
| Hs.567352 \|\| \|\| Transcribed locus, strongly similar to XP_001087364.1 similar to ribosomal protein L18a [Macaca mulatta] (2) | Hs.567352 | 1.78 | 0.00 | | BM563814 |
| Hs.2484 \|\| TCL1A \|\| T-cell leukemia/lymphoma 1A (1) | Hs.2484 | 1.78 | 0.89 | 8115 | BQ057554 |
| Hs.111577 \|\| ITM2C \|\| Integral membrane protein 2C (1) | Hs.111577 | 1.77 | 4.01 | 81618 | NM_030926 |
| Hs.16970 \|\| C13orf30 \|\| Chromosome 13 open reading frame 30 (1) | Hs.16970 | 1.75 | 4.01 | 144809 | NM_182508 |
| Hs.433670 \|\| FTL \|\| Ferritin, light polypeptide (1) | Hs.433670 | 1.75 | 0.00 | 2512 | BF244604 |
| Hs.664357 \|\| LOC728184 \|\| Similar to ribosomal protein L10 (2) | Hs.664357 | 1.75 | 0.50 | | CB994728 |
| Hs.205163 \|\| MRPL3 \|\| Mitochondrial ribosomal protein L3 (1) | Hs.205163 | 1.72 | 4.01 | 11222 | BM541805 |
| Hs.89575 \|\| CD79B \|\| CD79b molecule, immunoglobulin-associated beta (1) | Hs.89575 | 1.72 | 0.00 | 974 | AK223210 |
| Hs.469694 \|\| ZNF134 \|\| Zinc finger protein 134 (1) | Hs.469694 | 1.72 | 0.72 | 7693 | BC042636 |
| Hs.523443 \|\| HBB \|\| Hemoglobin, beta (1) | Hs.523443 | 1.72 | 1.75 | 3043 | BM811415 |
| Hs.607640 \|\| NBPF14 \|\| Neuroblastoma breakpoint family, member 14 (1) | Hs.607640 | 1.71 | 0.00 | 25832 | AK095459 |
| Hs.378103 \|\| RPS5 \|\| Ribosomal protein S5 (1) | Hs.378103 | 1.70 | 0.50 | 6193 | BG165682 |
| \|\| \|\| *mitoch. cont. ESTs \|\| \|\| \|\| \|\| \|\| \|\| 433353 | *mitoch. cont. 594112 | 1.70 | 0.00 | | |
| Hs.381061 \|\| RPL19 \|\| Ribosomal protein L19 (1) | Hs.381061 | 1.69 | 0.95 | 6143 | BF698920 |
| Hs.155975 \|\| PTPRCAP \|\| Protein tyrosine phosphatase, receptor type, C-associated protein (1) | Hs.155975 | 1.69 | 0.50 | 5790 | BQ051978 |
| \|\| \|\| \|\| \|\| AA663826 \|\| \|\| \|\| \|\| 224578 | 969844 | 1.68 | 2.61 | | AK024901 |
| Hs.637971 \|\| \|\| Transcribed locus, moderately similar to XP_001108613.1 similar to 60S acidic ribosomal protein P1 isoform 2 [Macaca mulatta] (1) | Hs.637971 | 1.68 | 0.72 | | BG213931 |
| Hs.171995 \|\| KLK3 \|\| Kallikrein-related peptidase 3 (2) | Hs.171995 | 1.68 | 2.61 | 354 | AF335478 |
| \|\| \|\| \|\| \|\| AI053574 \|\| \|\| \|\| \|\| 115157 | 1861675 | 1.68 | 4.88 | | AI053574 |
| Hs.668269 \|\| \|\| Transcribed locus (1) | Hs.668269 | 1.67 | 2.61 | | BJ997318 |
| Hs.381126 \|\| RPS14 \|\| Ribosomal protein S14 (2) | Hs.381126 | 1.67 | 1.75 | 6208 | BC110792 |
| Hs.520973 \|\| HSPB1 \|\| Heat shock 27kDa protein 1 (1) | Hs.520973 | 1.67 | 1.67 | 3315 | BM454532 |
| Hs.632274 \|\| FLJ33706 \|\| Hypothetical protein FLJ33706 (1) | Hs.632274 | 1.66 | 0.50 | 284805 | AK091025 |
| Hs.666908 \|\| \|\| Transcribed locus (1) | Hs.666908 | 1.66 | 2.02 | | AI733986 |
| Hs.600402 \|\| \|\| Transcribed locus (1) | Hs.600402 | 1.66 | 1.75 | | AA581483 |
| Hs.425091 \|\| FAM44B \|\| Family with sequence similarity 44, member B (2) | Hs.425091 | 1.66 | 1.67 | 91272 | BM908302 |
| Hs.515517 \|\| RPL18 \|\| Ribosomal protein L18 (1) | Hs.515517 | 1.65 | 0.95 | 6141 | BF970882 |
| Hs.513266 \|\| NDUFB10 \|\| NADH dehydrogenase (ubiquinone) 1 beta subcomplex, 10, 22kDa (1) | Hs.513266 | 1.65 | 2.02 | 4716 | BQ278464 |
| Hs.436161 \|\| MRPS18C \|\| Mitochondrial ribosomal protein S18C (1) | Hs.436161 | 1.64 | 0.95 | 51023 | NM_016067 |
| Hs.425125 \|\| RPL29 \|\| Ribosomal protein L29 (2) | Hs.425125 | 1.64 | 0.00 | 6159 | BM554029 |
| Hs.406620 \|\| \|\| CDNA: FLJ21700 fis, clone COL09849, highly similar to HSU14972 Human ribosomal protein S10 mRNA (2) | Hs.406620 | 1.64 | 4.88 | | AK025353 |
| Hs.434207 \|\| \|\| Transcribed locus (1) | Hs.434207 | 1.63 | 0.00 | | DB329727 |
| \|\| \|\| *mitoch. cont. \|\| \|\| \|\| \|\| \|\| \|\| 433311 | *mitoch. cont. 1203893 | 1.63 | 0.95 | | |
| \|\| \|\| \|\| \|\| AA650138 \|\| \|\| \|\| \|\| 112694 | 1190928 | 1.63 | 0.95 | | AI821072 |
| Hs.602741 \|\| \|\| Transcribed locus, moderately similar to XP_528269.1 cleavage and polyadenylation specific factor 1, 160kDa [Pan troglodytes] (1) | Hs.602741 | 1.63 | 1.67 | | AI733518 |
| Hs.272011 \|\| \|\| **Transcribed locus, strongly similar to XP_531236.1 hypothetical protein XP_531236 [Pan troglodytes] (1) | Hs.272011 | 1.63 | 0.00 | | CD556322 |
| \|\| \|\| *mitoch. cont. ESTs \|\| \|\| \|\| \|\| \|\| \|\| 433352 | *mitoch. cont. 593482 | 1.62 | 0.95 | | |
| Hs.387576 \|\| LOC440991 \|\| **Similar to 40S ribosomal protein S3 (2) | Hs.387576 | 1.62 | 1.24 | | BM918217 |
| Hs.594584 \|\| \|\| Transcribed locus (1) | Hs.594584 | 1.62 | 3.52 | | BQ924201 |
| Hs.510635 \|\| IGHG1 \|\| Immunoglobulin heavy constant gamma 1 (G1m marker) (5) | Hs.510635 | 1.61 | 2.15 | 3500 | AK090461 |
| Hs.509264 \|\| KLHDC2 \|\| Kelch domain containing 2 (1) | Hs.509264 | 1.61 | 0.72 | 23588 | AK056298 |
| Hs.31431 \|\| FN3KRP \|\| Fructosamine-3-kinase-related protein (1) | Hs.31431 | 1.61 | 4.44 | 79672 | AL136631 |
| Hs.53155 \|\| CFP \|\| Complement factor properdin (1) | Hs.53155 | 1.61 | 2.15 | 5199 | AK122955 |
| Hs.591900 \|\| \|\| Transcribed locus (1) | Hs.591900 | 1.60 | 0.95 | | AL699047 |
| Hs.148609 \|\| C6orf157 \|\| Chromosome 6 open reading frame 157 (1) | Hs.148609 | 1.60 | 0.95 | 90025 | BC036049 |
| Hs.600123 \|\| \|\| Transcribed locus (1) | Hs.600123 | 1.60 | 1.67 | | BX493113 |
| \|\| \|\| \|\| \|\| R52054 \|\| \|\| \|\| \|\| 115153 | 154220 | 1.60 | 0.37 | | R52054 |
| \|\| \|\| \|\| \|\| AI347886 \|\| \|\| \|\| \|\| 318139 | 1916973 | 1.59 | 0.00 | | AI624855 |
| Hs.633768 \|\| \|\| Transcribed locus, weakly similar to XP_001087364.1 similar to ribosomal protein L18a [Macaca mulatta] (1) | Hs.633768 | 1.59 | 0.72 | | W81118 |
| Hs.617340 \|\| PNPLA8 \|\| Patatin-like phospholipase domain containing 8 (1) | Hs.617340 | 1.59 | 2.61 | 50640 | AL834147 |
| \|\| \|\| \|\| \|\| AA987218 \|\| \|\| \|\| \|\| 103085 | 1603301 | 1.58 | 2.98 | | AA987218 |
| Hs.182825 \|\| RPL35 \|\| Ribosomal protein L35 (2) | Hs.182825 | 1.58 | 4.88 | 11224 | CR622666 |
| Hs.442609 \|\| MRPL38 \|\| Mitochondrial ribosomal protein L38 (1) | Hs.442609 | 1.58 | 0.00 | 64978 | NM_032478 |
| Hs.308332 \|\| RPL10L \|\| Ribosomal protein L10-like (1) | Hs.308332 | 1.58 | 0.95 | 140801 | BU561557 |
| Hs.103561 \|\| ARL6IP4 \|\| ADP-ribosylation-like factor 6 interacting protein 4 (1) | Hs.103561 | 1.58 | 0.95 | 51329 | BM907906 |
| \|\| \|\| \|\| \|\| N39101 \|\| \|\| \|\| \|\| 109162 | 276519 | 1.57 | 1.43 | | N39101 |
| Hs.82848 \|\| SELL \|\| Selectin L (lymphocyte adhesion molecule 1) (1) | Hs.82848 | 1.57 | 0.50 | 6402 | BC142711 |
| Hs.509163 \|\| ERGIC1 \|\| Endoplasmic reticulum-golgi intermediate compartment (ERGIC) 1 (1) | Hs.509163 | 1.57 | 1.24 | 57222 | BX647892 |
| Hs.129581 \|\| \|\| Transcribed locus (1) | Hs.129581 | 1.57 | 2.02 | | BX093286 |
| Hs.592338 \|\| TYMS \|\| Thymidylate synthetase (1) | Hs.592338 | 1.57 | 1.75 | 7298 | BQ056428 |
| Hs.432503 \|\| UBXD3 \|\| UBX domain containing 3 (1) | Hs.432503 | 1.56 | 0.95 | 127733 | BX648631 |
| Hs.635296 \|\| \|\| Transcribed locus (1) | Hs.635296 | 1.56 | 0.95 | | AW205487 |
| Hs.117210 \|\| \|\| Transcribed locus (1) | Hs.117210 | 1.56 | 4.01 | | BX110079 |
| \|\| \|\| *mitoch. cont. ESTs \|\| \|\| \|\| \|\| \|\| \|\| 433346 | *mitoch. cont. 511605 | 1.56 | 0.72 | | |
| \|\| \|\| \|\| \|\| AI251604 \|\| \|\| \|\| \|\| 116549 | 1985022 | 1.55 | 1.43 | | AI251604 |
| \|\| \|\| *mitoch. cont. ESTs \|\| \|\| \|\| \|\| \|\| \|\| 433369 | *mitoch. cont. 841448 | 1.55 | 2.61 | | |
| Hs.90443 \|\| NDUFS8 \|\| NADH dehydrogenase (ubiquinone) Fe-S protein 8, 23kDa (NADH-coenzyme Q reductase) (1) | Hs.90443 | 1.55 | 4.44 | 4728 | AK002110 |
| Hs.9234 \|\| TMEM147 \|\| Transmembrane protein 147 (1) | Hs.9234 | 1.55 | 0.00 | 10430 | CR603882 |
| Hs.61188 \|\| XRCC6BP1 \|\| XRCC6 binding protein 1 (1) | Hs.61188 | 1.55 | 2.98 | 91419 | AF078164 |
| Hs.356578 \|\| MRPL54 \|\| Mitochondrial ribosomal protein L54 (1) | Hs.356578 | 1.54 | 0.95 | 116541 | BM920672 |
| \|\| \|\| \|\| \|\| AA131607 \|\| \|\| \|\| \|\| 109977 | 503864 | 1.54 | 0.50 | | AA131607 |
| Hs.12106 \|\| MMAB \|\| Methylmalonic aciduria (cobalamin deficiency) cblB type (1) | Hs.12106 | 1.54 | 3.52 | 326625 | NM_052845 |
| Hs.661374 \|\| \|\| Transcribed locus (1) | Hs.661374 | 1.54 | 0.50 | | CD518596 |
| Hs.540469 \|\| \|\| Transcribed locus (1) | Hs.540469 | 1.54 | 3.52 | | N71076 |
| Hs.694996 \|\| LOC283345 \|\| RPL13-2 pseudogene (1) | Hs.694996 | 1.54 | 0.89 | 283345 | BQ128378 |
| Hs.656255 \|\| KIAA0114 \|\| KIAA0114 (1) | Hs.656255 | 1.53 | 1.43 | 57291 | BG035218 |
| Hs.517969 \|\| APEH \|\| N-acylaminoacyl-peptide hydrolase (1) | Hs.517969 | 1.53 | 0.00 | 327 | BC000362 |
| Hs.1501 \|\| SDC2 \|\| Syndecan 2 (1) | Hs.1501 | 1.53 | 0.37 | 6383 | BC030133 |
| Hs.574757 \|\| \|\| Transcribed locus (1) | Hs.574757 | 1.53 | 0.50 | | AI792253 |
| Hs.103106 \|\| LSM2 \|\| LSM2 homolog, U6 small nuclear RNA associated (S. cerevisiae) (1) | Hs.103106 | 1.52 | 2.15 | 57819 | BM799638 |
| Hs.44298 \|\| MRPS17 \|\| Mitochondrial ribosomal protein S17 (1) | Hs.44298 | 1.52 | 2.15 | 51373 | AK026553 |
| Hs.512544 \|\| \|\| CDNA clone 5267715 (1) | Hs.512544 | 1.52 | 1.24 | | BC036215 |
| Hs.655145 \|\| \|\| CDNA clone 30318308 (1) | Hs.655145 | 1.51 | 4.88 | | BC070327 |
| Hs.528993 \|\| RALBP1 \|\| RalA binding protein 1 (1) | Hs.528993 | 1.51 | 0.00 | 10928 | NM_006788 |
| Hs.279915 \|\| TIMM8B \|\| Translocase of inner mitochondrial membrane 8 homolog B (yeast) (1) | Hs.279915 | 1.51 | 4.44 | 26521 | BM920842 |
| Hs.661894 \|\| LOC728953 \|\| Similar to ribosomal protein S19 (1) | Hs.661894 | 1.51 | 0.00 | 728953 | CD050805 |
| Hs.409065 \|\| FEN1 \|\| Flap structure-specific endonuclease 1 (1) | Hs.409065 | 1.51 | 2.61 | 2237 | NM_004111 |
| Hs.603077 \|\| \|\| Transcribed locus (1) | Hs.603077 | 1.51 | 0.95 | | AL600535 |
| Hs.512610 \|\| LSM7 \|\| LSM7 homolog, U6 small nuclear RNA associated (S. cerevisiae) (1) | Hs.512610 | 1.51 | 4.88 | 51690 | AK127573 |
| Hs.659093 \|\| \|\| Transcribed locus (1) | Hs.659093 | 1.51 | 0.95 | | AI546979 |
| Hs.534404 \|\| RPL10 \|\| Ribosomal protein L10 (1) | Hs.534404 | 1.51 | 1.24 | 6134 | NM_006013 |
| Hs.585105 \|\| C19orf54 \|\| Chromosome 19 open reading frame 54 (1) | Hs.585105 | 1.50 | 2.02 | 284325 | NM_198476 |
| \|\| \|\| \|\| \|\| W90748 \|\| \|\| \|\| \|\| 118422 | 418297 | 1.50 | 2.15 | | W90748 |
| \|\| \|\| \|\| \|\| AI082622 \|\| \|\| \|\| \|\| 313463 | 1660570 | 1.50 | 0.37 | | AI082622 |
| \|\| \|\| \|\| \|\| N79496 \|\| \|\| \|\| \|\| 120418 | 301364 | 1.50 | 1.67 | | N79496 |
| Hs.306083 \|\| C22orf32 \|\| Chromosome 22 open reading frame 32 (1) | Hs.306083 | 1.50 | 2.61 | 91689 | AK095636 |
| \|\| \|\| \|\| \|\| AI090818 \|\| \|\| \|\| \|\| 314567 | 1692237 | 1.50 | 0.95 | | AI090818 |
| Hs.130204 \|\| \|\| Transcribed locus (1) | Hs.130204 | 1.49 | 0.50 | | AW874683 |
| Hs.303116 \|\| SDF2L1 \|\| Stromal cell-derived factor 2-like 1 (1) | Hs.303116 | 1.49 | 2.02 | 23753 | BU588938 |
| Hs.543417 \|\| \|\| Transcribed locus (1) | Hs.543417 | 1.49 | 4.01 | | AI304357 |
| Hs.145566 \|\| \|\| Transcribed locus (1) | Hs.145566 | 1.49 | 1.24 | | AI793042 |
| Hs.631630 \|\| PRKACA \|\| Protein kinase, cAMP- dependent, catalytic, alpha (1) | Hs.631630 | 1.49 | 3.52 | 5566 | AK131561 |
| Hs.12393 \|\| TGDS \|\| TDP-glucose 4,6-dehydratase (1) | Hs.12393 | 1.49 | 1.24 | 23483 | NM_014305 |
| Hs.441124 \|\| APOBEC3C \|\| Apolipoprotein B mRNA editing enzyme, catalytic polypeptide-like 3C (1) | Hs.441124 | 1.49 | 1.67 | 27350 | AK094570 |
| Hs.105606 \|\| TMEM160 \|\| Transmembrane protein 160 (1) | Hs.105606 | 1.49 | 1.24 | 54958 | BQ673305 |
| Hs.154510 \|\| CBR3 \|\| Carbonyl reductase 3 (1) | Hs.154510 | 1.49 | 0.50 | 874 | AB041012 |
| \|\| \|\| \|\| \|\| AA629827 \|\| \|\| \|\| \|\| 101550 | 884590 | 1.49 | 0.00 | | AA629827 |
| Hs.504620 \|\| PHB2 \|\| Prohibitin 2 (1) | Hs.504620 | 1.48 | 2.02 | 11331 | BF204697 |
| Hs.198003 \|\| SARDH \|\| Sarcosine dehydrogenase (1) | Hs.198003 | 1.48 | 2.61 | 1757 | NM_007101 |
| Hs.666437 \|\| \|\| **Transcribed locus (1) | Hs.666437 | 1.48 | 0.37 | | |
| Hs.517155 \|\| TMEPAI \|\| Transmembrane, prostate androgen induced RNA (3) | Hs.517155 | 1.48 | 4.44 | 56937 | NM_020182 |
| Hs.526817 \|\| \|\| Transcribed locus (1) | Hs.526817 | 1.48 | 0.00 | | BG194040 |
| Hs.587092 \|\| \|\| MRNA; cDNA DKFZp686P18215 (from clone DKFZp686P18215) (1) | Hs.587092 | 1.48 | 2.15 | | BX538202 |
| \|\| \|\| \|\| \|\| AI254145 \|\| \|\| \|\| \|\| 112264 | 1985835 | 1.48 | 1.67 | | AI254145 |
| Hs.127401 \|\| IFT172 \|\| Intraflagellar transport 172 homolog (Chlamydomonas) (1) | Hs.127401 | 1.48 | 0.95 | 26160 | NM_015662 |
| Hs.600905 \|\| \|\| Transcribed locus (1) | Hs.600905 | 1.48 | 1.24 | | AA669226 |
| Hs.387208 \|\| FAU \|\| Finkel-Biskis-Reilly murine sarcoma virus (FBR-MuSV) ubiquitously expressed (fox derived); ribosomal protein S30 (2) | Hs.387208 | 1.48 | 2.02 | 2197 | BC051834 |
| Hs.655949 \|\| RP3-377H14.5 \|\| Hypothetical protein FLJ35429 (1) | Hs.655949 | 1.47 | 3.52 | 285830 | AL832418 |
| Hs.477115 \|\| ABHD10 \|\| Abhydrolase domain containing 10 (1) | Hs.477115 | 1.47 | 4.01 | | CA419362 |
| Hs.661380 \|\| \|\| Transcribed locus (1) | Hs.661380 | 1.47 | 0.72 | | DB300749 |
| Hs.353163 \|\| TMEM99 \|\| Transmembrane protein 99 (1) | Hs.353163 | 1.47 | 1.75 | 147184 | NM_145274 |
| Hs.461722 \|\| TRAPPC2L \|\| Trafficking protein particle complex 2-like (1) | Hs.461722 | 1.47 | 0.95 | 51693 | AK126779 |
| Hs.520070 \|\| CUTA \|\| CutA divalent cation tolerance homolog (E. coli) (2) | Hs.520070 | 1.47 | 0.95 | 51596 | BM913222 |
| Hs.464734 \|\| SNRPD1 \|\| Small nuclear ribonucleoprotein D1 polypeptide 16kDa (1) | Hs.464734 | 1.47 | 1.75 | 6632 | CR613811 |
| Hs.586423 \|\| EEF1A1 \|\| Eukaryotic translation elongation factor 1 alpha 1 (2) | Hs.586423 | 1.47 | 1.67 | 1915 | NM_001402 |
| Hs.44017 \|\| MRPL41 \|\| Mitochondrial ribosomal protein L41 (1) | Hs.44017 | 1.46 | 0.37 | 64975 | BQ955623 |
| Hs.198269 \|\| NDUFA3 \|\| NADH dehydrogenase (ubiquinone) 1 alpha subcomplex, 3, 9kDa (1) | Hs.198269 | 1.46 | 2.02 | 4696 | CD248715 |
| Hs.505104 \|\| KLHDC5 \|\| Kelch domain containing 5 (1) | Hs.505104 | 1.46 | 0.37 | 57542 | NM_020782 |
| Hs.504270 \|\| LOC387820 \|\| Similar to DnaJ (Hsp40) homolog, subfamily B, member 6 isoform a (1) | Hs.504270 | 1.46 | 4.44 | | BQ067277 |
| Hs.239500 \|\| MGC13114 \|\| Hypothetical protein MGC 13114 (1) | Hs.239500 | 1.46 | 0.95 | 84326 | AK057077 |
| Hs.7236 \|\| NOSIP \|\| Nitric oxide synthase interacting protein (1) | Hs.7236 | 1.46 | 0.95 | 51070 | BC047314 |
| Hs.439481 \|\| SUPT4H1 \|\| Suppressor of Ty 4 homolog 1 (S. cerevisiae) (1) | Hs.439481 | 1.46 | 1.67 | 6827 | BM543526 |
| Hs.655088 \|\| SAP30BP \|\| SAP30 binding protein (1) | Hs.655088 | 1.45 | 2.15 | 29115 | BX647066 |
| Hs.651950 \|\| NUF2 \|\| NUF2, NDC80 kinetochore complex component, homolog (S. cerevisiae) (2) | Hs.651950 | 1.45 | 1.75 | 83540 | NM_145697 |
| Hs.327631 \|\| RDH13 \|\| Retinol dehydrogenase 13 (all-trans/9-cis) (1) | Hs.327631 | 1.45 | 2.98 | 112724 | AK122764 |
| Hs.486304 \|\| \|\| Transcribed locus, strongly similar to XP_001091393.1 similar to 60S ribosomal protein L29 (Cell surface heparin-binding protein HIP) isoform 2 [Macaca mulatta] (1) | Hs.486304 | 1.45 | 0.95 | | BM904327 |
| Hs.643480 \|\| WDR54 \|\| WD repeat domain 54 (1) | Hs.643480 | 1.45 | 1.67 | 84058 | BM542003 |
| Hs.516632 \|\| DNAJC10 \|\| DnaJ (Hsp40) homolog, subfamily C, member 10 (3) | Hs.516632 | 1.45 | 0.00 | 54431 | AL832632 |
| Hs.655241 \|\| LOC339229 \|\| Hypothetical protein LOC339229 (1) | Hs.655241 | 1.45 | 2.02 | 339229 | AY007126 |
| Hs.521212 \|\| AKR1B1 \|\| Aldo-keto reductase family 1, member B1 (aldose reductase) (1) | Hs.521212 | 1.45 | 0.37 | 231 | CR610878 |
| \|\| \|\| \|\| \|\| \|\| \|\| \|\| \|\| 307748 | 449309 | 1.45 | 1.24 | | |
| Hs.658192 I \| C10orf4 \|\| Chromosome 10 open reading frame 4 (1) | Hs.658192 | 1.45 | 0.95 | 118924 | NM_145246 |
| Hs.370703 \|\| CRYL1 \|\| Crystallin, lambda 1 (1) | Hs.370703 | 1.45 | 0.95 | 51084 | BC071810 |
| Hs.694812 \|\| \|\| Transcribed locus, strongly similar to XP_001084658.1 similar to 60S ribosomal protein L23a [Macaca mulatta] (1) | Hs.694812 | 1.45 | 2.98 | | |
| Hs.279784 \|\| PREB \|\| Prolactin regulatory element binding (1) | Hs.279784 | 1.44 | 1.43 | 10113 | BC036185 |
| Hs.193832 \|\| GPATCH4 \|\| G patch domain containing 4 (1) | Hs.193832 | 1.44 | 1.75 | 54865 | NM_182679 |
| \|\| \|\| \|\| \|\| AA598678 \|\| \|\| \|\| \|\| 103752 | 898257 | 1.44 | 0.95 | | AI821728 |
| Hs.311190 \|\| MRPL14 \|\| Mitochondrial ribosomal protein L14 (1) | Hs.311190 | 1.44 | 4.44 | 64928 | BM910935 |
| Hs.101337 \|\| UCP3 \|\| Uncoupling protein 3 (mitochondrial, proton carrier) (1) | Hs.101337 | 1.44 | 3.52 | 7352 | NM_003356 |
| \|\| \|\| \|\| \|\| AI251374 \|\| \|\| \|\| \|\| 100437 | 1985603 | 1.44 | 0.50 | | AI251374 |
| Hs.356187 \|\| LOC51255 \|\| Hypothetical protein LOC51255 (1) | Hs.356187 | 1.44 | 0.95 | 51255 | CD242398 |
| Hs.195155 \|\| SLC38A5 \|\| Solute carrier family 38, member 5 (1) | Hs.195155 | 1.44 | 0.95 | 92745 | AF276889 |
| Hs.654405 \|\| HLA-DRB1 \|\| Major histocompatibility complex, class II, DR beta 1 (6) | Hs.654405 | 1.44 | 2.02 | | AK225690 |
| Hs.331491 \|\| WDR21A \|\| WD repeat domain 21A (1) | Hs.331491 | 1.44 | 0.37 | 26094 | AL080157 |
| Hs.654406 \|\| HLA-DRB4 \|\| Major histocompatibility complex, class II, DR beta 4 (1) | Hs.654406 | 1.44 | 2.98 | | BF342032 |
| \|\| \|\| *mitoch. cont. EST \|\| \|\| \|\| \|\| \|\| \|\| 433310 | *mitoch. cont. 1169042 | 1.44 | 2.15 | | |
| Hs.241543 \|\| POLDIP2 \|\| Polymerase (DNA-directed), delta interacting protein 2 (1) | Hs.241543 | 1.43 | 4.88 | 26073 | NM_015584 |
| Hs.109358 \|\| ATP10B \|\| ATPase, Class V, type 10B (1) | Hs.109358 | 1.43 | 2.98 | 23120 | NM_025153 |
| Hs.239 \|\| FOXM1 \|\| Forkhead box M1 (1) | Hs.239 | 1.43 | 4.01 | 2305 | NM_202002 |
| Hs.638652 \|\| LOC92755 \|\| **Hypothetical gene LOC92755 (1) | Hs.638652 | 1.43 | 2.98 | | BM479284 |
| Hs.266331 \|\| FCRLA \|\| Fc receptor-like A (1) | Hs.266331 | 1.43 | 2.61 | 84824 | BX649184 |
| Hs.483238 \|\| ARHGAP29 \|\| Rho GTPase activating protein 29 (1) | Hs.483238 | 1.43 | 0.50 | 9411 | NM_004815 |
| Hs.446270 \|\| DHX36 \|\| DEAH (Asp-Glu-Ala-His) box polypeptide 36 (2) | Hs.446270 | 1.43 | 1.67 | 170506 | NM_020865 |
| Hs.592084 \|\| NUDT16L1 \|\| Nudix (nucleoside diphosphate linked moiety X)-type motif 16-like 1 (1) | Hs.592084 | 1.43 | 2.61 | 84309 | BQ679635 |
| Hs.499484 \|\| LOC728509 \|\| **Similar to ribosomal protein S19 (1) | Hs.499484 | 1.42 | 0.72 | 728509 | BU536723 |
| Hs.528320 \|\| APOA1BP \|\| Apolipoprotein A-I binding protein (1) | Hs.528320 | 1.42 | 2.15 | 128240 | AK123518 |
| Hs.368554 \|\| RNF121 \|\| Ring finger protein 121 (2) | Hs.368554 | 1.42 | 1.75 | 55298 | AK094508 |
| \|\| \|\| \|\| \|\| \|\| \|\| \|\| \|\| 472862 | KR:4 | 1.42 | 1.75 | | |
| Hs.530735 \|\| MS4A7 \|\| Membrane-spanning 4-domains, subfamily A, member 7 (2) | Hs.530735 | 1.42 | 2.15 | 58475 | NM_021201 |
| \|\| \|\| \|\| \|\| AI251235 \|\| \|\| \|\| \|\| 110301 | 1985520 | 1.42 | 0.89 | | AI251235 |
| Hs.666233 \|\| \|\| Transcribed locus, weakly similar to XP_001103164.1 similar to 60S ribosomal protein L12 [Macaca mulatta] (1) | Hs.666233 | 1.42 | 2.98 | | BX103731 |
| Hs.189716 \|\| NDUFAB1 \|\| NADH dehydrogenase (ubiquinone) 1, alpha/beta subcomplex, 1, 8kDa (1) | Hs.189716 | 1.42 | 2.61 | 4706 | BQ646109 |
| \|\| \|\| \|\| \|\| \|\| \|\| \|\| \|\| 309043 | 449329 | 1.42 | 2.15 | | |
| Hs.436913 \|\| CHCHD7 \|\| Coiled-coil-helix-coiled-coil-helix domain containing 7 (1) | Hs.436913 | 1.42 | 3.52 | 79145 | AK098285 |
| Hs.446852 \|\| EIF3S6IP \|\| Eukaryotic translation initiation factor 3, subunit 6 interacting protein (1) | Hs.446852 | 1.42 | 4.88 | 51386 | AK056129 |
| Hs.180312 \|\| MRPS16 \|\| Mitochondrial ribosomal protein S16 (3) | Hs.180312 | 1.42 | 0.72 | 51021 | NM_016065 |
| Hs.150064 \|\| \|\| MRNA; cDNA DKFZp781N0753 (from clone DKFZp781N0753) (1) | Hs.150064 | 1.42 | 0.50 | | CR627225 |
| Hs.371372 \|\| SDCCAG10 \|\| Serologically defined colon cancer antigen 10 (1) | Hs.371372 | 1.42 | 0.95 | 10283 | BX647971 |
| Hs.545361 \|\| \|\| Transcribed locus (1) | Hs.545361 | 1.41 | 1.75 | | AV699312 |
| Hs.189119 \|\| CXXC5 \|\| CXXC finger 5 (1) | Hs.189119 | 1.41 | 1.43 | 51523 | BC006428 |
| \|\| \|\| \|\| \|\| AA923631 \|\| \|\| \|\| \|\| 109579 | 1160370 | 1.41 | 0.89 | | AA923631 |
| Hs.664708 \|\| \|\| Transcribed locus (2) | Hs.664708 | 1.41 | 0.00 | | AW975319 |
| Hs.658502 \|\| \|\| Full length insert cDNA YU36C09 (1) | Hs.658502 | 1.41 | 0.37 | | BX114025 |
| Hs.387679 \|\| HLA-DQA1 \|\| Major histocompatibility complex, class II, DQ alpha 1 (3) | Hs.387679 | 1.41 | 1.67 | 3117 | AB209628 |
| Hs.654525 \|\| POU2AF1 \|\| POU domain, class 2, associating factor 1 (2) | Hs.654525 | 1.41 | 3.52 | 5450 | Z49194 |
| Hs.436617 \|\| CCDC5 \|\| Coiled-coil domain containing 5 (spindle associated) (1) | Hs.436617 | 1.41 | 2.61 | 115106 | BM799713 |
| Hs.390667 \|\| GSTK1 \|\| Glutathione S-transferase kappa 1 (1) | Hs.390667 | 1.41 | 2.61 | 373156 | BM912672 |
| Hs.3426 \|\| ERAL1 \|\| Era G-protein-like 1 (E. coli) (1) | Hs.3426 | 1.41 | 1.67 | 26284 | BX537542 |
| Hs.15977 \|\| NDUFB9 \|\| NADH dehydrogenase (ubiquinone) 1 beta subcomplex, 9, 22kDa (1) | Hs.15977 | 1.41 | 4.44 | 4715 | BU180150 |
| Hs.514435 \|\| SF3B3 \|\| Splicing factor 3b, subunit 3, 130kDa (1) | Hs.514435 | 1.41 | 0.37 | 23450 | D13642 |
| Hs.461113 \|\| CIRH1A \|\| Cirrhosis, autosomal recessive 1A (cirhin) (2) | Hs.461113 | 1.41 | 0.37 | 84916 | NM_032830 |
| Hs.523463 \|\| RPL27A \|\| Ribosomal protein L27a (3) | Hs.523463 | 1.41 | 2.02 | 6157 | NM_000990 |
| Hs.445162 \|\| BTLA \|\| Band T lymphocyte associated (1) | Hs.445162 | 1.41 | 1.75 | 151888 | NM_181780 |
| Hs.659436 \|\| \|\| Transcribed locus (1) | Hs.659436 | 1.41 | 4.01 | | CX760743 |
| Hs.431460 \|\| ICAM2 \|\| Intercellular adhesion molecule 2 (1) | Hs.431460 | 1.40 | 0.95 | 3384 | BQ214437 |
| Hs.528222 \|\| NDUFS4 \|\| NADH dehydrogenase (ubiquinone) Fe-S protein 4, 18kDa (NADH-coenzyme Q reductase) (1) | Hs.528222 | 1.40 | 3.52 | 4724 | AB062482 |
| Hs.435981 \|\| ERCC1 \|\| Excision repair cross- complementing rodent repair deficiency, complementation group 1 (includes overlapping antisense sequence) (1) | Hs.435981 | 1.40 | 0.89 | 2067 | AK092039 |
| \|\| \|\| \|\| \|\| AA883418 \|\| \|\| \|\| \|\| 314549 | 1467816 | 1.40 | 1.67 | | AA883418 |
| Hs.655660 \|\| RIC3 \|\| Resistance to inhibitors of cholinesterase 3 homolog (C. elegans) (1) | Hs.655660 | 1.40 | 0.95 | 79608 | AL832601 |
| \|\| \|\| \|\| \|\| AA669558 \|\| \|\| \|\| \|\| 108962 | 856804 | 1.40 | 0.95 | | AA669558 |
| Hs.465543 \|\| BTBD2 \|\| BTB (POZ) domain containing 2 (1) | Hs.465543 | 1.40 | 4.01 | 55643 | AK056818 |
| Hs.571886 \|\| AKR7A2 \|\| Aldo-keto reductase family 7, member A2 (aflatoxin aldehyde reductase) (1) | Hs.571886 | 1.40 | 2.15 | 8574 | NM_003689 |
| \|\| \|\| \|\| \|\| AA629529 \|\| \|\| \|\| \|\| 225133 | 884335 | 1.40 | 1.67 | | AA629529 |
| Hs.492716 \|\| WDR67 \|\| WD repeat domain 67 (1) | Hs.492716 | 1.40 | 1.75 | 93594 | AK056434 |
| Hs.69499 \|\| TRIAP1 \|\| TP53 regulated inhibitor of apoptosis 1 (1) | Hs.69499 | 1.40 | 2.02 | 51499 | BM910747 |
| \|\| \|\| \|\| \|\| \|\| \|\| \|\| \|\| 472870 | KR:10 | 1.40 | 2.15 | | |
| Hs.518236 \|\| SEC61A1 \|\| Sec61 alpha 1 subunit (S. cerevisiae) (2) | Hs.518236 | 1.40 | 2.15 | 29927 | NM_013336 |
| Hs.132519 \|\| FLJ39660 \|\| Hypothetical protein FLJ39660 (1) | Hs.132519 | 1.40 | 0.37 | 284992 | NM_00108053 9 |
| Hs.356331 \|\| PPIA \|\| Peptidylprolyl isomerase A (cyclophilin A) (1) | Hs.356331 | 1.40 | 3.52 | 5478 | NM_021130 |
| Hs.591229 \|\| DLEU1 \|\| **Deleted in lymphocytic leukemia, 1 (1) | Hs.591229 | 1.40 | 0.50 | 10301 | AK095311 |
| Hs.265829 \|\| ITGA3 \|\| Integrin, alpha 3 (antigen CD49C, alpha 3 subunit of VLA-3 receptor) (2) | Hs.265829 | 1.40 | 4.01 | 3675 | NM_002204 |
| \|\| \|\| *mitoch. cont. ESTs \|\| \|\| \|\| \|\| \|\| \|\| 433372 | *mitoch. cont. 916506 | 1.40 | 0.72 | | |
| Hs.75056 \|\| TIMM13 \|\| Translocase of inner mitochondrial membrane 13 homolog (yeast) (1) | Hs.75056 | 1.40 | 1.67 | 26517 | NM_012458 |
| Hs.170310 \|\| CECR1 \|\| Cat eye syndrome chromosome region, candidate 1 (2) | Hs.170310 | 1.40 | 2.15 | 51816 | NM_017424 |
| Hs.657046 \|\| KCNT2 \|\| Potassium channel, subfamily T, member 2 (1) | Hs.657046 | 1.40 | 0.95 | 343450 | BX647852 |
| Hs.127054 \|\| \|\| Transcribed locus (1) | Hs.127054 | 1.40 | 1.24 | | DN913748 |
| Hs.528385 \|\| DHRS4 \|\| Dehydrogenase/reductase (SDR family) member 4 (1) | Hs.528385 | 1.40 | 0.95 | 10901 | BM919053 |
| \|\| \|\| \|\| \|\| \|\| \|\| \|\| \|\| 310180 | 449403 | 1.40 | 1.24 | | |
| \|\| \|\| \|\| \|\| AI298070 \|\| \|\| \|\| \|\| 311495 | 1896493 | 1.40 | 2.61 | | BX092992 |
| \|\| \|\| \|\| \|\| AA865709 \|\| \|\| \|\| \|\| 317307 | 1469142 | 1.40 | 0.50 | | AA865709 |
| Hs.444536 \|\| SLC5A8 \|\| Solute carrier family 5 (iodide transporter), member 8 (1) | Hs.444536 | 1.39 | 4.44 | 160728 | AF536216 |
| Hs.653258 \|\| MGC11257 \|\| Hypothetical protein MGC11257 (1) | Hs.653258 | 1.39 | 2.98 | 84310 | BC025971 |
| Hs.645604 \|\| \|\| Clone 121687 mRNA sequence (1) | Hs.645604 | 1.39 | 2.98 | | |
| Hs.658289 \|\| RHOC \|\| Ras homolog gene family, member C (1) | Hs.658289 | 1.39 | 0.95 | 389 | B1000596 |
| \|\| \|\| \|\| \|\| AA788600 \|\| \|\| \|\| \|\| 101697 | 1127206 | 1.39 | 3.52 | | AA788600 |
| Hs.418233 \|\| MRPL24 \|\| Mitochondrial ribosomal protein L24 (1) | Hs.418233 | 1.39 | 0.72 | 79590 | BQ883105 |
| Hs.657019 \|\| \|\| Transcribed locus, weakly similar to XP_001110088.1 similar to steroid 5 alpha-reductase 2-like 2 [Macaca mulatta] (1) | Hs.657019 | 1.39 | 0.50 | | BX363410 |
| Hs.95990 \|\| PKLR \|\| Pyruvate kinase, liver and RBC (1) | Hs.95990 | 1.39 | 0.89 | 5313 | M15465 |
| Hs.529059 \|\| EIF3S4 \|\| Eukaryotic translation initiation factor 3, subunit 4 delta, 44kDa (1) | Hs.529059 | 1.39 | 1.75 | 8666 | CR613864 |
| \|\| \|\| *mitoch. cont. \|\| \|\| \|\| \|\| \|\| \|\| 433371 | *mitoch. cont. 915948 | 1.39 | 4.01 | | |
| Hs.655961 \|\| \|\| MRNA full length insert cDNA clone EUROIMAGE 113222 (1) | Hs.655961 | 1.39 | 4.88 | | AL360140 |
| Hs.693723 \|\| CCDC88C \|\| Coiled-coil domain containing 88C (1) | Hs.693723 | 1.39 | 3.52 | 440193 | NM_00108041 4 |
| Hs.58685 \|\| CD5 \|\| CD5 molecule (1) | Hs.58685 | 1.39 | 0.95 | 921 | NM_014207 |
| \|\| \|\| \|\| \|\| AA988876 \|\| \|\| \|\| \|\| 116043 | 1602730 | 1.39 | 4.44 | | AA988876 |
| Hs.438429 \|\| RPS19 \|\| Ribosomal protein S19 (1) | Hs.438429 | 1.39 | 2.15 | 6223 | BQ066037 |
| Hs.91579 \|\| IMP4 \|\| IMP4, U3 small nucleolar ribonucleoprotein, homolog (yeast) (1) | Hs.91579 | 1.39 | 1.43 | 92856 | CR619192 |
| \|\| \|\| \|\| \|\| \|\| \|\| \|\| \|\| 306817 | 449283 | 1.39 | 1.43 | | |
| Hs.9661 \|\| PSMB10 \|\| Proteasome (prosome, macropain) subunit, beta type, 10 (2) | Hs.9661 | 1.39 | 0.95 | 5699 | BM906146 |
| Hs.513147 \|\| SLC7A7 \|\| Solute carrier family 7 (cationic amino acid transporter, y+ system), member 7 (2) | Hs.513147 | 1.39 | 4.01 | 9056 | AB209591 |
| Hs.439991 \|\| TMEM177 \|\| Transmembrane protein 177 (1) | Hs.439991 | 1.39 | 4.44 | 80775 | AK057313 |
| Hs.335355 \|\| SLC44A4 \|\| Solute carrier family 44, member 4 (1) | Hs.335355 | 1.39 | 2.15 | 80736 | NM_025257 |
| Hs.191734 \|\| MGST3 \|\| Microsomal glutathione S-transferase 3 (1) | Hs.191734 | 1.38 | 1.43 | 4259 | BX537737 |
| Hs.430075 \|\| COX7C \|\| Cytochrome c oxidase subunit VIIc (1) | Hs.430075 | 1.38 | 3.52 | 1350 | CR599916 |
| Hs.632235 \|\| SHBG \|\| Sex hormone-binding globulin (1) | Hs.632235 | 1.38 | 2.61 | 6462 | CD013956 |
| \|\| \|\| *mitoch. cont. cellular retinoic acid-binding protein 2 \|\| \|\| \|\| \|\| \|\| \|\| 433344 | *mitoch. cont. 484576 | 1.38 | 2.15 | | |
| Hs.668533 \|\| \|\| Transcribed locus (1) | Hs.668533 | 1.38 | 0.95 | | AI347542 |
| Hs.667739 \|\| \|\| Transcribed locus (1) | Hs.667739 | 1.38 | 2.98 | | AI792494 |
| Hs.532840 \|\| C19orf25 \|\| Chromosome 19 open reading frame 25 (1) | Hs.532840 | 1.38 | 1.75 | 148223 | AK056004 |
| Hs.430299 \|\| PXMP2 \|\| Peroxisomal membrane protein 2, 22kDa (1) | Hs.430299 | 1.38 | 4.88 | 5827 | BM454192 |
| Hs.25590 \|\| STC1 \|\| Stanniocalcin 1 (1) | Hs.25590 | 1.38 | 2.61 | 6781 | U25997 |
| \|\| \|\| \|\| \|\| \|\| \|\| \|\| \|\| 472864 | KR:9 | 1.38 | 2.61 | | |
| Hs.633942 \|\| \|\| CDNA FLJ26232 fis, clone ADG09495 (2) | Hs.633942 | 1.38 | 2.15 | | AK129743 |
| Hs.428 \|\| FLT3LG \|\| Fms-related tyrosine kinase 3 ligand (2) | Hs.428 | 1.38 | 3.52 | 2323 | CR609170 |
| Hs.654884 \|\| \|\| Transcribed locus (1) | Hs.654884 | 1.38 | 2.15 | | BG939675 |
| Hs.657657 \|\| \|\| Transcribed locus (1) | Hs.657657 | 1.38 | 2.61 | | BX093986 |
| Hs.355307 \|\| CD27 \|\| CD27 molecule (1) | Hs.355307 | 1.38 | 2.61 | 939 | BC012160 |
| Hs.666639 \|\| LOC646808 \|\| Hypothetical LOC646808 (1) | Hs.666639 | 1.38 | 2.61 | | AI421806 |
| Hs.654425 \|\| UMOD \|\| Uromodulin (uromucoid, Tamm-Horsfall glycoprotein) (1) | Hs.654425 | 1.38 | 1.75 | 7369 | AK091961 |
| Hs.568109 \|\| LOC646282 \|\| Similar to alpha-2-glycoprotein 1, zinc (1) | Hs.568109 | 1.38 | 4.01 | | AK307369 |
| Hs.469060 \|\| POLE4 \|\| Polymerase (DNA-directed), epsilon 4 (p12 subunit) (1) | Hs.469060 | 1.38 | 1.75 | 56655 | BM924454 |
| Hs.517502 \|\| MIAT \|\| Myocardial infarction associated transcript (non-protein coding) (2) | Hs.517502 | 1.38 | 0.72 | 100129936 | AK124820 |
| Hs.485130 \|\| HLA-DPB1 \|\| Major histocompatibility complex, class II, DP beta 1 (1) | Hs.485130 | 1.38 | 4.44 | 3115 | BC013184 |
| Hs.406982 \|\| BEYLA \|\| Hypothetical LOC497634 (1) | Hs.406982 | 1.38 | 4.88 | 497634 | AK097475 |
| \|\| \|\| \|\| \|\| \|\| \|\| \|\| \|\| 472872 | KR:8 | 1.38 | 0.95 | | |
| Hs.549079 \|\| LOC442578 \|\| **Similar to Cohesin subunit SA-3 (Stromal antigen 3) (Stromalin 3) (SCC3 homolog 3) (1) | Hs.549079 | 1.37 | 2.61 | | DR033752 |
| \|\| \|\| \|\| \|\| \|\| \|\| \|\| \|\| 306807 | 449510 | 1.37 | 1.43 | | |
| Hs.76884 \|\| ID3 \|\| Inhibitor of DNA binding 3, dominant negative helix-loop-helix protein (1) | Hs.76884 | 1.37 | 2.02 | 3399 | AK225965 |
| Hs.249196 \|\| DLX6 \|\| Distal-less homeobox 6 (1) | Hs.249196 | 1.37 | 1.75 | 1750 | NM_005222 |
| Hs.148272 \|\| CCM2 \|\| Cerebral cavernous malformation 2 (1) | Hs.148272 | 1.37 | 2.98 | 83605 | BC063663 |
| Hs.659857 \|\| EID3 \|\| EP300 interacting inhibitor of differentiation 3 (1) | Hs.659857 | 1.37 | 4.88 | 493861 | AK098698 |
| \|\| \|\| \|\| \|\| AI093359 \|\| \|\| \|\| \|\| 315840 | 1695115 | 1.37 | 2.15 | | AI093359 |
| Hs.472024 \|\| C20orf30 \|\| Chromosome 20 open reading frame 30 (2) | Hs.472024 | 1.37 | 0.37 | 29058 | NM_00100992 4 |
| Hs.513126 \|\| FANCI \|\| Fanconi anemia, complementation group I (1) | Hs.513126 | 1.37 | 0.95 | 55215 | BC146804 |
| Hs.519601 \|\| ID4 \|\| Inhibitor of DNA binding 4, dominant negative helix-loop-helix protein (1) | Hs.519601 | 1.37 | 4.44 | 3400 | NM_001546 |
| Hs.20521 \|\| PRMT1 \|\| Protein arginine methyltransferase 1 (1) | Hs.20521 | 1.37 | 0.72 | 3276 | CR622298 |
| Hs.412418 \|\| LOC153328 \|\| Similar to CG4995 gene product (2) | Hs.412418 | 1.37 | 2.15 | | BG215411 |
| \|\| \|\| \|\| \|\| AA629356 \|\| \|\| \|\| \|\| 224222 | 743729 | 1.37 | 2.61 | | AA629356 |
| \|\| \|\| \|\| \|\| AA531182 \|\| \|\| \|\| \|\| 101655 | 995732 | 1.37 | 4.01 | | AA531182 |
| Hs.591456 \|\| POLR3GL \|\| Polymerase (RNA) III (DNA directed) polypeptide G (32kD)-like (2) | Hs.591456 | 1.37 | 1.43 | 84265 | BC050418 |
| Hs.411865 \|\| IPO4 \|\| Importin 4 (1) | Hs.411865 | 1.37 | 4.01 | 79711 | AK094897 |
| Hs.432329 \|\| PLXNA1 \|\| Plexin A1 (1) | Hs.432329 | 1.36 | 4.44 | 5361 | NM_032242 |
| Hs.666889 \|\| \|\| Transcribed locus (1) | Hs.666889 | 1.36 | 2.61 | | AI792585 |
| Hs.535464 \|\| LOC728689 \|\| Similar to eukaryotic translation initiation factor 3, subunit 8, 110kDa (1) | Hs.535464 | 1.36 | 1.75 | 728689 | NM_00109966 1 |
| Hs.213470 \|\| PSMB7 \|\| Proteasome (prosome, macropain) subunit, beta type, 7 (2) | Hs.213470 | 1.36 | 4.01 | 5695 | AK308756 |
| Hs.495240 \|\| WDR34 \|\| WD repeat domain 34 (1) | Hs.495240 | 1.36 | 4.88 | 89891 | BM479044 |
| Hs.122186 \|\| \|\| Transcribed locus (1) | Hs.122186 | 1.36 | 1.67 | 4868 | NM_004646 |
| Hs.390221 \|\| TEX14 \|\| Testis expressed 14 (1) | Hs.390221 | 1.36 | 2.61 | 56155 | AL834143 |
| Hs.563801 \|\| \|\| Transcribed locus (1) | Hs.563801 | 1.36 | 4.01 | | AI088903 |
| Hs.9999 \|\| EMP3 \|\| Epithelial membrane protein 3 (1) | Hs.9999 | 1.36 | 2.98 | 2014 | BM556279 |
| Hs.356626 \|\| P117 \|\| Hypothetical protein P117 (1) | Hs.356626 | 1.36 | 2.02 | 125988 | BM563199 |
| Hs.655161 \|\| PLSCR3 \|\| Phospholipid scramblase 3 (1) | Hs.655161 | 1.36 | 3.52 | 57048 | AF289602 |
| \|\| \|\| \|\| \|\| \|\| \|\| \|\| \|\| 307421 | 449376 | 1.36 | 1.75 | | |
| \|\| \|\| \|\| \|\| \|\| \|\| \|\| \|\| 310693 | 449275 | 1.36 | 2.61 | | |
| Hs.2 \|\| NAT2 \|\| N-acetyltransferase 2 (arylamine N-acetyltransferase) (1) | Hs.2 | 1.36 | 1.67 | 10 | BC067218 |
| Hs.642877 \|\| MALAT1 \|\| Metastasis associated lung adenocarcinoma transcript 1 (non-coding RNA) (5) | Hs.642877 | 1.36 | 2.02 | 378938 | EF177381 |
| \|\| \|\| \|\| \|\| A1018643 \|\| \|\| \|\| \|\| 307816 | 1642186 | 1.36 | 2.15 | | AI018643 |
| Hs.21577 \|\| SNUPN \|\| Snurportin 1 (1) | Hs.21577 | 1.36 | 2.15 | 10073 | NM_005701 |
| Hs.418175 \|\| VPS28 \|\| Vacuolar protein sorting 28 homolog (S. cerevisiae) (1) | Hs.418175 | 1.36 | 0.95 | 51160 | BC050712 |
| Hs.120817 \|\| LIN9 \|\| Lin-9 homolog (C. elegans) (1) | Hs.120817 | 1.36 | 2.15 | 286826 | BC045625 |
| Hs. 129589 \|\| LOC643770 \|\| Hypothetical LOC643770 (1) | Hs.129589 | 1.35 | 3.52 | 643770 | AK126817 |
| Hs.523829 \|\| POLD4 \|\| Polymerase (DNA-directed), delta 4 (1) | Hs.523829 | 1.35 | 4.88 | 57804 | AB209274 |
| Hs.649349 \|\| \|\| Transcribed locus (1) | Hs.649349 | 1.35 | 2.98 | | AV722917 |
| \|\| \|\| \|\| \|\| AA581501 \|\| \|\| \|\| \|\| 103798 | 800675 | 1.35 | 1.24 | | AA581501 |
| Hs.187823 \|\| C2orf43 \|\| Chromosome 2 open reading frame 43 (1) | Hs.187823 | 1.35 | 2.15 | 60526 | AK025473 |
| Hs.534255 \|\| B2M \|\| Beta-2-microglobulin (2) | Hs.534255 | 1.35 | 4.88 | | BU153377 |
| Hs.34492 \|\| AS3MT \|\| Arsenic (+3 oxidation state) methyltransferase (1) | Hs.34492 | 1.35 | 1.43 | 119032 | AK098071 |
| Hs.284207 \|\| C6orf129 \|\| Chromosome 6 open reading frame 129 (1) | Hs.284207 | 1.35 | 2.98 | 154467 | BU597496 |
| Hs.654957 \|\| PPA2 \|\| Pyrophosphatase (inorganic) 2 (1) | Hs.654957 | 1.35 | 2.61 | 27068 | AL833123 |
| Hs.654718 \|\| PYCR2 \|\| Pyrroline-5-carboxylate reductase family, member 2 (1) | Hs.654718 | 1.35 | 0.95 | 29920 | AK001508 |
| \|\| \|\| *mitoch. cont. \|\| \|\| \|\| \|\| \|\| \|\| 433303 | *mitoch. cont. 1011702 | 1.35 | 2.61 | | |
| Hs.378885 \|\| PIGW \|\| Phosphatidylinositol glycan anchor biosynthesis, class W (1) | Hs.378885 | 1.35 | 1.67 | 284098 | NM_178517 |
| \|\| \|\| \| \| \|\| AI216134 \|\| \|\| \|\| \|\| 313738 | 1883883 | 1.35 | 4.01 | | AI216134 |
| Hs.654377 \|\| LDHC \|\| Lactate dehydrogenase C (1) | Hs.654377 | 1.35 | 0.50 | 3948 | BC064388 |
| Hs.655259 \|\| MRPS5 \|\| Mitochondrial ribosomal protein S5 (2) | Hs.655259 | 1.35 | 1.75 | 64969 | AK124892 |
| Hs.37916 \|\| DPP7 \|\| Dipeptidyl-peptidase 7 (1) | Hs.37916 | 1.35 | 4.88 | 29952 | AK128156 |
| Hs.23170 \|\| FTSJ1 \|\| FtsJ homolog 1 (E. coli) (2) | Hs.23170 | 1.35 | 2.61 | 24140 | NM_177439 |
| Hs.I71596 \|\| EPHA2 \|\| EPH receptor A2 (2) | Hs.171596 | 1.35 | 0.95 | 1969 | NM_004431 |
| Hs.488237 \|\| CD160 \|\| CD160 molecule (1) | Hs.488237 | 1.35 | 1.75 | 11126 | AK128370 |
| Hs.485262 \|\| MTCH1 \|\| Mitochondrial carrier homolog 1 (C. elegans) (1) | Hs.485262 | 1.35 | 4.01 | 23787 | BC040659 |
| Hs.534453 \|\| NDUFA13 \|\| NADH dehydrogenase (ubiquinone) 1 alpha subcomplex, 13 (3) | Hs.534453 | 1.35 | 4.44 | 51079 | BM809498 |
| Hs.666247 \|\| \|\| Transcribed locus (1) | Hs.666247 | 1.35 | 4.44 | | BX098298 |
| Hs.647403 \|\| ABCA6 \|\| ATP-binding cassette, sub-family A (ABC1), member 6 (1) | Hs.647403 | 1.35 | 1.75 | 23460 | BX647672 |
| Hs.655138 \|\| WBP11 \|\| WW domain binding protein 11 (1) | Hs.655138 | 1.34 | 2.15 | 51729 | BC023532 |
| Hs.471528 \|\| C2orf33 \|\| Chromosome 2 open reading frame 33 (1) | Hs.471528 | 1.34 | 3.52 | 56947 | NM_020194 |
| Hs.77897 \|\| SF3A3 \|\| Splicing factor 3a, subunit 3, 60kDa (1) | Hs.77897 | 1.34 | 2.61 | 10946 | AK128438 |
| Hs.185118 \|\| \|\| Transcribed locus, strongly similar to XP_001157816.1 hypothetical protein [Pan troglodytes] (1) | Hs.185118 | 1.34 | 4.01 | | BX114699 |
| Hs.518475 \|\| RFC4 \|\| Replication factor C (activator 1) 4, 37kDa (1) | Hs.518475 | 1.34 | 2.98 | 1974 | CR612348 |
| Hs.288761 \|\| TMEM134 \|\| Transmembrane protein 134 (1) | Hs.288761 | 1.34 | 2.61 | 80194 | AY007143 |
| Hs.355952 \|\| LOC150223 \|\| LOC150223 protein (1) | Hs.355952 | 1.34 | 1.67 | 150223 | BM906422 |
| Hs.645834 \|\| \|\| Transcribed locus (1) | Hs.645834 | 1.34 | 4.01 | | |
| Hs.218362 \|\| PDDC1 \|\| Parkinson disease 7 domain containing 1 (1) | Hs.218362 | 1.34 | 0.95 | 347862 | NM_182612 |
| Hs.652115 \|\| SARS2 \|\| Seryl-tRNA synthetase 2, mitochondrial (1) | Hs.652115 | 1.34 | 3.52 | 54938 | NM_017827 |
| Hs.466919 \|\| GEMIN7 \|\| Gem (nuclear organelle) associated protein 7 (1) | Hs.466919 | 1.34 | 0.50 | 79760 | NM_024707 |
| Hs.455693 \|\| DKFZp686F0839 \|\| Hypothetical gene supported by BX648662 (1) | Hs.455693 | 1.34 | 4.44 | 400070 | BX648662 |
| Hs.122115 \|\| EP400NL \|\| EP400 N-terminal like (1) | Hs.122115 | 1.34 | 4.44 | 347918 | AK128208 |
| Hs.586206 \|\| \|\| CDNA FLJ36043 fis, clone TESTI2017582 (1) | Hs.586206 | 1.34 | 2.15 | | AK093362 |
| Hs.368934 \|\| C17orf45 \|\| Chromosome 17 open reading frame 45 (1) | Hs.368934 | 1.34 | 2.61 | 125144 | BG027878 |
| Hs.517008 \|\| C20orf152 \|\| Chromosome 20 open reading frame 152 (1) | Hs.517008 | 1.34 | 2.15 | 140894 | BC030782 |
| Hs.547509 \|\| SMARCE1 \|\| SWI/SNF related, matrix associated, actin dependent regulator of chromatin, subfamily e, member 1 (1) | Hs.547509 | 1.34 | 0.95 | | AI866183 |
| Hs.591916 \|\| COX15 \|\| COX15 homolog, cytochrome c oxidase assembly protein (yeast) (1) | Hs.591916 | 1.34 | 2.15 | 1355 | NM_078470 |
| \|\| \|\| \|\| \|\| AI277171 \|\| \|\| \|\| \|\| 316887 | 1893626 | 1.34 | 0.95 | | AI277171 |
| Hs.368282 \|\| RP11-125A7.3 \|\| KIAA0564 protein (1) | Hs.368282 | 1.33 | 1.24 | 23078 | NM_015058 |
| Hs.212051 \|\| \|\| Transcribed locus (1) | Hs.212051 | 1.33 | 2.02 | | BM903565 |
| Hs.7768 \|\| FIBP \|\| Fibroblast growth factor (acidic) intracellular binding protein (1) | Hs.7768 | 1.33 | 2.98 | 9158 | BQ050043 |
| \|\| \|\| *mitoch. cont. EST \|\| \|\| \|\| \|\| \|\| \|\| 433309 | *mitoch. cont. 1161030 | 1.33 | 4.88 | | |
| Hs.603817 \|\| \|\| Transcribed locus (1) | Hs.603817 | 1.33 | 4.88 | | DB074239 |
| Hs.56281 \|\| ASB12 \|\| Ankyrin repeat and SOCS box-containing 12 (1) | Hs.56281 | 1.33 | 1.75 | 142689 | AK096896 |
| Hs.644420 \|\| TAOK3 \|\| TAO kinase 3 (2) | Hs.644420 | 1.33 | 2.61 | 51347 | NM_016281 |
| Hs.508154 \|\| ANKRD37 \|\| Ankyrin repeat domain 37 (1) | Hs.508154 | 1.33 | 4.01 | 353322 | AK127395 |
| Hs.499839 \|\| RPL7A \|\| Ribosomal protein L7a (1) | Hs.499839 | 1.33 | 4.44 | 6130 | BM908813 |
| \|\| \|\| \|\| \|\| AA946585 \|\| \|\| \|\| \|\| 101924 | 1588600 | 1.33 | 1.67 | | AA946585 |
| Hs.500098 \|\| \|\| Transcribed locus (1) | Hs.500098 | 1.33 | 3.52 | | BX106979 |
| Hs.112058 \|\| SIVA1 \|\| SIVA1, apoptosis-inducing factor (1) | Hs.112058 | 1.33 | 3.52 | 10572 | AK128704 |
| Hs.82921 \|\| C60rf165 \|\| Chromosome 6 open reading frame 165 (1) | Hs.82921 | 1.33 | 2.98 | 154313 | NM_178823 |
| Hs.663946 \|\| \|\| Transcribed locus, strongly similar to XP_509465.2 similar to FLJ39378 protein, partial [Pan troglodytes] (1) | Hs.663946 | 1.33 | 4.01 | | AI792136 |
| \|\| \|\| \|\| \|\| AA635173 \|\| \|\| \|\| \|\| 312027 | 1031004 | 1.33 | 3.52 | | AA635173 |
| \|\| \|\| *mitoch. cont. ESTs, Moderately similar to ALUC_HUMAN !!!! ALU CLASS C WARNING ENTRY!!! [H.sapiens] \|\| \|\| \|\| \|\| \|\| \|\| 433373 | *mitoch. cont. 916749 | 1.33 | 4.01 | | |
| Hs.645489 \|\| TRA2A \|\| Transformer-2 alpha (1) | Hs.645489 | 1.33 | 2.61 | 29896 | NM_013293 |
| Hs.633523 \|\| \|\| Transcribed locus (1) | Hs.633523 | 1.33 | 2.98 | | BX090705 |
| Hs.471401 \|\| BCS1L \|\| BCS1-like (yeast) (1) | Hs.471401 | 1.33 | 2.02 | 617 | AK096210 |
| Hs.464652 \|\| TNFSF5IP1 \|\| Tumor necrosis factor superfamily, member 5-induced protein 1 (1) | Hs.464652 | 1.33 | 0.50 | 56984 | AK225882 |
| Hs.654970 \|\| IL17RB \|\| Interleukin 17 receptor B (1) | Hs.654970 | 1.33 | 2.61 | 55540 | NM_018725 |
| Hs.528889 \|\| ATPAF2 \|\| ATP synthase mitochondrial F1 complex assembly factor 2 (1) | Hs.528889 | 1.33 | 2.98 | 91647 | AF070584 |
| Hs.655324 \|\| CLDN18 \|\| Claudin 18 (1) | Hs.655324 | 1.33 | 2.98 | 51208 | BC142708 |
| \|\| \|\| \|\| \|\| AI268348 \|\| \|\| \|\| \|\| 318185 | 1880873 | 1.33 | 0.72 | | AI268348 |
| Hs.136102 \|\| ZC3H13 \|\| Zinc finger CCCH-type containing 13 (5) | Hs.136102 | 1.33 | 1.75 | 23091 | AY283618 |
| Hs.79769 \|\| PCDH1 \|\| Protocadherin 1 (1) | Hs.79769 | 1.33 | 2.02 | 5097 | NM_032420 |
| Hs.655329 \|\| MRPS18B \|\| Mitochondrial ribosomal protein S 18B (2) | Hs.655329 | 1.33 | 0.72 | 28973 | AK126124 |
| Hs.18946 \|\| MRP826 \|\| Mitochondrial ribosomal protein S26 (1) | Hs.18946 | 1.33 | 1.67 | 64949 | BQ071472 |
| Hs.279761 \|\| CHMP4A \|\| Chromatin modifying protein 4A (1) | Hs.279761 | 1.33 | 4.44 | 29082 | AK094345 |
| Hs.503453 \|\| \|\| Transcribed locus (1) | Hs.503453 | 1.33 | 3.52 | | BX102205 |
| Hs.472270 \|\| RIN2 \|\| Ras and Rab interactor 2 (1) | Hs.472270 | 1.33 | 4.44 | 54453 | NM_018993 |
| \|\| \|\| \|\| \|\| \|\| \|\| \|\| \|\| 472878 | KR:11 | 1.33 | 0.95 | | |
| Hs.209151 \|\| LYRM5 \|\| LYR motif containing 5 (1) | Hs.209151 | 1.33 | 2.02 | 144363 | CR616971 |
| Hs.647062 \|\| RFC2 \|\| Replication factor C (activator 1) 2, 40kDa (1) | Hs.647062 | 1.33 | 2.98 | 5982 | NM_181471 |
| Hs.663273 \|\| \|\| Transcribed locus (1) | Hs.663273 | 1.33 | 2.02 | | DA339984 |
| \|\| \|\| \|\| \|\| T98266 \|\| \|\| \|\| \|\| 118919 | 122019 | 1.33 | 4.01 | | T98266 |
| Hs.80485 \|\| ADIPOQ \|\| Adiponectin, C1Q and collagen domain containing (1) | Hs.80485 | 1.33 | 2.02 | 9370 | NM_004797 |
| Hs.180933 \|\| CXXC1 \|\| CXXC finger 1 (PHD domain) (1) | Hs.180933 | 1.33 | 1.67 | 30827 | NM_00110165 4 |
| Hs.125352 \|\| \|\| CDNA: FLJ23065 fis, clone LNG04894 (1) | Hs.125352 | 1.33 | 2.98 | | AK026718 |
| \|\| \|\| *mitoch. cont. ESTs \|\| \|\| \|\| \|\| \|\| \|\| 433355 | *mitoch. cont. 62241 | 1.33 | 2.98 | | |
| Hs.253319 \|\| PIGU \|\| Phosphatidylinositol glycan anchor biosynthesis, class U (1) | Hs.253319 | 1.33 | 0.95 | 128869 | AK095356 |
| Hs.534261 \|\| MRPL46 \|\| Mitochondrial ribosomal protein L46 (1) | Hs.534261 | 1.33 | 2.98 | 26589 | AK092948 |
| Hs.474643 \|\| C22orf28 \|\| Chromosome 22 open reading frame 28 (1) | Hs.474643 | 1.32 | 4.44 | 51493 | NM_014306 |
| Hs.470259 \|\| FAM77C \|\| Family with sequence similarity 77, member C (1) | Hs.470259 | 1.32 | 2.02 | 79570 | AK022712 |
| Hs.433291 \|\| ARD1A \|\| ARD1 homolog A, N-acetyltransferase (S. cerevisiae) (2) | Hs.433291 | 1.32 | 2.61 | 8260 | BM926113 |
| Hs.112556 \|\| ZNF202 \|\| Zinc finger protein 202 (2) | Hs.112556 | 1.32 | 4.44 | 7753 | NM_003455 |
| Hs.654653 \|\| C16orf5 \|\| Chromosome 16 open reading frame 5 (2) | Hs.654653 | 1.32 | 4.88 | 29965 | AF131218 |
| Hs.284297 \|\| DUS3L \|\| Dihydrouridine synthase 3-like (S. cerevisiae) (1) | Hs.284297 | 1.32 | 1.75 | 56931 | AL365411 |
| Hs.573606 \|\| \|\| Transcribed locus (1) | Hs.573606 | 1.32 | 4.44 | | AI733498 |
| Hs.372265 \|\| BXDC1 \|\| Brix domain containing 1 (1) | Hs.372265 | 1.32 | 3.52 | 84154 | AL833613 |
| Hs.211079 \|\| ZNHIT1 \|\| Zinc finger, HIT type 1 (2) | Hs.211079 | 1.32 | 4.01 | 10467 | BM807959 |
| Hs.1545 \|\| CDX1 \|\| Caudal type homeobox transcription factor 1 (1) | Hs.1545 | 1.32 | 4.44 | 1044 | NM_001804 |
| Hs.34136 \|\| C21orf6 \|\| Chromosome 21 open reading frame 6 (2) | Hs.34136 | 1.32 | 2.15 | 10069 | CR592518 |
| Hs.174103 \|\| ITGAL \|\| Integrin, alpha L (antigen CD11A (p180), lymphocyte function-associated antigen 1; alpha polypeptide) (1) | Hs.174103 | 1.32 | 1.67 | 3683 | NM_002209 |
| Hs.114084 \|\| ENPP7 \|\| Ectonucleotide pyrophosphatase/phosphodiesterase 7 (1) | Hs.114084 | 1.32 | 4.01 | 339221 | AK126250 |
| Hs.196176 \|\| ECH1 \|\| Enoyl Coenzyme A hydratase 1, peroxisomal (2) | Hs.196176 | 1.32 | 2.61 | 1891 | AK126566 |
| Hs.449601 \|\| LOC96610 \|\| Hypothetical gene LOC96610 (1) | Hs.449601 | 1.32 | 2.98 | 96610 | BX641141 |
| Hs.306212 \|\| ATP8B3 \|\| ATPase, Class I, type 8B, member 3 (1) | Hs.306212 | 1.32 | 0.50 | 148229 | AK125969 |
| \|\| \|\| \|\| \|\| N50680 \|\| \|\| \|\| \|\| 118128 | 280838 | 1.32 | 4.44 | | N50680 |
| Hs.649307 \|\| C3orf10 \|\| Chromosome 3 open reading frame 10 (1) | Hs.649307 | 1.32 | 2.02 | 55845 | BF102725 |
| Hs.514063 \|\| CENTA2 \|\| Centaurin, alpha 2 (1) | Hs.514063 | 1.32 | 2.98 | 55803 | NM_018404 |
| Hs.522087 \|\| OPRS1 \|\| Opioid receptor, sigma 1 (3) | Hs.522087 | 1.32 | 3.52 | 10280 | AK130502 |
| Hs.463350 \|\| HOXB9 \|\| Homeobox B9 (1) | Hs.463350 | 1.32 | 0.95 | 3219 | NM_024017 |
| Hs.405153 \|\| NOD1 \|\| Nucleotide-binding oligomerization domain containing 1 (1) | Hs.405153 | 1.32 | 2.15 | 10392 | AK023969 |
| \|\| \|\| \|\| \|\| N72912 \|\| \|\| \|\| \|\| 117090 | 291609 | 1.31 | 2.61 | | N72912 |
| Hs.458598 \|\| UTP14A \|\| UTP14, U3 small nucleolar ribonucleoprotein, homolog A (yeast) (2) | Hs.458598 | 1.31 | 2.15 | 10813 | NM_006649 |
| Hs.591987 \|\| TRIM44 \|\| Tripartite motif-containing 44 (1) | Hs.591987 | 1.31 | 4.01 | 54765 | NM_017583 |
| Hs.514495 \|\| SRP68 \|\| Signal recognition particle 68kDa (3) | Hs.514495 | 1.31 | 0.95 | 6730 | AK098159 |
| Hs.350927 \|\| SLC25A6 \|\| Solute carrier family 25 (mitochondrial carrier; adenine nucleotide translocator), member 6 (3) | Hs.350927 | 1.31 | 4.44 | 293 | CR593822 |
| Hs.475525 \|\| NUP210 \|\| Nucleoporin 210kDa (1) | Hs.475525 | 1.31 | 2.98 | 23225 | NM_024923 |
| \|\| \|\| *mitoch. cont. \|\| \|\| \|\| \|\| \|\| \|\| 433330 | *mitoch. cont. 1683822 | 1.31 | 4.01 | | |
| Hs.632762 \|\| PDXP \|\| Pyridoxal (pyridoxine, vitamin B6) phosphatase (1) | Hs.632762 | 1.31 | 1.75 | 57026 | BC064922 |
| Hs.529369 \|\| AFAP1 \|\| Actin filament associated protein 1 (1) | Hs.529369 | 1.31 | 3.52 | 60312 | NM_198595 |
| Hs.157160 \|\| MRPS34 \|\| Mitochondrial ribosomal protein S34 (1) | Hs.157160 | 1.31 | 1.67 | 65993 | BF206087 |
| Hs.632685 \|\| TRUB2 \|\| TruB pseudouridine (psi) synthase homolog 2 (E. coli) (1) | Hs.632685 | 1.31 | 1.75 | 26995 | AK130008 |
| Hs.631520 \|\| ANKRD39 \|\| Ankyrin repeat domain 39 (2) | Hs.631520 | 1.31 | 4.88 | 51239 | BQ920586 |
| Hs.181297 \|\| LOC283663 \|\| Hypothetical protein LOC283663 (2) | Hs.181297 | 1.31 | 1.75 | 283663 | AK097083 |
| Hs.435579 \|\| BRDG1 \|\| BCR downstream signaling 1 (1) | Hs.435579 | 1.31 | 2.98 | 26228 | NM_012108 |
| Hs.202522 \|\| Clorf85 \|\| Chromosome 1 open reading frame 85 (1) | Hs.202522 | 1.31 | 3.52 | 112770 | BF690855 |
| Hs.471975 \|\| C20orf116 \|\| Chromosome 20 open reading frame 116 (1) | Hs.471975 | 1.31 | 4.01 | 65992 | AK310809 |
| Hs.11615 \|\| STYXL1 \|\| Serine/threonine/tyrosine interacting-like 1 (1) | Hs.11615 | 1.31 | 1.75 | 51657 | AK308912 |
| Hs.129673 \|\| EIF4A1 \|\| Eukaryotic translation initiation factor 4A, isoform 1 (3) | Hs.129673 | 1.31 | 0.95 | 1973 | BG033657 |
| Hs.591449 \|\| MTF2 \|\| Metal response element binding transcription factor 2 (1) | Hs.591449 | 1.31 | 1.67 | 22823 | NM_007358 |
| Hs.313247 \|\| C11orf79 \|\| Chromosome 11 open reading frame 79 (1) | Hs.313247 | 1.31 | 1.24 | 54949 | BX648946 |
| Hs.515617 \|\| FIZ1 \|\| FLT3-interacting zinc finger 1 (1) | Hs.515617 | 1.31 | 1.67 | 84922 | NM_032836 |
| Hs.572393 \|\| \|\| Transcribed locus, moderately similar to XP_516989.1 similar to Protein-tyrosine phosphatase, non-receptor type 11 (Protein-tyrosine phosphatase SYP) [Pan troglodytes] (1) | Hs.572393 | 1.31 | 3.52 | | BX103027 |
| \|\| \|\| \|\| \|\| AA757852 \|\| \|\| \|\| \|\| 224263 | 395794 | 1.31 | 3.52 | | AA757852 |
| Hs.337594 \|\| SDSL \|\| Serine dehydratase-like (1) | Hs.337594 | 1.31 | 1.43 | 113675 | BC009849 |
| Hs.523215 \|\| NDUFB8 \|\| NADH dehydrogenase (ubiquinone) 1 beta subcomplex, 8, 19kDa (1) | Hs.523215 | 1.31 | 3.52 | 4714 | BM923464 |
| \|\| \|\| \|\| \|\| R83852 \|\| \|\| \|\| \|\| 119303 | 193937 | 1.31 | 2.61 | | R83852 |
| Hs.409223 \|\| SSR4 \|\| Signal sequence receptor, delta (translocon-associated protein delta) (3) | Hs.409223 | 1.31 | 2.98 | 6748 | AK057117 |
| Hs.664769 \|\| \|\| Transcribed locus (1) | Hs.664769 | 1.31 | 1.43 | | BX107027 |
| Hs.221737 \|\| LIN7B \|\| Lin-7 homolog B (C. elegans) (1) | Hs.221737 | 1.30 | 2.02 | 64130 | BG749971 |
| Hs.598054 \|\| \|\| Transcribed locus, weakly similar to XP_850240.1 similar to Retrovirus-related Pol polyprotein from transposon 297, partial [Canis familiaris] (1) | Hs.598054 | 1.30 | 2.15 | | AA151772 |
| Hs.546288 \|\| RPS9 \|\| Ribosomal protein S9 (2) | Hs.546288 | 1.30 | 4.01 | 6203 | AK095055 |
| Hs.666452 \|\| \|\| Transcribed locus (1) | Hs.666452 | 1.30 | 3.52 | | AK093608 |
| \|\| \|\| \|\| \|\| AA757464 \|\| \|\| \|\| \|\| 223853 | 395898 | 1.30 | 2.61 | | AA757464 |
| Hs.270428 \|\| SUCLG1 \|\| Succinate-CoA ligase, GDP-forming, alpha subunit (1) | Hs.270428 | 1.30 | 4.01 | 8802 | AK125502 |
| Hs.256549 \|\| NUBP2 \|\| Nucleotide binding protein 2 (MinD homolog, E. coli) (1) | Hs.256549 | 1.30 | 2.15 | 10101 | AK124460 |
| Hs.153177 \|\| RPS28 \|\| Ribosomal protein S28 (2) | Hs.153177 | 1.30 | 3.52 | 6234 | CD048071 |
| Hs.388170 \|\| TSR1 \|\| TSR1, 20S rRNA accumulation, homolog (S. cerevisiae) (1) | Hs.388170 | 1.30 | 1.67 | 55720 | NM_018128 |
| Hs.128096 \|\| ZMYND19 \|\| Zinc finger, MYND-type containing 19 (1) | Hs.128096 | 1.30 | 2.15 | 116225 | AJ298882 |
| Hs.18029 \|\| C8orf32 \|\| Chromosome 8 open reading frame 32 (1) | Hs.18029 | 1.30 | 4.88 | 55093 | AK225643 |
| Hs.527688 \|\| TAF3 \|\| TAF3 RNA polymerase II, TATA box binding protein (TBP)-associated factor, 140kDa (1) | Hs.527688 | 1.30 | 2.98 | | BQ027547 |
| Hs.30954 \|\| PMVK \|\| Phosphomevalonate kinase (1) | Hs.30954 | 1.30 | 4.44 | 10654 | BC006089 |
| Hs.433738 \|\| GGTL3 \|\| Gamma-glutamyltransferase-like 3 (1) | Hs.433738 | 1.30 | 4.88 | 2686 | BC141843 |
| Hs.593949 \|\| \|\| Transcribed locus, weakly similar to XP_001091488.1 similar to phospholipase C, delta 4 isoform 2 [Macaca mulatta] (1) | Hs.593949 | 1.30 | 1.67 | | CD367475 |
| Hs.114412 \|\| TXNL1 \|\| Thioredoxin-like 1 (1) | Hs.114412 | 1.30 | 4.44 | 9352 | BX640857 |
| Hs.620849 \|\| \|\| Transcribed locus, strongly similar to XP_507735.1 similar to hypothetical protein [Pan troglodytes] (1) | Hs.620849 | 1.30 | 4.44 | | BG121957 |
| Hs.98013 \|\| \|\| Transcribed locus (1) | Hs.98013 | 1.30 | 4.01 | | CF272589 |
| Hs.413494 \|\| \|\| Transcribed locus (1) | Hs.413494 | 1.30 | 3.52 | | BX111013 |
| Hs.336431 \|\| TUBGCP6 \|\| Tubulin, gamma complex associated protein 6 (1) | Hs.336431 | 1.30 | 2.61 | 85378 | BC151209 |
| Hs.346868 \|\| EBNA1BP2 \|\| EBNA1 binding protein 2 (1) | Hs.346868 | 1.30 | 4.88 | 10969 | CR605987 |
| Hs.660022 \|\| MCART6 \|\| Mitochondrial carrier triple repeat 6 (2) | Hs.660022 | 1.30 | 1.67 | 401612 | AL833609 |
| \|\| \|\| \|\| \|\| AA629913 \|\| \|\| \|\| \|\| 223728 | 884666 | 1.30 | 2.15 | | AA629913 |
| Hs.517897 \|\| ENDOGL1 \|\| Endonuclease G-like 1 (1) | Hs.517897 | 1.30 | 3.52 | 9941 | AK023235 |
| Hs.541894 \|\| KIAA1641 \|\| KIAA1641 (2) | Hs.541894 | 1.30 | 3.52 | 57730 | BC146835 |
| Hs.171280 \|\| HSD17B10 \|\| Hydroxysteroid (17-beta) dehydrogenase 10 (2) | Hs.171280 | 1.29 | 2.15 | 3028 | BQ940058 |
| Hs.74647 \|\| TRA@ \|\| T cell receptor alpha locus (2) | Hs.74647 | 1.29 | 4.44 | 6955 | X73617 |
| Hs.301040 \|\| CABLES2 \|\| Cdk5 and Abl enzyme substrate 2 (1) | Hs.301040 | 1.29 | 2.61 | 81928 | NM_031215 |
| Hs.602961 \|\| \|\| Transcribed locus (1) | Hs.602961 | 1.29 | 2.61 | | AI792540 |
| Hs.513055 \|\| WDR61 \|\| WD repeat domain 61 (2) | Hs.513055 | 1.29 | 2.15 | 80349 | AK025098 |
| Hs.668456 \|\| \|\| Transcribed locus, strongly similar to XP_001148749.1 hypothetical protein [Pan troglodytes] (1) | Hs.668456 | 1.29 | 4.44 | | BX115983 |
| Hs.134857 \|\| EFCAB2 \|\| EF-hand calcium binding domain 2 (1) | Hs.134857 | 1.29 | 4.44 | | B1000882 |
| Hs.17883 \|\| PPM1G \|\| Protein phosphatase 1G (formerly 2C), magnesium-dependent, gamma isoform (1) | Hs.17883 | 1.29 | 2.61 | 5496 | AB209197 |
| Hs.636619 \|\| SNHG7 \|\| Small nucleolar RNA host gene (non-protein coding) 7 (1) | Hs.636619 | 1.29 | 3.52 | 677824 | AK054908 |
| Hs.10784 \|\| FAM46A \|\| Family with sequence similarity 46, member A (1) | Hs.10784 | 1.29 | 2.02 | 55603 | NM_017633 |
| Hs.445952 \|\| Clorf35 \|\| Chromosome 1 open reading frame 35 (1) | Hs.445952 | 1.29 | 2.98 | 79169 | AK126087 |
| Hs.649101 \|\| \|\| Transcribed locus (1) | Hs.649101 | 1.29 | 4.01 | | BU934681 |
| Hs.63224 \|\| \|\| Transcribed locus (1) | Hs.63224 | 1.29 | 2.61 | | AI732399 |
| \|\| \|\| \|\| \|\| AA905615 \|\| \|\| \|\| \|\| 307838 | 1506637 | 1.29 | 2.61 | | AA905615 |
| Hs.279914 \|\| ZNF232 \|\| Zinc finger protein 232 (1) | Hs.279914 | 1.29 | 0.95 | 7775 | NM_014519 |
| Hs.109778 \|\| WDR48 \|\| WD repeat domain 48 (1) | Hs.109778 | 1.29 | 4.01 | 64689 | AK124755 |
| Hs.78781 \|\| VEGFB \|\| Vascular endothelial growth factor B (1) | Hs.78781 | 1.29 | 4.01 | 7423 | BM546651 |
| \|\| \|\| *mitoch. cont. ESTs, Weakly similar to ALUF_HUMAN !!!! ALU CLASS F WARNING ENTRY!!! [H.sapiens] \|\| \|\| \|\| \|\| \|\| \|\| 433358 | *mitoch. cont. 76099 | 1.29 | 4.01 | | |
| \|\| \|\| \|\| \|\| AI301319 \|\| \|\| \|\| \|\| 315515 | 1901205 | 1.29 | 0.95 | | AI301319 |
| Hs.631717 \|\| GCHFR \|\| GTP cyclohydrolase I feedback regulator (2) | Hs.631717 | 1.29 | 4.88 | 2644 | BQ054887 |
| Hs.600503 \|\| LOC400129 \|\| Similar to SWI/SNF-related matrix-associated actin-dependent regulator of chromatin e1 (1) | Hs.600503 | 1.29 | 1.75 | | BM800811 |
| Hs.234282 \|\| VPS11 \|\| Vacuolar protein sorting 11 homolog (S. cerevisiae) (1) | Hs.234282 | 1.28 | 2.61 | 55823 | NM_021729 |
| Hs.98370 \|\| CYP2S1 \|\| Cytochrome P450, family 2, subfamily S, polypeptide 1 (1) | Hs.98370 | 1.28 | 4.44 | 29785 | NM_030622 |
| Hs.100426 \|\| BRMS1 \|\| Breast cancer metastasis suppressor 1 (1) | Hs.100426 | 1.28 | 4.44 | 25855 | BM472938 |
| Hs.486084 \|\| C60rf203 \|\| Chromosome 6 open reading frame 203 (1) | Hs.486084 | 1.28 | 2.61 | 51250 | AK091564 |
| Hs.343244 \|\| AP1G2 \|\| Adaptor-related protein complex 1, gamma 2 subunit (3) | Hs.343244 | 1.28 | 1.75 | 8906 | AF068706 |
| Hs.29679 \|\| CRSP3 \|\| **Cofactor required for Sp1 transcriptional activation, subunit 3, 130kDa (1) | Hs.29679 | 1.28 | 4.88 | 9439 | NM_004830 |
| Hs.482605 \|\| JMY \|\| Junction-mediating and regulatory protein (1) | Hs.482605 | 1.28 | 4.01 | 133746 | BX648731 |
| Hs.404183 \|\| MAP3K14 \|\| Mitogen-activated protein kinase kinase kinase 14 (1) | Hs.404183 | 1.28 | 2.98 | 9020 | Y10256 |
| Hs.13645 \|\| THYN1 \|\| Thymocyte nuclear protein 1 (1) | Hs.13645 | 1.28 | 2.02 | 29087 | NM_014174 |
| Hs.653377 \|\| \|\| Transcribed locus, strongly similar to XP_001100407.1 insulin-like growth factor 1 receptor [Macaca mulatta] (1) | Hs.653377 | 1.28 | 2.98 | | |
| \|\| \|\| \|\| \|\| AI053806 \|\| \|\| \|\| \|\| 105737 | 1861822 | 1.28 | 2.15 | | AI053806 |
| Hs.410924 \|\| TCHP \|\| Trichoplein, keratin filament binding (3) | Hs.410924 | 1.28 | 4.44 | 84260 | NM_032300 |
| Hs.648101 \|\| CARD11 \|\| Caspase recruitment domain family, member 11 (1) | Hs.648101 | 1.28 | 2.61 | 84433 | NM_032415 |
| Hs.593659 \|\| \|\| Transcribed locus, strongly similar to XP_518149.1 hypothetical protein XP_518149 [Pan troglodytes] (1) | Hs.593659 | 1.28 | 1.75 | | CA942841 |
| Hs.222510 \|\| DAZAP1 \|\| DAZ associated protein 1 (1) | Hs.222510 | 1.28 | 4.01 | 26528 | AK124583 |
| \|\| \|\| \|\| \|\| AA668231 \|\| \|\| \|\| 226869 | 852985 | 1.28 | 2.98 | | AA668231 |
| Hs.656909 \|\| MTP18 \|\| Mitochondrial protein 18 kDa (1) | Hs.656909 | 1.28 | 3.52 | 51537 | BF214503 |
| Hs.665977 \|\| \|\| CDNA FLJ45360 fis, clone BRHIP3013698 (1) | Hs.665977 | 1.28 | 4.44 | | AK127293 |
| \|\| \|\| \|\| \|\| AI054353 \|\| \|\| \|\| \|\| 112269 | 1861227 | 1.28 | 2.98 | | AI054353 |
| Hs.511801 \|\| C17orf49 \|\| Chromosome 17 open reading frame 49 (1) | Hs.511801 | 1.28 | 2.61 | 124944 | CR600192 |
| Hs.410355 \|\| RAD52 \|\| RAD52 homolog (S. cerevisiae) (1) | Hs.410355 | 1.28 | 1.75 | 100130219 | XM_001714414 |
| Hs.654431 \|\| ALOX12 \|\| Arachidonate 12-lipoxygenase (1) | Hs.654431 | 1.28 | 4.44 | 239 | NM_000697 |
| Hs.416994 \|\| POP7 \|\| Processing of precursor 7, ribonuclease P subunit (S. cerevisiae) (1) | Hs.416994 | 1.28 | 3.52 | 10248 | BU944802 |
| Hs.424312 \|\| PDLIM4 \|\| PDZ and LIM domain 4 (1) | Hs.424312 | 1.28 | 2.98 | 8572 | NM_003687 |
| Hs.27283 \|\| GABRB1 \|\| Gamma-aminobutyric acid (GABA) A receptor, beta 1 (1) | Hs.27283 | 1.28 | 2.61 | 2560 | AK127001 |
| Hs.525094 \|\| \|\| Transcribed locus (1) | Hs.525094 | 1.28 | 4.88 | | AA127874 |
| Hs.4096 \|\| COQ10A \|\| Coenzyme Q10 homolog A (S. cerevisiae) (1) | Hs.4096 | 1.28 | 1.43 | 93058 | NM_144576 |
| \|\| \|\| \|\| \|\| AI144050 \|\| \|\| \|\| \|\| 107701 | 1861166 | 1.27 | 1.75 | | AI144050 |
| Hs.514602 \|\| SMYD4 \|\| SET and MYND domain containing 4 (1) | Hs.514602 | 1.27 | 3.52 | 114826 | NM_052928 |
| Hs.40183 \|\| \|\| Transcribed locus (1) | Hs.40183 | 1.27 | 2.61 | 100129238 | XM_001716016 |
| \|\| \|\| \|\| \|\| AI251238 \|\| \|\| \|\| \|\| 117575 | 1985528 | 1.27 | 4.44 | | AI251238 |
| \|\| \|\| \|\| \|\| AI350836 \|\| \|\| \|\| \|\| 316670 | 1947284 | 1.27 | 1.75 | | AI350836 |
| Hs.659560 \|\| \|\| Transcribed locus (1) | Hs.659560 | 1.27 | 2.61 | | BX115671 |
| Hs.655741 \|\| \|\| CDNA FLJ35281 fis, clone PROST2007248 (1) | Hs.655741 | 1.27 | 2.98 | | AK092600 |
| Hs.301755 \|\| \|\| CDNA FLJ37243 fis, clone BRAMY2004387 (1) | Hs.301755 | 1.27 | 2.61 | | AK094562 |
| Hs.545054 \|\| \|\| Transcribed locus (1) | Hs.545054 | 1.27 | 3.52 | | N52802 |
| Hs.119598 \|\| RPL3 \|\| Ribosomal protein L3 (1) | Hs.119598 | 1.27 | 3.52 | 6122 | BC036582 |
| Hs.2561 \|\| NGFB \|\| Nerve growth factor, beta polypeptide (1) | Hs.2561 | 1.27 | 4.01 | 4803 | BC032517 |
| Hs.416216 \|\| DUSP12 \|\| Dual specificity phosphatase 12 (3) | Hs.416216 | 1.27 | 4.01 | 11266 | BC006286 |
| Hs.634846 \|\| \|\| CDNA FLJ31249 fis, clone KIDNE2005327 (1) | Hs.634846 | 1.27 | 2.61 | | AK055811 |
| Hs.507948 \|\| COG3 \|\| Component of oligomeric golgi complex 3 (1) | Hs.507948 | 1.27 | 4.01 | 83548 | AF349676 |
| Hs.648871 \|\| \|\| CDNA FLJ41644 fis, clone FEBRA2009362 (1) | Hs.648871 | 1.27 | 3.52 | | AK123638 |
| Hs.584807 \|\| TCF19 \|\| Transcription factor 19 (SC1) (1) | Hs.584807 | 1.27 | 4.44 | 6941 | NM_007109 |
| Hs.654803 \|\| ZNF496 \|\| Zinc finger protein 496 (1) | Hs.654803 | 1.27 | 4.88 | 84838 | BX647480 |
| Hs.456465 \|\| \|\| Clone HLS_IMAGE_1592675 mRNA sequence (1) | Hs.456465 | 1.27 | 4.01 | | DQ786214 |
| Hs.446522 \|\| RPL14 \|\| Ribosomal protein L14 (2) | Hs.446522 | 1.27 | 2.61 | 9045 | BF572302 |
| Hs.28914 \|\| APRT \|\| Adenine phosphoribosyltransferase (1) | Hs.28914 | 1.27 | 3.52 | 353 | CR622599 |
| Hs.406756 \|\| C4orf27 \|\| Chromosome 4 open reading frame 27 (1) | Hs.406756 | 1.27 | 2.61 | 54969 | BM458019 |
| Hs.524094 \|\| ZCCHC17 \|\| Zinc finger, CCHC domain containing 17 (1) | Hs.524094 | 1.27 | 4.44 | 51538 | NM_016505 |
| Hs.592081 \|\| STUB1 \|\| STIP1 homology and U-box containing protein 1 (3) | Hs.592081 | 1.27 | 3.52 | 10273 | NM_005861 |
| Hs.505575 \|\| GALNT6 \|\| UDP-N-acetyl-alpha-D-galactosamine:polypeptide N-acetylgalactosaminyltransferase 6 (GalNAc-T6) (1) | Hs.505575 | 1.26 | 3.52 | 11226 | NM_007210 |
| Hs.524690 \|\| PPIE \|\| Peptidylprolyl isomerase E (cyclophilin E) (1) | Hs.524690 | 1.26 | 2.02 | | AA922493 |
| Hs.555172 \|\| \|\| Transcribed locus (1) | Hs.555172 | 1.26 | 3.52 | | H99213 |
| Hs.208124 \|\| ESR1 \|\| Estrogen receptor 1 (2) | Hs.208124 | 1.26 | 4.01 | 2099 | NM_00112274 2 |
| Hs.534141 \|\| ADCK2 \|\| AarF domain containing kinase 2 (1) | Hs.534141 | 1.26 | 2.02 | 90956 | NM_052853 |
| \|\| \|\| \|\| \|\| R07196 \|\| \|\| \|\| \|\| 99379 | 126847 | 1.26 | 4.88 | | R07196 |
| Hs.600639 \|\| \|\| Transcribed locus, strongly similar to XP_001106378.1 hypothetical protein [Macaca mulatta] (1) | Hs.600639 | 1.26 | 2.15 | | BX092362 |
| Hs.274876 \|\| \|\| Transcribed locus (1) | Hs.274876 | 1.26 | 0.95 | | DB340270 |
| Hs.24212 \|\| LPHN2 \|\| Latrophilin 2 (1) | Hs.24212 | 1.26 | 2.61 | 23266 | NM_012302 |
| Hs.231883 \|\| OGFOD1 \|\| 2-oxoglutarate and iron-dependent oxygenase domain containing 1 (1) | Hs.231883 | 1.26 | 1.43 | 55239 | NM_018233 |
| Hs.6418 \|\| GPR175 \|\| G protein-coupled receptor 175 (1) | Hs.6418 | 1.26 | 2.61 | 131601 | AK056759 |
| Hs.71787 \|\| MRPS7 \|\| **Mitochondrial ribosomal protein S7 (1) | Hs.71787 | 1.26 | 3.52 | 51081 | CR626245 |
| Hs.431307 \|\| MRPL40 \|\| Mitochondrial ribosomal protein L40 (1) | Hs.431307 | 1.26 | 3.52 | 64976 | AK123768 |
| Hs.50850 \|\| FREM1 \|\| FRAS1 related extracellular matrix 1 (1) | Hs.50850 | 1.26 | 4.01 | | AK054690 |
| Hs.663265 \|\| \|\| Transcribed locus (1) | Hs.663265 | 1.26 | 2.61 | | BI334758 |
| \|\| \|\| \|\| \|\| N80424 \|\| \|\| \|\| \|\| 111374 | 290525 | 1.26 | 2.61 | | N80424 |
| \|\| \|\| \|\| \|\| \|\| \|\| \|\|306969 | 449443 | 1.26 | 2.61 | | |
| Hs.5308 \|\| UBA52 \|\| Ubiquitin A-52 residue ribosomal protein fusion product 1 (2) | Hs.5308 | 1.26 | 1.75 | 7311 | NM-00103393 0 |
| Hs.98330 \|\| \|\| Transcribed locus, strongly similar to XP_529770.1 hypothetical protein XP_529770 [Pan troglodytes] (1) | Hs.98330 | 1.26 | 2.61 | | BX093206 |
| Hs.483564 \|\| PFDN1 \|\| Prefoldin subunit 1 (1) | Hs.483564 | 1.26 | 4.88 | 5201 | AK093558 |
| \|\| \|\| \|\| \|\| \|\| EST \|\| \|\| \|\| 248439 | 309669 | 1.26 | 3.52 | | \|\| |
| Hs.662251 \|\| \|\| Transcribed locus (1) | Hs.662251 | 1.26 | 2.61 | | AI922966 |
| Hs. 193480 \|\| \|\| Transcribed locus (2) | Hs.193480 | 1.26 | 4.01 | | BF675806 |
| Hs.76662 \|\| ZDHHC16 \|\| Zinc finger, DHHC-type containing 16 (1) | Hs.76662 | 1.26 | 2.61 | 84287 | AB209272 |
| Hs.544589 \|\| \|\| Transcribed locus (1) | Hs.544589 | 1.26 | 4.88 | | AI014781 |
| \|\| \|\| \|\| \|\| H78609 \|\| \|\| \|\| \|\| 220161 | 234965 | 1.26 | 3.52 | | H78609 |
| \|\| \|\| \|\| \|\| AI002468 \|\| \|\| \|\| \|\| 110079 | 1592987 | 1.25 | 4.88 | | AI002468 |
| Hs.666059 \|\| \|\| Transcribed locus (1) | Hs.666059 | 1.25 | 4.88 | | BG680201 |
| Hs.597128 \|\| \|\| Transcribed locus (1) | Hs.597128 | 1.25 | 1.67 | | N59381 |
| Hs.531064 \|\| NEDD8 \|\| Neural precursor cell expressed, developmentally down-regulated 8 (1) | Hs.531064 | 1.25 | 2.61 | 4738 | AK125214 |
| Hs.435302 \|\| ZNF3 \|\| Zinc finger protein 3 (2) | Hs.435302 | 1.25 | 1.67 | 7551 | BX647182 |
| Hs.662372 \|\| \|\| Transcribed locus (1) | Hs.662372 | 1.25 | 4.44 | | BX103714 |
| Hs.404807 \|\| LOC348761 \|\| Hypothetical protein LOC348761 (1) | Hs.404807 | 1.25 | 4.88 | 348761 | BC047542 |
| \|\| \|\| \|\| \|\| AA775365 \|\| \|\| \|\| \|\| 316464 | 878683 | 1.25 | 3.52 | | AA775365 |
| Hs.596573 \|\| \|\| Transcribed locus (1) | Hs.596573 | 1.25 | 4.88 | | BQ947148 |
| \|\| \|\| \|\| \|\| AA757474 \|\| \|\| \|\| \|\| 308970 | 395899 | 1.25 | 4.01 | | AA757474 |
| Hs.411573 \|\| GUCY1B2 \|\| Guanylate cyclase 1, soluble, beta 2 (1) | Hs.411573 | 1.25 | 4.88 | | CR615161 |
| \|\| \|\| \|\| \|\| AA777175 \|\| \|\| \|\| \|\| 307824 | 378393 | 1.25 | 4.88 | | AA777175 |
| Hs.652166 \|\| MGC4677 \|\| Hypothetical protein MGC4677 (1) | Hs.652166 | 1.25 | 2.15 | 112597 | BG112065 |
| Hs.447530 \|\| HAPLN3 \|\| Hyaluronan and proteoglycan link protein 3 (1) | Hs.447530 | 1.24 | 4.01 | 145864 | BC053689 |
| Hs.77978 \|\| ZMIZ2 \|\| Zinc finger, MIZ-type containing 2 (2) | Hs.77978 | 1.24 | 4.01 | 83637 | NM_031449 |
| \|\| \|\| \|\| \|\| AI991606 \|\| \|\| \|\| \|\| 330964 | 2497556 | 1.24 | 4.01 | | AI991606 |
| \|\| \|\| \|\| \|\| AI053591 \|\| \|\| \|\| \|\| 107411 | 1861693 | 1.24 | 4.44 | | AI053591 |
| Hs.278362 \|\| MEA1 \|\| Male-enhanced antigen 1 (1) | Hs.278362 | 1.24 | 4.44 | 4201 | BI224374 |
| Hs.405877 \|\| \|\| CDNA FLJ32881 fis, clone TESTI2004153 (1) | Hs.405877 | 1.24 | 3.52 | | AK057443 |
| Hs.555934 \|\| SPINK4 \|\| Serine peptidase inhibitor, Kazal type 4 (1) | Hs.555934 | 1.24 | 2.61 | 27290 | AA502919 |
| Hs.506861 \|\| DDX54 \|\| DEAD (Asp-Glu-Ala-Asp) box polypeptide 54 (1) | Hs.506861 | 1.24 | 4.01 | 79039 | NM_00111132 2 |
| Hs.437638 \|\| XBP1 \|\| X-box binding protein 1 (2) | Hs.437638 | 1.24 | 4.44 | 7494 | AK093842 |
| Hs.472491 \|\| OXCT2 \|\| 3-oxoacid CoA transferase 2 (1) | Hs.472491 | 1.24 | 1.24 | 64064 | AK024440 |
| Hs.657887 \|\| ACPL2 \|\| Acid phosphatase-like 2 (1) | Hs.657887 | 1.24 | 2.61 | 92370 | BC036701 |
| Hs.584957 \|\| TOR3A \|\| Torsin family 3, member A (1) | Hs.584957 | 1.24 | 2.15 | 64222 | NM_022371 |
| Hs.4900 \|\| CIAPIN1 \|\| Cytokine induced apoptosis inhibitor 1 (2) | Hs.4900 | 1.24 | 2.15 | 57019 | NM_020313 |
| Hs.523395 \|\| MUC5B \|\| Mucin 5B, oligomeric mucus/gel-forming (1) | Hs.523395 | 1.24 | 2.61 | 727897 | NM_002458 |
| Hs.283741 \|\| EXOSC5 \|\| Exosome component 5 (1) | Hs.283741 | 1.24 | 4.44 | 56915 | BM450105 |
| Hs.664374 \|\| \|\| CDNA FLJ36327 fis, clone THYMU2005748 (1) | Hs.664374 | 1.24 | 3.52 | | AK093646 |
| Hs.659200 \|\| CENPL \|\| Centromere protein L (1) | Hs.659200 | 1.24 | 3.52 | 91687 | NM-00112718 1 |
| \|\| \|\| \|\| \|\| R20435 \|\| \|\| \|\| \|\| 308392 | 32895 | 1.24 | 4.01 | | R20435 |
| Hs.110839 \|\| ZKSCAN5 \|\| Zinc finger with KRAB and SCAN domains 5 (1) | Hs.110839 | 1.23 | 4.88 | 23660 | BX648490 |
| Hs.479384 \|\| KIAA0746 \|\| KIAA0746 protein (3) | Hs.479384 | 1.23 | 3.52 | 23231 | NM-015187 |
| Hs.659831 \|\| \|\| Transcribed locus (1) | Hs.659831 | 1.23 | 4.44 | | BX113836 |
| Hs.3416 \|\| ADFP \|\| Adipose differentiation-related protein (2) | Hs.3416 | 1.23 | 4.44 | 123 | NM_001122 |
| Hs.532286 \|\| YTHDF2 \|\| YTH domain family, member 2 (1) | Hs.532286 | 1.23 | 3.52 | 51441 | NM_016258 |
| Hs.62716 \|\| \|\| Transcribed locus (1) | Hs.62716 | 1.23 | 4.88 | | BX096436 |
| Hs.655256 \|\| SLC35E2 \|\| Solute carrier family 35, member E2 (3) | Hs.655256 | 1.23 | 2.15 | | CR936618 |
| Hs.155742 \|\| GRHPR \|\| Glyoxylate reductase/hydroxypyruvate reductase (3) | Hs.155742 | 1.23 | 4.44 | 9380 | AK024386 |
| Hs.209945 \|\| TDP1 \|\| Tyrosyl-DNA phosphodiesterase 1 (2) | Hs.209945 | 1.23 | 2.98 | 55775 | NM_018319 |
| Hs.570322 \|\| \|\| Transcribed locus (1) | Hs.570322 | 1.23 | 4.88 | | DA230405 |
| Hs.558393 \|\| RRM1 \|\| Ribonucleotide reductase M1 polypeptide (1) | Hs.558393 | 1.23 | 4.88 | 6240 | NM_001033 |
| Hs.539079 \|\| \|\| Transcribed locus, strongly similar to XP_944638.1 hypothetical protein XP_944638 [Homo sapiens] (1) | Hs.539079 | 1.23 | 3.52 | | DB332104 |
| Hs.515475 \|\| SYMPK \|\| Symplekin (2) | Hs.515475 | 1.23 | 2.61 | 8189 | AB210010 |
| Hs.634915 \|\| \|\| Transcribed locus, moderately similar to XP_001094962.1 similar to hydroxypyruvate isomerase homolog isoform 5 [Macaca mulatta] (1) | Hs.634915 | 1.23 | 2.15 | | BU633294 |
| Hs.664984 \|\| \|\| Transcribed locus (1) | Hs.664984 | 1.23 | 2.98 | | BQ706403 |
| Hs.655326 \|\| DNAH11 \|\| Dynein, axonemal, heavy chain 11 (2) | Hs.655326 | 1.23 | 3.52 | 8701 | NM_003777 |
| Hs.75841 \|\| ERP29 \|\| Endoplasmic reticulum protein 29 (1) | Hs.75841 | 1.23 | 3.52 | 10961 | NM_006817 |
| Hs.661446 \|\| \|\| HC6 (HC6) (1) | Hs.661446 | 1.23 | 4.01 | | AF269286 |
| Hs.13885 \|\| MED10 \|\| Mediator of RNA polymerase II transcription, subunit 10 homolog (NUT2, S. cerevisiae) (1) | Hs.13885 | 1.22 | 2.61 | 84246 | BG335164 |
| Hs.631918 \|\| CCDC12 \|\| Coiled-coil domain containing 12 (1) | Hs.631918 | 1.22 | 2.98 | 151903 | AK098120 |
| Hs.593430 \|\| MIB2 \|\| Mindbomb homolog 2 (Drosophila) (2) | Hs.593430 | 1.22 | 2.02 | 142678 | BC035078 |
| Hs.656558 \|\| ZNF493 \|\| Zinc finger protein 493 (2) | Hs.656558 | 1.22 | 4.01 | 284443 | NM_00107667 8 |
| Hs.444276 \|\| NUP37 \|\| Nucleoporin 37kDa (1) | Hs.444276 | 1.22 | 3.52 | 79023 | BU535474 |
| Hs.72550 \|\| HMMR \|\| Hyaluronan-mediated motility receptor (RHAMM) (1) | Hs.72550 | 1.22 | 2.98 | 3161 | AF032862 |
| Hs.16936 \|\| C20orf198 \|\| Chromosome 20 open reading frame 198 (2) | Hs.16936 | 1.22 | 2.02 | 128439 | AK091209 |
| Hs.110445 \|\| SBDS \|\| Shwachman-Bodian-Diamond syndrome (1) | Hs.110445 | 1.22 | 2.61 | 51119 | AY169963 |
| Hs.191887 \|\| SEC61B \|\| Sec61 beta subunit (2) | Hs.191887 | 1.22 | 4.44 | 10952 | BG573843 |
| Hs.436102 \|\| ISG20L1 \|\| Interferon stimulated exonuclease gene 20kDa-like 1 (1) | Hs.436102 | 1.22 | 4.01 | 64782 | AB209323 |
| Hs.406461 \|\| FAM86A \|\| Family with sequence similarity 86, member A (3) | Hs.406461 | 1.22 | 2.98 | 196483 | BC010084 |
| Hs.520026 \|\| VARS \|\| Valyl-tRNA synthetase (2) | Hs.520026 | 1.22 | 4.88 | 7407 | NM_006295 |
| Hs.602319 \|\| KIAA1648 \|\| KIAA1648 protein (1) | Hs.602319 | 1.21 | 4.88 | 284900 | AB051435 |
| Hs.533444 \|\| HMGCL \|\| 3-hydroxymethyl-3-methylglutaryl-Coenzyme A lyase (hydroxymethylglutaricaciduria) (1) | Hs.533444 | 1.21 | 4.44 | 3155 | AK122801 |
| Hs.647094 \|\| FASTK \|\| Fas-activated serine/threonine kinase (1) | Hs.647094 | 1.21 | 2.98 | 10922 | CR611266 |
| Hs.634845 \|\| \|\| Transcribed locus (1) | Hs.634845 | 1.21 | 4.01 | | AW504569 |
| Hs.283610 I \| ATG4B \|\| ATG4 autophagy related 4 homolog B (S. cerevisiae) (1) | Hs.283610 | 1.21 | 4.01 | 23192 | AK125277 |
| Hs.694959 \|\| SFT2D3 \|\| SFT2 domain containing 3 (1) | Hs.694959 | 1.21 | 4.01 | 84826 | NM_032740 |
| Hs.562561 \|\| \|\| Full-length cDNA clone CSODI086YK13 of Placenta Cot 25-normalized of Homo sapiens (human) (1) | Hs.562561 | 1.21 | 4.88 | | CR594409 |
| Hs.489287 \|\| CPSF4 \|\| Cleavage and polyadenylation specific factor 4, 30kDa (2) | Hs.489287 | 1.21 | 3.52 | 10898 | NM_006693 |
| Hs.591952 \|\| SLC43A1 \|\| Solute carrier family 43, member 1 (2) | Hs.591952 | 1.21 | 2.98 | 8501 | NM_003627 |
| Hs.578450 \|\| MESDC2 \|\| Mesoderm development candidate 2 (1) | Hs.578450 | 1.21 | 4.88 | 23184 | NM_015154 |
| Hs.27693 \|\| PPIL1 \|\| Peptidylprolyl isomerase (cyclophilin)-like 1 (1) | Hs.27693 | 1.21 | 4.88 | 51645 | AY359032 |
| \|\| \|\| \|\| \|\| AA992653 \|\| \|\| \|\| \|\| 306951 | 1623179 | 1.21 | 4.44 | | AA992653 |
| Hs.227457 \|\| C6orf130 \|\| Chromosome 6 open reading frame 130 (3) | Hs.227457 | 1.21 | 2.98 | 221443 | AK290939 |
| Hs.655405 \|\| EDG5 \|\| Endothelial differentiation, sphingolipid G-protein-coupled receptor, 5 (1) | Hs.655405 | 1.20 | 2.98 | 9294 | NM_004230 |
| Hs.520133 \|\| RPL7L1 \|\| Ribosomal protein L7-like 1(2) | Hs.520133 | 1.20 | 2.98 | 285855 | AK074339 |
| Hs.654822 \|\| \|\| Transcribed locus, strongly similar to XP_513538.1 similar to LIM domain transcription factor LMO4 (LIM-only protein 4) (LMO-4) (Breast tumor autoantigen) [Pan troglodytes] (1) | Hs.654822 | 1.20 | 3.52 | | BE569007 |
| Hs.656313 \|\| TMCO4 \| Transmembrane and coiled-coil domains 4 (1) | Hs.656313 | 1.20 | 3.52 | 255104 | BX640987 |
| Hs.592166 \|\| HOXA10 \|\| Homeobox A10 (1) | Hs.592166 | 1.20 | 2.98 | 3206 | NM_018951 |
| Hs.351558 \|\| U2AF1L4 \|\| U2 small nuclear RNA auxiliary factor 1-like 4 (1) | Hs.351558 | 1.20 | 4.44 | 199746 | BC049208 |
| Hs. 380118 \|\| RBMX \|\| RNA binding motif protein, X-linked (3) | Hs.380118 | 1.20 | 4.88 | 27316 | BX647131 |
| Hs.632708 \|\| TSPAN31 \|\| Tetraspanin 31 (1) | Hs.632708 | 1.20 | 4.88 | 6302 | AB209699 |
| Hs.537126 \|\| TIRAP \|\| Toll-interleukin 1 receptor (TIR) domain containing adaptor protein (1) | Hs.537126 | 1.20 | 4.01 | 114609 | NM_00103966 1 |
| Hs.648086 \|\| LOC441212 \|\| Retinitis pigmentosa 9 pseudogene (1) | Hs.648086 | 1.20 | 4.88 | 441212 | CR618508 |
| Hs.211594 \|\| PSMC4 \|\| Proteasome (prosome, macropain) 26S subunit, ATPase, 4 (2) | Hs.211594 | 1.20 | 2.98 | 5704 | CR611800 |
| Hs.308418 \|\| SUHW3 \|\| Suppressor of hairy wing homolog 3 (Drosophila) (1) | Hs.308418 | 1.20 | 4.88 | 55609 | BC051728 |
| Hs.15961 \|\| C9orf23 \|\| Chromosome 9 open reading frame 23 (2) | Hs.15961 | 1.20 | 4.88 | 138716 | BM467200 |
| Hs.437195 \|\| TMEM118 \|\| Transmembrane protein 118 (1) | Hs.437195 | 1.19 | 4.88 | 84900 | NM_00110990 3 |
| Hs.25597 \|\| ELOVL1 \|\| Elongation of very long chain fatty acids (FEN1/Elo2, SUR4/Elo3, yeast)-like 1 (1) | Hs.25597 | 1.19 | 2.98 | 64834 | CR600869 |
| Hs.522662 \|\| TSR2 \|\| TSR2, 20S rRNA accumulation, homolog (S. cerevisiae) (1) | Hs.522662 | 1.19 | 3.52 | 90121 | CR622248 |
| | 0 | | | | |
| | 0 | | | | |
| Negative genes (931); decreased after treatment | 0 | | | | |

| Gene Name | Gene ID | Fold Change | q-value(%) | GeneID | UGRepAcc |
|---|---|---|---|---|---|
| Hs.112341 \|\| PI3 \|\| Peptidase inhibitor 3, skin-derived (SKALP) (1) | Hs.112341 | 0.54 | 0.00 | 5266 | CF596521 |
| \|\| \|\| \|\| \|\| AA281932 \|\| \|\| \|\| \|\| 114949 | 712604 | 0.57 | 0.00 | | AA281932 |
| Hs.561555 \|\| \|\| Transcribed locus (1) | Hs.561555 | 0.59 | 0.00 | | BI828537 |
| Hs.576971 \|\| \|\| Transcribed locus (1) | Hs.576971 | 0.60 | 0.00 | | AA494443 |
| Hs.584845 \|\| PLXNC1 \|\| Plexin C1 (1) | Hs.584845 | 0.61 | 0.00 | 10154 | AB208934 |
| Hs.483486 \|\| JMJD1B \|\| Jumonji domain containing 1B (1) | Hs.483486 | 0.63 | 0.00 | 51780 | NM_016604 |
| Hs.645286 \|\| GFPT1 \|\| Glutamine-fructose-6-phosphate transaminase 1 (1) | Hs.645286 | 0.64 | 0.00 | 2673 | BC045641 |
| Hs.663896 \|\| \|\| Transcribed locus (1) | Hs.663896 | 0.64 | 0.00 | | BF574014 |
| Hs.519909 \|\| MARCKS \|\| Myristoylated alanine-rich protein kinase C substrate (1) | Hs.519909 | 0.65 | 0.00 | 4082 | NM_002356 |
| Hs.335513 \|\| F13A1 \|\| Coagulation factor XIII, A1 polypeptide (1) | Hs.335513 | 0.65 | 0.00 | 2162 | BC027963 |
| Hs.25277 \|\| CENTD3 \|\| Centaurin, delta 3 (1) | Hs.25277 | 0.65 | 0.00 | 64411 | NM_022481 |
| Hs.382202 \|\| CHI3L1 \|\| Chitinase 3-like 1 (cartilage glycoprotein-39) (1) | Hs.382202 | 0.66 | 0.00 | 1116 | NM_001276 |
| Hs.500015 \|\| CDK5R1 \|\| Cyclin-dependent kinase 5, regulatory subunit 1 (p35) (1) | Hs.500015 | 0.66 | 0.00 | 8851 | BC030792 |
| Hs. 159306 \|\| ABR \|\| Active BCR-related gene (1) | Hs.159306 | 0.66 | 0.00 | 29 | AK124547 |
| Hs.486357 \|\| SMPDL3A \|\| Sphingomyelin phosphodiesterase, acid-like 3A (1) | Hs.486357 | 0.67 | 0.00 | 10924 | AK096144 |
| Hs.511149 \|\| SNAP23 \|\| Synaptosomal-associated protein, 23kDa (1) | Hs.511149 | 0.67 | 0.00 | 8773 | BC022890 |
| Hs.591588 \|\| HK2 \|\| Hexokinase 2 (1) | Hs.591588 | 0.67 | 0.00 | 3099 | NM_000189 |
| Hs.79334 \|\| NFIL3 \|\| Nuclear factor, interleukin 3 regulated (1) | Hs.79334 | 0.67 | 0.00 | 4783 | NM_005384 |
| Hs.491682 \|\| PRKDC \|\| Protein kinase, DNA-activated, catalytic polypeptide (1) | Hs.491682 | 0.67 | 0.00 | 5591 | NM_006904 |
| Hs.567284 \|\| HIVEP1 \|\| Human immunodeficiency virus type I enhancer binding protein 1 (1) | Hs.567284 | 0.67 | 0.00 | 3096 | X51435 |
| Hs.448851 \|\| USP6 \|\| Ubiquitin specific peptidase 6 (Tre-2 oncogene) (1) | Hs.448851 | 0.68 | 0.00 | 9098 | BX647719 |
| Hs.556496 \|\| TANK \|\| TRAF family member-associated NFKB activator (2) | Hs.556496 | 0.68 | 0.00 | 10010 | NM_004180 |
| Hs.589962 \|\| TOPORS \|\| Topoisomerase I binding, arginine/serine-rich (1) | Hs.589962 | 0.68 | 0.00 | 10210 | NM_005802 |
| Hs.67846 \|\| LILRB4 \|\| Leukocyte immunoglobulin-like receptor, subfamily B (with TM and ITIM domains), member 4 (1) | Hs.67846 | 0.68 | 0.00 | 11006 | BC026309 |
| Hs.513743 \|\| MON1B \|\| MON1 homolog B (yeast) (1) | Hs.513743 | 0.68 | 0.00 | 22879 | AB020679 |
| Hs.532375 \|\| SLC25A44 \|\| Solute carrier family 25, member 44 (1) | Hs.532375 | 0.68 | 0.00 | 9673 | BC039854 |
| Hs.99855 \|\| FPRL1 \|\| Formyl peptide receptor-like 1 (1) | Hs.99855 | 0.68 | 0.45 | 2358 | M84562 |
| Hs.155097 \|\| CA2 \|\| Carbonic anhydrase II (2) | Hs.155097 | 0.68 | 0.00 | 760 | AK123309 |
| Hs.186486 \|\| MAP3K5 \|\| Mitogen-activated protein kinase kinase kinase 5 (2) | Hs.186486 | 0.69 | 0.00 | 4217 | U67156 |
| Hs.460857 \|\| AYTL1 \|\| Acyltransferase like 1 (1) | Hs.460857 | 0.69 | 0.00 | 54947 | BX641069 |
| Hs.442344 \|\| IRS2 \|\| Insulin receptor substrate 2 (1) | Hs.442344 | 0.69 | 0.00 | 8660 | NM_003749 |
| Hs.419240 \|\| SLC2A3 \|\| Solute carrier family 2 (facilitated glucose transporter), member 3 (1) | Hs.419240 | 0.69 | 0.00 | 6515 | AB209607 |
| Hs.443861 \|\| SRPK1 \|\| SFRS protein kinase 1 (1) | Hs.443861 | 0.69 | 0.00 | 6732 | BC038292 |
| Hs.422336 \|\| RGS19 \|\| Regulator of G-protein signalling 19 (1) | Hs.422336 | 0.69 | 0.00 | 10287 | CR591957 |
| \|\| \|\| \|\| \|\| T99230 \|\| \|\| \|\| \|\| 109997 | 122405 | 0.69 | 0.00 | | T99230 |
| Hs. 154299 \|\| F2RL1 \|\| Coagulation factor II (thrombin) receptor-like 1 (1) | Hs.154299 | 0.69 | 0.00 | 2150 | NM_005242 |
| Hs.198241 \|\| AOC3 \|\| Amine oxidase, copper containing 3 (vascular adhesion protein 1) (1) | Hs.198241 | 0.70 | 0.00 | 8639 | BC036368 |
| Hs.654597 \|\| CENTB2 \|\| Centaurin, beta 2 (1) | Hs.654597 | 0.70 | 0.45 | 23527 | NM-012287 |
| Hs.660544 \|\| \|\| Transcribed locus (1) | Hs.660544 | 0.70 | 0.45 | | AA044825 |
| Hs.470882 \|\| CALCRL \|\| Calcitonin receptor-like (1) | Hs.470882 | 0.70 | 0.00 | 10203 | NM_005795 |
| Hs.285197 \|\| SRPK2 \|\| SFRS protein kinase 2 (2) | Hs.285197 | 0.70 | 0.00 | 6733 | U88666 |
| Hs.474751 \|\| MYH9 \|\| Myosin, heavy chain 9, non-muscle (1) | Hs.474751 | 0.70 | 0.00 | 4627 | NM_002473 |
| Hs.646490 \|\| BNIP2 \|\| BCL2/adenovirus E1B 19kDa interacting protein 2 (2) | Hs.646490 | 0.70 | 0.45 | 663 | NM_004330 |
| Hs.190334 \|\| RAP1A \|\| RAP1A, member of RAS oncogene family (1) | Hs.190334 | 0.70 | 0.59 | 5906 | NM_00101093 5 |
| Hs.476052 \|\| SNRK \|\| SNF related kinase (1) | Hs.476052 | 0.70 | 0.00 | 54861 | NM_017719 |
| Hs.317593 \|\| TMCO3 \|\| Transmembrane and coiled-coil domains 3 (1) | Hs.317593 | 0.70 | 0.00 | 55002 | NM_017905 |
| Hs.114286 \|\| CD9 \|\| CD9 molecule (1) | Hs.114286 | 0.70 | 0.59 | 928 | AK025016 |
| Hs.654754 \|\| SSH2 \|\| Slingshot homolog 2 (Drosophila) (1) | Hs.654754 | 0.71 | 0.00 | 85464 | NM_033389 |
| Hs.35125 \|\| FLJ10357 \|\| Hypothetical protein FLJ10357 (2) | Hs.35125 | 0.71 | 0.00 | 55701 | NM_018071 |
| Hs.464071 \|\| PGD \|\| Phosphogluconate dehydrogenase (1) | Hs.464071 | 0.71 | 0.00 | 5226 | NM_002631 |
| Hs.329266 \|\| HIP1 \|\| Huntingtin interacting protein 1 (1) | Hs.329266 | 0.71 | 0.00 | 3092 | NM_005338 |
| Hs.473761 \|\| RTN3 \|\| Reticulon 3 (2) | Hs.473761 | 0.71 | 0.45 | | CD557026 |
| Hs.594861 \|\| \|\| CDNA FLJ13601 fis, clone PLACE1010069 (1) | Hs.594861 | 0.71 | 0.45 | | AL049435 |
| Hs.132868 \|\| USP32 \|\| Ubiquitin specific peptidase 32(1) | Hs.132868 | 0.71 | 0.00 | 84669 | NM_032582 |
| Hs.595378 \|\| \|\| CDNA clone 5314178 (1) | Hs.595378 | 0.71 | 0.00 | | BC047111 |
| Hs.656286 \|\| CCL15 \|\| **chemokine (C-C motif) ligand 15 (1) | Hs.656286 | 0.71 | 0.00 | 6359 | NM_004167 |
| Hs.529925 \|\| UBR2 \|\| Ubiquitin protein ligase E3 component n-recognin 2 (1) | Hs.529925 | 0.71 | 0.45 | 23304 | BX647467 |
| Hs.654824 \|\| TM9SF2 \|\| Transmembrane 9 superfamily member 2 (1) | Hs.654824 | 0.71 | 0.00 | 9375 | BC110656 |
| Hs.654609 \|\| NUMB \|\| Numb homolog (Drosophila) (1) | Hs.654609 | 0.71 | 0.00 | 8650 | AK226069 |
| Hs.477155 \|\| ATP6V1A \|\| ATPase, H+ transporting, lysosomal 70kDa, V1 subunit A (1) | Hs.477155 | 0.71 | 0.59 | 523 | NM_001690 |
| \|\| \|\| \|\| \|\| N69252 \|\| \|\| \|\| \|\| 114886 | 294740 | 0.71 | 0.00 | | W03215 |
| Hs.657926 \|\| PELI2 \|\| Pellino homolog 2 (Drosophila) (1) | Hs.657926 | 0.71 | 0.00 | 57161 | NM_021255 |
| Hs.195471 \|\| PFKFB3 \|\| 6-phosphofructo-2-kinase/fructose-2,6-biphosphatase 3 (2) | Hs.195471 | 0.71 | 0.59 | | AK094645 |
| Hs.533055 \|\| PCAF \|\| P300/CBP-associated factor (2) | Hs.533055 | 0.71 | 0.00 | 8850 | NM_003884 |
| Hs.693806 \|\| \|\| **Antigen MMSA-2 mRNA sequence (1) | Hs.693806 | 0.71 | 0.00 | | BX424266 |
| Hs.644421 \|\| UBE2B \| **Ubiquitin-conjugating enzyme E2B (RAD6 homolog) (2) | Hs.644421 | 0.71 | 0.45 | 7320 | NM_003337 |
| Hs.283378 \|\| RELL1 \|\| Receptor expressed in lymphoid tissues like 1 (1) | Hs.283378 | 0.71 | 0.45 | 768211 | NM_00108540 0 |
| Hs.499659 \|\| GARNL4 \|\| GTPase activating Rap/RanGAP domain-like 4 (1) | Hs.499659 | 0.72 | 0.00 | 23108 | AJ628446 |
| Hs.458513 \|\| PPP1R3B \|\| Protein phosphatase 1, regulatory (inhibitor) subunit 3B (1) | Hs.458513 | 0.72 | 0.45 | 79660 | NM_024607 |
| Hs. 105941 \|\| \|\| Transcribed locus (1) | Hs.105941 | 0.72 | 0.00 | | DB519379 |
| Hs.136398 \|\| \|\| Transcribed locus (1) | Hs.136398 | 0.72 | 0.45 | | BG436386 |
| \|\| \|\| \|\| \|\| AI261606 \|\| \|\| \|\| \|\| 104570 | 2028924 | 0.72 | 0.45 | | AI261606 |
| Hs.514681 \|\| MAP2K4 \|\| Mitogen-activated protein kinase kinase 4 (1) | Hs.514681 | 0.72 | 0.00 | 6416 | AK131544 |
| Hs.220971 \|\| FOSL2 \|\| FOS-like antigen 2 (3) | Hs.220971 | 0.72 | 0.45 | 2355 | BX647822 |
| Hs.126941 \|\| FAM49B \|\| Family with sequence similarity 49, member B (1) | Hs.126941 | 0.72 | 0.00 | 51571 | CR749628 |
| Hs.597419 \|\| \|\| Transcribed locus (1) | Hs.597419 | 0.72 | 0.00 | | AU155802 |
| Hs.79033 \|\| QPCT \|\| Glutaminyl-peptide cyclotransferase (glutaminyl cyclase) (1) | Hs.79033 | 0.72 | 0.59 | 25797 | NM_012413 |
| Hs.515660 \|\| ZNF324 \|\| Zinc finger protein 324 (1) | Hs.515660 | 0.72 | 0.45 | 25799 | AK092341 |
| Hs.510078 \|\| SGK \|\| Serum/glucocorticoid regulated kinase (2) | Hs.510078 | 0.72 | 0.45 | 6446 | BX537882 |
| Hs.648434 \|\| \|\| MRNA full length insert cDNA clone EUROIMAGE 1509279 (1) | Hs.648434 | 0.72 | 0.59 | 79627 | AL137346 |
| Hs.482660 \|\| ZFYVE16 \|\| Zinc finger, FYVE domain containing 16 (1) | Hs.482660 | 0.72 | 0.59 | 9765 | NM_00110525 1 |
| Hs.494146 \|\| TMEM2 \|\| Transmembrane protein 2 (1) | Hs.494146 | 0.72 | 0.00 | 23670 | AF137030 |
| Hs.592298 \|\| PPM1A \|\| Protein phosphatase 1A (formerly 2C), magnesium-dependent, alpha isoform (1) | Hs.592298 | 0.72 | 0.45 | 5494 | NM_021003 |
| Hs.298573 \|\| SH3BP5L \|\| SH3-binding domain protein 5-like (1) | Hs.298573 | 0.72 | 0.59 | 80851 | AK125837 |
| Hs.268774 \|\| SIPA1L2 \|\| Signal-induced proliferation-associated 1 like 2 (1) | Hs.268774 | 0.73 | 0.72 | 57568 | AY168879 |
| Hs.158315 \|\| IL18RAP \|\| Interleukin 18 receptor accessory protein (1) | Hs.158315 | 0.73 | 0.00 | 8807 | AF077346 |
| Hs.434980 \|\| APP \|\| Amyloid beta (A4) precursor protein (peptidase nexin-II, Alzheimer disease) (1) | Hs.434980 | 0.73 | 0.59 | 351 | NM_000484 |
| Hs.159028 \|\| BTN2A1 \|\| Butyrophilin, subfamily 2, member A1 (1) | Hs.159028 | 0.73 | 0.59 | 11120 | BC016661 |
| Hs.8118 \|\| SMCHD1 \|\| Structural maintenance of chromosomes flexible hinge domain containing 1 (1) | Hs.8118 | 0.73 | 0.72 | 23347 | NM_015295 |
| Hs.36959 \|\| ZFAND3 \|\| Zinc finger, AN1-type domain 3 (1) | Hs.36959 | 0.73 | 0.00 | 60685 | AK023284 |
| Hs.129683 \|\| UBE2D1 \|\| Ubiquitin-conjugating enzyme E2D 1 (UBC4/5 homolog, yeast) (1) | Hs.129683 | 0.73 | 0.89 | 7321 | NM_003338 |
| Hs.433512 \|\| ACTR3 \|\| ARP3 actin-related protein 3 homolog (yeast) (2) | Hs.433512 | 0.73 | 0.59 | 10096 | BC044590 |
| Hs.633085 \|\| CD36 \|\| **CD36 molecule (thrombospondin receptor) (1) | Hs.633085 | 0.73 | 0.45 | 948 | AL832074 |
| Hs.171695 \|\| DUSP1 \|\| Dual specificity phosphatase 1 (1) | Hs.171695 | 0.73 | 0.45 | 1843 | AK127679 |
| Hs.23735 \|\| KCNF1 \|\| Potassium voltage-gated channel, subfamily F, member 1 (1) | Hs.23735 | 0.73 | 0.00 | 3754 | BC026110 |
| Hs.61812 \|\| PTPN12 \|\| Protein tyrosine phosphatase, non-receptor type 12 (1) | Hs.61812 | 0.73 | 0.00 | 5782 | BC050008 |
| \|\| \|\| \|\| \|\| T82854 \|\| \|\| \|\| \|\| 115035 | 110872 | 0.73 | 0.72 | | T82854 |
| Hs.61438 \|\| RPGR \|\| Retinitis pigmentosa GTPase regulator (1) | Hs.61438 | 0.74 | 0.45 | 6103 | NM_00103485 3 |
| Hs.517517 \|\| EP300 \|\| E1A binding protein p300 (1) | Hs.517517 | 0.74 | 0.00 | 2033 | U01877 |
| Hs.534052 \|\| ZFP36 \|\| Zinc finger protein 36, C3H type, homolog (mouse) (1) | Hs.534052 | 0.74 | 0.45 | 7538 | CR597101 |
| Hs.593923 \|\| \|\| Transcribed locus (1) | Hs.593923 | 0.74 | 0.98 | | AV755499 |
| Hs.25292 \|\| JUNB \|\| Jun B proto-oncogene (2) | Hs.25292 | 0.74 | 0.45 | 3726 | CR601699 |
| Hs.35861 \|\| TMEM158 \|\| Transmembrane protein 158 (1) | Hs.35861 | 0.74 | 0.45 | 25907 | NM_015444 |
| Hs.511386 \|\| DMXL2 \|\| Dmx-like 2 (1) | Hs.511386 | 0.74 | 0.98 | 23312 | NM_015263 |
| Hs.170986 \|\| GALNT3 \|\| UDP-N-acetyl-alpha-D-galactosamine:polypeptide N-acetylgalactosaminyltransferase 3 (GalNAc-T3) (1) | Hs.170986 | 0.74 | 0.72 | 2591 | NM_004482 |
| Hs.170999 \|\| LBA1 \|\| Lupus brain antigen 1 (1) | Hs.170999 | 0.74 | 0.89 | 9881 | NM_014831 |
| Hs.660642 \|\| ZNF438 \|\| Zinc finger protein 438 (1) | Hs.660642 | 0.74 | 0.59 | 220929 | AK131357 |
| Hs.24030 \|\| SLC31A2 \|\| Solute carrier family 31 (copper transporters), member 2 (1) | Hs.24030 | 0.74 | 0.89 | 1318 | AK131071 |
| Hs.287537 \|\| GALNAC4S-6ST \|\| B cell RAG associated protein (1) | Hs.287537 | 0.74 | 0.59 | 51363 | CR749804 |
| Hs.444448 \|\| KCNK5 \|\| Potassium channel, subfamily K, member 5 (1) | Hs.444448 | 0.74 | 0.45 | 8645 | NM_003740 |
| \|\| \|\| \|\| \|\| AI262370 \|\| \|\| \|\| \|\| 316765 | 1871101 | 0.74 | 0.00 | | AI262370 |
| Hs.465087 \|\| SMAD7 \|\| SMAD family member 7 (1) | Hs.465087 | 0.74 | 0.98 | 4092 | AF010193 |
| Hs.644654 \|\| \|\| Transcribed locus (1) | Hs.644654 | 0.74 | 0.45 | | BP304613 |
| Hs.459759 \|\| CREBBP \|\| CREB binding protein (Rubinstein-Taybi syndrome) (2) | Hs.459759 | 0.74 | 0.45 | 1387 | NM_004380 |
| Hs.513779 \|\| CRISPLD2 \|\| Cysteine-rich secretory protein LCCL domain containing 2 (1) | Hs.513779 | 0.74 | 0.89 | 83716 | AL136861 |
| Hs.493649 \|\| POU2F1 \|\| POU domain, class 2, transcription factor 1 (1) | Hs.493649 | 0.74 | 0.45 | 5451 | AK091438 |
| Hs.510521 \|\| RCOR1 \|\| REST corepressor 1 (1) | Hs.510521 | 0.74 | 0.59 | 23186 | NM_015156 |
| Hs.469820 \|\| RALB \|\| V-ral simian leukemia viral oncogene homolog B (ras related; GTP binding protein) (2) | Hs.469820 | 0.74 | 0.00 | 5899 | AK127675 |
| Hs.585464 \|\| CCDC7 \|\| Coiled-coil domain containing 7 (1) | Hs.585464 | 0.74 | 0.59 | 221016 | BX647771 |
| Hs.279815 \|\| CSAD \|\| Cysteine sulfinic acid decarboxylase (1) | Hs.279815 | 0.74 | 0.45 | 51380 | AK122681 |
| \|\| \|\| \|\| \|\| AI871056 \|\| \|\| \|\| \|\| 331322 | 2430220 | 0.74 | 0.45 | | AI871056 |
| Hs.152717 \|\| FAM65A \|\| Family with sequence similarity 65, member A (1) | Hs.152717 | 0.74 | 0.59 | 79567 | AK127792 |
| Hs.179260 \|\| C14orf4 \|\| Chromosome 14 open reading frame 4 (2) | Hs.179260 | 0.74 | 0.72 | 64207 | BC108292 |
| Hs.516726 \|\| SCG2 \|\| Secretogranin II (chromogranin C) (1) | Hs.516726 | 0.74 | 0.00 | 7857 | BC022509 |
| Hs.470412 \|\| RBMS1 \|\| RNA binding motif, single stranded interacting protein 1 (2) | Hs.470412 | 0.75 | 0.00 | 5937 | NM_016839 |
| Hs.654600 \|\| HIF1A \|\| Hypoxia-inducible factor 1, alpha subunit (basic helix-loop-helix transcription factor) (2) | Hs.654600 | 0.75 | 0.89 | 3091 | NM_001530 |
| Hs.514107 \|\| CCL3 \|\| Chemokine (C-C motif) ligand 3 (1) | Hs.514107 | 0.75 | 1.24 | 6348 | CR591007 |
| Hs.504641 \|\| CD163 \|\| CD163 molecule (1) | Hs.504641 | 0.75 | 0.00 | 9332 | NM_004244 |
| Hs.449628 \|\| NRBF2 \|\| Nuclear receptor binding factor 2 (1) | Hs.449628 | 0.75 | 0.72 | 29982 | NM_030759 |
| \|\| \|\| \|\| \|\| T91080 \|\| \|\| \|\| \|\| 110513 | 112565 | 0.75 | 0.45 | | T91080 |
| Hs.647659 \|\| GPR177 \|\| G protein-coupled receptor 177 (1) | Hs.647659 | 0.75 | 0.59 | 79971 | BX537492 |
| Hs.15396 \|\| LOC196264 \|\| Hypothetical protein LOC196264 (1) | Hs.15396 | 0.75 | 0.98 | 196264 | AK095399 |
| Hs.592340 \|\| ZFP161 \|\| Zinc finger protein 161 homolog (mouse) (1) | Hs.592340 | 0.75 | 0.45 | 7541 | NM_003409 |
| Hs.494977 \|\| MEGF9 \|\| Multiple EGF-like-domains 9 (1) | Hs.494977 | 0.75 | 0.72 | 1955 | NM_00108049 7 |
| Hs.593122 \|\| \|\| Transcribed locus (1) | Hs.593122 | 0.75 | 0.59 | | N24233 |
| Hs.368971 \|\| NCOA6 \|\| Nuclear receptor coactivator 6 (1) | Hs.368971 | 0.75 | 0.45 | 23054 | AF208227 |
| Hs.313 \|\| SPP1 \|\| Secreted phosphoprotein 1 (osteopontin, bone sialoprotein I, early T-lymphocyte activation 1) (1) | Hs.313 | 0.75 | 0.72 | 6696 | BF698898 |
| Hs.269408 \|\| E2F3 \|\| E2F transcription factor 3 (1) | Hs.269408 | 0.75 | 0.45 | 1871 | NM_001949 |
| Hs.694913 \|\| \|\| Mesenchymal stem cell protein DSC96 (1) | Hs.694913 | 0.75 | 0.59 | | |
| Hs.657569 \|\| GALNACT-2 \|\| Chondroitin sulfate GalNAcT-2 (1) | Hs.657569 | 0.75 | 0.59 | 55454 | BX647369 |
| Hs.7886 \|\| PELI1 \|\| Pellino homolog 1 (Drosophila) (1) | Hs.7886 | 0.75 | 0.72 | 57162 | AF302505 |
| Hs.132342 \|\| LPIN2 \|\| Lipin 2 (1) | Hs.132342 | 0.75 | 1.75 | 9663 | BC152448 |
| Hs.233458 \|\| NFYC \|\| Nuclear transcription factor Y, gamma (1) | Hs.233458 | 0.75 | 0.45 | 4802 | AK055329 |
| Hs.651923 \|\| CNN2 \|\| Calponin 2 (1) | Hs.651923 | 0.75 | 0.00 | 1265 | NM_004368 |
| Hs.654950 \|\| \|\| Transcribed locus (1) | Hs.654950 | 0.75 | 0.59 | | BQ574775 |
| Hs.463439 \|\| SPAG9 \|\| Sperm associated antigen 9 (1) | Hs.463439 | 0.75 | 0.59 | 9043 | AY850123 |
| Hs.268963 \|\| UBAP1 \|\| Ubiquitin associated protein 1 (1) | Hs.268963 | 0.75 | 0.59 | 51271 | AK074969 |
| Hs.131087 \|\| \|\| Transcribed locus (1) | Hs.131087 | 0.75 | 1.24 | | BX111162 |
| Hs.593232 \|\| \|\| Full length insert cDNA clone YY93G09 (2) | Hs.593232 | 0.75 | 0.59 | | BM479406 |
| Hs.558541 \|\| C14orf138 \|\| Chromosome 14 open reading frame 138 (1) | Hs.558541 | 0.75 | 0.45 | 79609 | NM_024558 |
| Hs.449415 \|\| EIF2C2 \|\| Eukaryotic translation initiation factor 2C, 2 (4) | Hs.449415 | 0.75 | 0.45 | 27161 | BC054491 |
| Hs.655826 \|\| \|\| Transcribed locus (1) | Hs.655826 | 0.75 | 0.59 | | BM919533 |
| \|\| \|\| \|\| \|\| R19408 \|\| \|\| \|\| \|\| 118853 | 130031 | 0.75 | 0.00 | | R19408 |
| Hs.592801 \|\| \|\| CDNA FLJ30652 fis, clone DFNES2000011 (1) | Hs.592801 | 0.75 | 0.00 | | BX641108 |
| Hs.443837 \|\| NPEPPS \|\| Aminopeptidase puromycin sensitive (1) | Hs.443837 | 0.75 | 0.00 | 9520 | NM_006310 |
| Hs.68061 \|\| SPHK1 \|\| Sphingosine kinase 1 (1) | Hs.68061 | 0.75 | 0.72 | 8877 | AK095578 |
| \|\| \|\| \|\| \|\| R91385 \|\| \|\| \|\| \|\| 308765 | 195943 | 0.75 | 1.43 | | R91385 |
| Hs. 12341 \|\| ADAR \|\| Adenosine deaminase, RNA-specific (1) | Hs.12341 | 0.75 | 0.59 | 103 | X79448 |
| Hs.37982 \|\| NEDD9 \|\| Neural precursor cell expressed, developmentally down-regulated 9 (1) | Hs.37982 | 0.75 | 1.43 | | AU156739 |
| Hs.514276 \|\| SP2 \|\| Sp2 transcription factor (1) | Hs.514276 | 0.75 | 0.45 | 6668 | D28588 |
| Hs.79299 \|\| LHFPL2 \|\| Lipoma HMGIC fusion partner-like 2 (1) | Hs.79299 | 0.76 | 1.24 | 10184 | NM_005779 |
| Hs.632370 \|\| UBE4B \|\| Ubiquitination factor E4B (UFD2 homolog, yeast) (2) | Hs.632370 | 0.76 | 0.59 | 10277 | NM_00110556 2 |
| Hs.412355 \|\| LASS1 \|\| LAG1 longevity assurance homolog 1 (S. cerevisiae) (1) | Hs.412355 | 0.76 | 0.59 | 10715 | NM_001492 |
| Hs.144700 \|\| EFNB1 \|\| Ephrin-B1 (1) | Hs.144700 | 0.76 | 0.59 | 1947 | NM_004429 |
| Hs.659456 \|\| NPAS3 \|\| Neuronal PAS domain protein 3 (1) | Hs.659456 | 0.76 | 0.45 | 64067 | AF164438 |
| Hs.99196 \|\| MAG1 \|\| Lung cancer metastasis-associated protein (1) | Hs.99196 | 0.76 | 0.59 | 84803 | DQ345298 |
| Hs.436062 \|\| GBE1 \|\| Glucan (1,4-alpha-), branching enzyme 1 (glycogen branching enzyme, Andersen disease, glycogen storage disease type IV) (1) | Hs.436062 | 0.76 | 0.98 | 2632 | AK125918 |
| Hs.596095 \|\| \|\| CDNA FLJ33254 fis, clone ASTR02005360 (1) | Hs.596095 | 0.76 | 0.59 | | FLJ33254 |
| \|\| \|\| \|\| \|\| AA909113 \|\| \|\| \|\| 310812 | 1542754 | 0.76 | 1.24 | | AA909113 |
| Hs.665004 \|\| \|\| CDNA FLJ36515 fis, clone TRACH2001810 (4) | Hs.665004 | 0.76 | 0.45 | | FLJ36515 |
| Hs.655310 \|\| ERICH1 \|\| Glutamate-rich 1 (1) | Hs.655310 | 0.76 | 0.59 | 157697 | BX647093 |
| Hs.84190 \|\| SLC19A1 \|\| Solute carrier family 19 (folate transporter), member 1 (1) | Hs.84190 | 0.76 | 0.45 | 6573 | AB209069 |
| Hs.12707 \|\| DNAJC13 \|\| DnaJ (Hsp40) homolog, subfamily C, member 13 (1) | Hs.12707 | 0.76 | 0.89 | 23317 | BC151246 |
| Hs.647090 \|\| TMEM176B \|\| Transmembrane protein 176B (1) | Hs.647090 | 0.76 | 1.75 | 28959 | AK097304 |
| Hs.131220 \|\| \|\| Clone MAGp998D064417Q2 mRNA sequence (1) | Hs.131220 | 0.76 | 0.89 | | AF508905 |
| Hs.24643 \|\| ZNF746 \|\| Zinc finger protein 746 (1) | Hs.24643 | 0.76 | 0.89 | 155061 | BC035586 |
| Hs.292472 \|\| SEC24B \|\| SEC24 related gene family, member B (S. cerevisiae) (1) | Hs.292472 | 0.76 | 0.59 | 10427 | AJ131245 |
| Hs.644684 \|\| \|\| Transcribed locus (1) | Hs.644684 | 0.76 | 1.43 | | BF795172 |
| Hs.271383 \|\| GALNS \|\| Galactosamine (N-acetyl)-6-sulfate sulfatase (Morquio syndrome, mucopolysaccharidosis type IVA) (1) | Hs.271383 | 0.76 | 0.00 | 2588 | BC050684 |
| Hs.471492 \|\| EIF2C4 \|\| Eukaryotic translation initiation factor 2C, 4 (2) | Hs.471492 | 0.76 | 1.24 | 192670 | BC152450 |
| Hs.143102 \|\| AOC2 \|\| Amine oxidase, copper containing 2 (retina-specific) (1) | Hs.143102 | 0.76 | 0.45 | 314 | NM_009590 |
| Hs.654872 \|\| \|\| Clone HLS_IMAGE_1881469 mRNA sequence (1) | Hs.654872 | 0.76 | 0.45 | | BM557567 |
| Hs.532363 \|\| HIPK1 \|\| Homeodomain interacting protein kinase 1 (2) | Hs.532363 | 0.76 | 0.72 | 204851 | NM_198268 |
| Hs.470316 \|\| ACVR1 \|\| Activin A receptor, type I (1) | Hs.470316 | 0.76 | 0.45 | 90 | BC036748 |
| Hs.592105 \|\| ST6GALNAC2 \|\| ST6 (alpha-N-acetyl-neuraminyl-2,3-beta-galactosyl-1,3)-N-acetylgalactosaminide alpha-2,6-sialyltransferase 2 (1) | Hs.592105 | 0.76 | 0.98 | 10610 | BC038114 |
| Hs.604577 \|\| \|\| Transcribed locus (1) | Hs.604577 | 0.76 | 0.45 | | BF509991 |
| Hs.659321 \|\| \|\| ZNF253 \|\| Zinc finger protein 253 (1) | Hs.659321 | 0.76 | 1.24 | 56242 | AK074757 |
| Hs.145586 \|\| COL4A6 \|\| Collagen, type IV, alpha 6 (1) | Hs.145586 | 0.76 | 0.59 | 1288 | NM_033641 |
| Hs.464137 \|\| ACOX1 \|\| Acyl-Coenzyme A oxidase 1, palmitoyl (2) | Hs.464137 | 0.76 | 1.24 | 51 | NM_004035 |
| Hs.187636 \|\| LRRK2 \|\| Leucine-rich repeat kinase 2 (1) | Hs.187636 | 0.77 | 0.72 | 120892 | NM_198578 |
| Hs.653530 \|\| \|\| **Transcribed locus (1) | Hs.653530 | 0.77 | 0.89 | | |
| Hs.104315 \|\| SHOC2 \|\| Soc-2 suppressor of clear homolog (C. elegans) (2) | Hs.104315 | 0.77 | 0.59 | 8036 | BC044752 |
| Hs.79347 \|\| ZNF592 \|\| Zinc finger protein 592 (1) | Hs.79347 | 0.77 | 0.45 | 9640 | NM_014630 |
| Hs.163893 \|\| PICALM \|\| Phosphatidylinositol binding clathrin assembly protein (2) | Hs.163893 | 0.77 | 0.59 | 8301 | NM_007166 |
| Hs.368996 \|\| RLN1 \|\| Relaxin 1 (1) | Hs.368996 | 0.77 | 0.45 | 6013 | BC005956 |
| Hs.302772 \|\| SH3BGRL2 \|\| SH3 domain binding glutamic acid-rich protein like 2 (2) | Hs.302772 | 0.77 | 0.98 | 83699 | AF340151 |
| Hs.10842 \|\| RAN \|\| RAN, member RAS oncogene family (1) | Hs.10842 | 0.77 | 1.67 | 5901 | NM_006325 |
| Hs.121396 \|\| C6orf170 \|\| Chromosome 6 open reading frame 170 (1) | Hs.121396 | 0.77 | 0.45 | 221322 | AK131446 |
| Hs.444202 \|\| SLC9A8 \|\| Solute carrier family 9 (sodium/hydrogen exchanger), member 8 (1) | Hs.444202 | 0.77 | 0.89 | 23315 | NM_015266 |
| Hs.561824 \|\| \|\| Transcribed locus (1) | Hs.561824 | 0.77 | 0.98 | | BG291190 |
| Hs.468908 \|\| MXD1 \|\| MAX dimerization protein 1 (1) | Hs.468908 | 0.77 | 1.24 | 4084 | BC098396 |
| Hs.633514 \|\| TIMP2 \|\| TIMP metallopeptidase inhibitor 2 (5) | Hs.633514 | 0.77 | 0.59 | 7077 | NM_003255 |
| Hs.145500 \|\| \|\| Transcribed locus (1) | Hs.145500 | 0.77 | 0.59 | 388795 | XM_371397 |
| Hs.148741 \|\| IBRDC2 \|\| IBR domain containing 2 (2) | Hs.148741 | 0.77 | 0.59 | 255488 | NM_182757 |
| Hs.584854 \|\| AVIL \|\| Advillin (1) | Hs.584854 | 0.77 | 1.67 | 10677 | BX647344 |
| Hs.463421 \|\| ABCC3 \|\| ATP-binding cassette, sub-family C (CFTR/MRP), member 3 (1) | Hs.463421 | 0.77 | 0.59 | 8714 | AF085692 |
| Hs.459106 \|\| AZIN1 \|\| Antizyme inhibitor 1 (2) | Hs.459106 | 0.77 | 0.89 | 51582 | NM_015878 |
| Hs.632828 \|\| HNRPH2 \|\| Heterogeneous nuclear ribonucleoprotein H2 (H') (1) | Hs.632828 | 0.77 | 0.72 | 3188 | NM_019597 |
| Hs.190043 \|\| MOSPD2 \|\| Motile sperm domain containing 2 (2) | Hs.190043 | 0.77 | 1.24 | 158747 | AL834345 |
| \|\| \|\| \|\| \|\| AI342954 \|\| \|\| \|\| 312038 | 1909706 | 0.77 | 1.24 | | AI342954 |
| Hs.159188 \|\| \|\| CDNA clone 30408657 (1) | Hs.159188 | 0.77 | 0.59 | 100131989 | BX538248 |
| Hs.288284 \|\| PQLC1 \|\| PQ loop repeat containing 1 (1) | Hs.288284 | 0.77 | 0.59 | 80148 | AK126188 |
| Hs.165195 \|\| VAPA \|\| VAMP (vesicle-associated membrane protein)-associated protein A, 33kDa (3) | Hs.165195 | 0.77 | 0.72 | | BM996423 |
| Hs.444213 \|\| TLE4 \|\| Transducin-like enhancer of split 4 (E(sp1) homolog, Drosophila) (1) | Hs.444213 | 0.77 | 0.98 | 7091 | CR749553 |
| Hs.528513 \|\| \|\| Transcribed locus (1) | Hs.528513 | 0.77 | 0.45 | | BX504481 |
| Hs.662448 \|\| \|\| Transcribed locus (1) | Hs.662448 | 0.77 | 0.72 | | |
| Hs.597222 \|\| \|\| Transcribed locus (1) | Hs.597222 | 0.77 | 0.59 | | AW136350 |
| Hs.521640 \|\| RAD23B \|\| RAD23 homolog B (S. cerevisiae) (3) | Hs.521640 | 0.77 | 0.45 | 5887 | NM_002874 |
| Hs.643896 \|\| VCL \|\| Vinculin (1) | Hs.643896 | 0.77 | 0.98 | 7414 | NM_014000 |
| Hs.483772 \|\| FBX038 \|\| F-box protein 38 (1) | Hs.483772 | 0.77 | 0.59 | 81545 | NM_205836 |
| Hs.551713 \|\| MBP \|\| Myelin basic protein (1) | Hs.551713 | 0.77 | 0.89 | 4155 | NM_00102510 0 |
| Hs.77318 \|\| PAFAH1B1 \|\| Platelet-activating factor acetylhydrolase, isoform Ib, alpha subunit 45kDa (1) | Hs.77318 | 0.77 | 0.98 | 5048 | NM_000430 |
| Hs.591865 \|\| LRRC6 \|\| Leucine rich repeat containing 6 (1) | Hs.591865 | 0.77 | 0.98 | 23639 | BC027589 |
| Hs.250083 \|\| SLC9A2 \|\| Solute carrier family 9 (sodium/hydrogen exchanger), member 2 (1) | Hs.250083 | 0.77 | 1.43 | 6549 | NM_003048 |
| Hs.693811 \|\| \|\| Transcribed locus (1) | Hs.693811 | 0.77 | 0.72 | | |
| Hs.656213 \|\| JAK2 \|\| Janus kinase 2 (a protein tyrosine kinase) (1) | Hs.656213 | 0.77 | 0.98 | 3717 | AF058925 |
| \|\| \|\| *mitoch. cont. ESTs, Weakly similar to 138022 hypothetical protein [H.sapiens] \|\| \|\| \|\| \|\| \|\| \|\| 433317 | *mitoch. cont. 1573953 | 0.77 | 0.45 | | |
| Hs.654922 \|\| TXNRD1 \|\| Thioredoxin reductase 1 (1) | Hs.654922 | 0.77 | 0.59 | | BG255626 |
| Hs.535060 \|\| KIAA2026 \|\| KIAA2026 (1) | Hs.535060 | 0.77 | 1.43 | 158358 | NM_00101796 9 |
| \|\| \|\| \|\| \|\| AI766746 \|\| \|\| \|\| \|\| 331181 | 2400300 | 0.77 | 0.98 | | AI766746 |
| Hs.654493 \|\| SAA4 \|\| Serum amyloid A4, constitutive (1) | Hs.654493 | 0.78 | 0.00 | 6291 | BG564326 |
| Hs.555927 \|\| NECAP1 \|\| NECAP endocytosis associated 1 (1) | Hs.555927 | 0.78 | 0.72 | 25977 | AK094805 |
| Hs.654450 \|\| F8 \|\| Coagulation factor VIII, procoagulant component (hemophilia A) (1) | Hs.654450 | 0.78 | 0.45 | 2157 | NM_000132 |
| Hs.525607 \|\| TNFAIP2 \|\| Tumor necrosis factor, alpha-induced protein 2 (1) | Hs.525607 | 0.78 | 1.67 | 7127 | NM_006291 |
| Hs.135108 \|\| Transcribed locus (1) | Hs.135108 | 0.78 | 0.59 | | DB351265 |
| \|\| \|\| \|\| \|\| R00841 \|\| \|\| \|\| \|\| 102524 | 123604 | 0.78 | 0.98 | | R00841 |
| Hs.534797 \|\| CTNNA1 \|\| **Catenin (cadherin-associated protein), alpha 1, 102kDa (3) | Hs.534797 | 0.78 | 0.59 | 1495 | NM_001903 |
| Hs.348514 \|\| \|\| CDNA FLJ32412 fis, clone SKMUS2000690 (1) | Hs.348514 | 0.78 | 0.98 | | BC014384 |
| Hs.58042 \|\| GFRA3 \|\| GDNF family receptor alpha 3 (1) | Hs.58042 | 0.78 | 1.24 | 2676 | NM_001496 |
| Hs.532357 \|\| TRIM21 \|\| Tripartite motif-containing 21 (1) | Hs.532357 | 0.78 | 0.59 | 6737 | NM_003141 |
| Hs.291533 \|\| \|\| Transcribed locus (1) | Hs.291533 | 0.78 | 0.89 | 6655 | NM_006939 |
| Hs.654819 \|\| ANKRD44 \|\| Ankyrin repeat domain 44 (1) | Hs.654819 | 0.78 | 1.43 | | BX116998 |
| Hs.654841 \|\| SLC35F1 \|\| Solute carrier family 35, member F1 (1) | Hs.654841 | 0.78 | 0.45 | 222553 | BC028615 |
| Hs.310333 \|\| SIGLEC5 \|\| Sialic acid binding Ig-like lectin 5 (1) | Hs.310333 | 0.78 | 0.89 | 8778 | NM_003830 |
| Hs.130036 \|\| \|\| CDNA FLJ42306 fis, clone TRACH2001646 (3) | Hs.130036 | 0.78 | 0.45 | | AK124299 |
| Hs.535011 \|\| FLJ31033 \|\| Hypothetical protein FLJ31033 (1) | Hs.535011 | 0.78 | 0.89 | 91351 | NM_00101296 7 |
| Hs.601138 \|\| \|\| Transcribed locus (1) | Hs.601138 | 0.78 | 1.24 | | AA705222 |
| Hs.441047 \|\| ADM \|\| Adrenomedullin (1) | Hs.441047 | 0.78 | 1.43 | 133 | CR603703 |
| Hs.654395 \|\| FCGR2B \|\| Fc fragment of IgG, low affinity IIb, receptor (CD32) (3) | Hs.654395 | 0.78 | 1.24 | 2213 | AB209585 |
| Hs.372003 \|\| FAM120A \|\| Family with sequence similarity 120A (1) | Hs.372003 | 0.78 | 1.24 | 23196 | BC075701 |
| Hs.522373 \|\| GSN \|\| Gelsolin (amyloidosis, Finnish type) (1) | Hs.522373 | 0.78 | 0.72 | 2934 | AF220263 |
| Hs.154276 \|\| BACH1 \|\| BTB and CNC homology 1, basic leucine zipper transcription factor 1 (1) | Hs.154276 | 0.78 | 2.61 | 571 | NM_206866 |
| \|\| \|\| \|\| \|\| AI269361 \|\| \|\| \|\| \|\| 311681 | 1865232 | 0.78 | 0.72 | | AI269361 |
| Hs.656593 \|\| ELF2 \|\| E74-like factor 2 (ets domain transcription factor) (1) | Hs.656593 | 0.78 | 0.98 | | AI702723 |
| Hs.18676 \|\| SPRY2 \|\| Sprouty homolog 2 (Drosophila) (1) | Hs.18676 | 0.78 | 1.24 | 10253 | BX648582 |
| Hs.73853 \|\| BMP2 \|\| Bone morphogenetic protein 2 (1) | Hs.73853 | 0.78 | 0.98 | 650 | NM_001200 |
| Hs.84084 \|\| APPBP2 \|\| Amyloid beta precursor protein (cytoplasmic tail) binding protein 2 (2) | Hs.84084 | 0.78 | 0.00 | 10513 | NM_006380 |
| Hs.81328 \|\| NFKBIA \|\| Nuclear factor of kappa light polypeptide gene enhancer in B-cells inhibitor, alpha (1) | Hs.81328 | 0.78 | 0.72 | 4792 | BM908726 |
| Hs.644685 \|\| \|\| Transcribed locus (1) | Hs.644685 | 0.78 | 1.24 | | |
| Hs.373550 \|\| TGIF1 \|\| TGFB-induced factor homeobox 1 (1) | Hs.373550 | 0.78 | 0.59 | 7050 | NM_170695 |
| Hs.591715 \|\| SAP30 \|\| Sin3A-associated protein, 30kDa (1) | Hs.591715 | 0.78 | 0.98 | 8819 | BC016757 |
| Hs.49582 \|\| PPP1R12A \|\| Protein phosphatase 1, regulatory (inhibitor) subunit 12A (3) | Hs.49582 | 0.78 | 0.59 | 4659 | AF458589 |
| Hs.7879 \|\| IFRD1 \|\| Interferon-related developmental regulator 1 (3) | Hs.7879 | 0.78 | 1.43 | 3475 | NM_00100724 5 |
| Hs.83722 \|\| EPS15 \|\| Epidermal growth factor receptor pathway substrate 15 (2) | Hs.83722 | 0.78 | 0.72 | 2060 | NM_001981 |
| Hs.654807 \|\| USP34 \|\| Ubiquitin specific peptidase 34 (1) | Hs.654807 | 0.78 | 0.72 | 9736 | NM_014709 |
| Hs.468840 \|\| PLEK \|\| Pleckstrin (1) | Hs.468840 | 0.78 | 1.67 | 5341 | NM_002664 |
| Hs.686786 \|\| \|\| Transcribed locus (1) | Hs.686786 | 0.78 | 1.24 | | |
| Hs.431550 \|\| MAP4K4 \|\| Mitogen-activated protein kinase kinase kinase kinase 4 (3) | Hs.431550 | 0.78 | 0.89 | 9448 | NM_145686 |
| Hs.658033 \|\| KIAA0240 \|\| KIAA0240 (3) | Hs.658033 | 0.78 | 0.89 | 23506 | NM_015349 |
| Hs.501149 \|\| PDZD8 \|\| PDZ domain containing 8 (1) | Hs.501149 | 0.78 | 0.45 | 118987 | BC028375 |
| \|\| \|\| \|\| \|\| R98877 \|\| \|\| \|\| \|\| 117932 | 200863 | 0.78 | 1.67 | | R98877 |
| \|\| \|\| \|\| \|\| AA976477 \|\| \|\| \|\| \|\| 223949 | 1591788 | 0.78 | 1.43 | | AA976477 |
| Hs.480116 \|\| WDFY3 \|\| WD repeat and FYVE domain containing 3 (3) | Hs.480116 | 0.78 | 0.98 | 23001 | NM_014991 |
| Hs.196102 \|\| RB1CC1 \|\| RB1-induciblecoiled-coil 1 (1) | Hs.196102 | 0.78 | 1.67 | 9821 | NM_014781 |
| Hs.202517 \|\| TMEM55A \|\| Transmembrane protein 55A (1) | Hs.202517 | 0.78 | 1.67 | 55529 | BC033892 |
| Hs.591848 \|\| MYST3 \|\| MYST histone acetyltransferase (monocytic leukemia) 3 (1) | Hs.591848 | 0.78 | 2.02 | 7994 | NM_00109941 2 |
| Hs.104019 \|\| TACC3 \|\| Transforming, acidic coiled-coil containing protein 3 (1) | Hs.104019 | 0.78 | 0.59 | 10460 | BC106071 |
| Hs.592115 \|\| TOP3A \|\| Topoisomerase (DNA) III alpha (1) | Hs.592115 | 0.78 | 0.00 | 7156 | NM_004618 |
| Hs.633334 \|\| NUP153 \|\| Nucleoporin 153kDa (1) | Hs.633334 | 0.78 | 0.98 | 9972 | AB210024 |
| Hs.523936 \|\| PRCP \|\| Prolylcarboxypeptidase (angiotensinase C) (1) | Hs.523936 | 0.78 | 0.98 | 5547 | NM_199418 |
| Hs.308628 \|\| ST8SIA4 \|\| ST8 alpha-N-acetyl-neuraminide alpha-2,8-sialyltransferase 4 (3) | Hs.308628 | 0.78 | 1.43 | 7903 | NM_005668 |
| Hs.591338 \|\| TNFAIP3 \|\| Tumor necrosis factor, alpha-induced protein 3 (3) | Hs.591338 | 0.78 | 0.89 | 7128 | BC041790 |
| Hs.269775 \|\| MAP3K7IP2 \|\| Mitogen-activated protein kinase kinase kinase 7 interacting protein 2 (1) | Hs.269775 | 0.78 | 1.24 | 23118 | AB018276 |
| Hs.631951 \|\| MAML1 \|\| Mastermind-like 1 (Drosophila) (1) | Hs.631951 | 0.78 | 1.24 | 9794 | AB209135 |
| Hs.584019 \|\| PPP6C \|\| Protein phosphatase 6, catalytic subunit (1) | Hs.584019 | 0.78 | 0.45 | 5537 | NM_00112335 5 |
| Hs.25647 \|\| FOS \|\| V-fos FBJ murine osteosarcoma viral oncogene homolog (2) | Hs.25647 | 0.78 | 0.98 | 2353 | BX647104 |
| Hs.367725 \|\| GATA2 \|\| GATA binding protein 2 (2) | Hs.367725 | 0.78 | 0.98 | 2624 | AK127845 |
| Hs.410889 \|\| TANC2 \|\| Tetratricopeptide repeat, ankyrin repeat and coiled-coil containing 2 (1) | Hs.410889 | 0.78 | 0.59 | 26115 | NM_025185 |
| Hs.478673 \|\| L1RAP \|\| Interleukin 1 receptor accessory protein (3) | Hs.478673 | 0.78 | 1.67 | 3556 | NM_002182 |
| Hs.464595 \|\| PPP4R1 \|\| Protein phosphatase 4, regulatory subunit 1 (1) | Hs.464595 | 0.78 | 0.59 | 9989 | NM_00104238 8 |
| Hs.496414 \|\| ATP7A \|\| ATPase, Cu++ transporting, alpha polypeptide (Menkes syndrome) (1) | Hs.496414 | 0.79 | 1.67 | 538 | L06133 |
| Hs.463964 \|\| WIP11 \|\| WD repeat domain, phosphoinositide interacting 1 (1) | Hs.463964 | 0.79 | 0.45 | 55062 | NM_017983 |
| Hs.519756 \|\| STK10 \|\| Serine/threonine kinase 10 (2) | Hs.519756 | 0.79 | 0.72 | 6793 | NM_005990 |
| Hs.459070 \|\| ARNT2 \|\| Aryl-hydrocarbon receptor nuclear translocator 2 (1) | Hs.459070 | 0.79 | 1.24 | 9915 | NM_014862 |
| Hs.192326 \|\| SNX27 \|\| Sorting nexin family member 27 (1) | Hs.192326 | 0.79 | 0.98 | 81609 | NM_030918 |
| Hs.462492 \|\| USP22 \|\| Ubiquitin specific peptidase 22 (1) | Hs.462492 | 0.79 | 0.45 | 23326 | AB028986 |
| Hs.406135 \|\| MGC70863 \|\| Similar to RPL23AP7 protein (1) | Hs.406135 | 0.79 | 0.45 | 284942 | BC065556 |
| Hs.529862 \|\| CSNK1A1 \|\| Casein kinase 1, alpha 1 (2) | Hs.529862 | 0.79 | 0.00 | 1452 | BX647478 |
| Hs.431850 \|\| MAPK1 \|\| Mitogen-activated protein kinase 1 (3) | Hs.431850 | 0.79 | 0.59 | 5594 | AL157438 |
| Hs.149261 \|\| RUNX1 \|\| Runt-related transcription factor 1 (acute myeloid leukemia 1; aml1 oncogene) (1) | Hs.149261 | 0.79 | 1.67 | 861 | NM_00100189 0 |
| Hs.268675 \|\| MEF2A \|\| Myocyte enhancer factor 2A (2) | Hs.268675 | 0.79 | 0.89 | 4205 | AL831995 |
| Hs.654497 \|\| LTBP1 \|\| Latent transforming growth factor beta binding protein 1 (1) | Hs.654497 | 0.79 | 0.98 | 4052 | NM_206943 |
| Hs.18631 \|\| MSL2L1 \|\| Male-specific lethal 2-like 1 (Drosophila) (1) | Hs.18631 | 0.79 | 0.98 | 55167 | NM_018133 |
| Hs.477892 \|\| GYG1 \|\| Glycogenin 1 (2) | Hs.477892 | 0.79 | 0.98 | 2992 | CR601242 |
| Hs.15738 \|\| RAB7A \|\| RAB7A, member RAS oncogene family (2) | Hs.15738 | 0.79 | 0.59 | 7879 | NM_004637 |
| Hs.546259 \|\| H3F3A \|\| H3 histone, family 3A (1) | Hs.546259 | 0.79 | 1.67 | 3020 | CR621187 |
| Hs.838 \|\| CD80 \|\| CD80 molecule (1) | Hs.838 | 0.79 | 0.72 | 941 | BC042665 |
| Hs.523360 \|\| INPP5A \|\| **Inositol polyphosphate-5-phosphatase, 40kDa (1) | Hs.523360 | 0.79 | 1.43 | | CA442008 |
| Hs.248472 \|\| ITGAX \|\| Integrin, alpha X (complement component 3 receptor 4 subunit) (1) | Hs.248472 | 0.79 | 1.43 | 3687 | NM_000887 |
| \|\| \|\| \|\| \|\| AI312516 \|\| \|\| \|\| \|\| 308714 | 1917588 | 0.79 | 0.72 | | AI312516 |
| Hs.517946 \|\| KLHL18 \|\| Kelch-like 18 (Drosophila) (1) | Hs.517946 | 0.79 | 0.98 | 23276 | AB062478 |
| Hs.469970 \|\| SFRS4 \|\| Splicing factor, arginine/serine-rich 4 (1) | Hs.469970 | 0.79 | 2.61 | 6429 | AB209856 |
| Hs.462590 \|\| MY018A \|\| Myosin XVIIIA (1) | Hs.462590 | 0.79 | 0.45 | 9220 | AK126861 |
| Hs.662836 \|\| \|\| Transcribed locus (1) | Hs.662836 | 0.79 | 0.72 | | AW971961 |
| Hs.124126 \|\| ARPC1A \|\| Actin related protein 2/3 complex, subunit 1A, 41kDa (1) | Hs.124126 | 0.79 | 1.24 | 10552 | NM_006409 |
| Hs.660511 \|\| \|\| Transcribed locus (1) | Hs.660511 | 0.79 | 0.89 | | BM673919 |
| Hs.116459 \|\| MAN2A2 \|\| Mannosidase, alpha, class 2A, member 2 (3) | Hs.116459 | 0.79 | 1.75 | 4122 | NM_006122 |
| Hs.655325 \|\| RHOT1 \|\| Ras homolog gene family, member T1 (1) | Hs.655325 | 0.79 | 1.43 | 55288 | NM_00103356 8 |
| Hs.520627 \|\| IQCE \|\| IQ motif containing E (1) | Hs.520627 | 0.79 | 0.98 | 23288 | NM_152558 |
| Hs.446559 \|\| PCNX \|\| Pecanex homolog (Drosophila) (2) | Hs.446559 | 0.79 | 0.98 | 22990 | NM_014982 |
| Hs.591184 \|\| PPM1D \|\| Protein phosphatase 1D magnesium-dependent, delta isoform (1) | Hs.591184 | 0.79 | 1.75 | 8493 | NM_003620 |
| Hs.274256 \|\| ELOVL7 \|\| ELOVL family member 7, elongation of long chain fatty acids (yeast) (1) | Hs.274256 | 0.79 | 0.72 | 79993 | BC094792 |
| Hs.591619 \|\| HRB \|\| HIV- Rev binding protein (3) | Hs.591619 | 0.79 | 1.67 | 3267 | NM_004504 |
| Hs.583391 \|\| NOMO1 \|\| NODAL modulator 1 (1) | Hs.583391 | 0.79 | 0.98 | 23420 | NM_014287 |
| Hs.407580 \|\| PKP4 \|\| Plakophilin 4 (1) | Hs.407580 | 0.79 | 0.59 | 8502 | NM_003628 |
| Hs.444517 \|\| FAM44A \|\| Family with sequence similarity 44, member A (2) | Hs.444517 | 0.79 | 1.43 | 259282 | NM_148894 |
| \|\| \|\| \|\| \|\| T66794 \|\| \|\| \|\| \|\| 104597 | 66316 | 0.79 | 0.59 | | T66794 |
| Hs.602604 \|\| \|\| Transcribed locus (1) | Hs.602604 | 0.79 | 0.98 | | AI000142 |
| Hs.533050 \|\| NQ02 \|\| NAD(P)H dehydrogenase, quinone 2 (1) | Hs.533050 | 0.79 | 1.67 | 4835 | AB209779 |
| Hs.536218 \|\| \|\| Transcribed locus (1) | Hs.536218 | 0.79 | 1.75 | | BQ706262 |
| Hs.501345 \|\| CTBP2 \|\| C-terminal binding protein 2 (3) | Hs.501345 | 0.79 | 1.24 | 1488 | NM_022802 |
| Hs.495473 \|\| NOTCH1 \|\| Notch homolog 1, translocation-associated (Drosophila) (2) | Hs.495473 | 0.79 | 0.98 | 4851 | NM_017617 |
| Hs.584806 \|\| TCEB3 \|\| Transcription elongation factor B (SIII), polypeptide 3 (110kDa, elongin A) (1) | Hs.584806 | 0.79 | 2.15 | 6924 | AK096079 |
| Hs.370168 \|\| CRLF3 \|\| Cytokine receptor-like factor 3 (1) | Hs.370168 | 0.79 | 2.61 | 51379 | NM_015986 |
| Hs.524750 \|\| NUP98 \|\| Nucleoporin 98kDa (1) | Hs.524750 | 0.79 | 0.72 | 4928 | NM_016320 |
| Hs.655772 \|\| ACSL3 \|\| Acyl-CoA synthetase long-chain family member 3 (3) | Hs.655772 | 0.79 | 0.59 | 2181 | NM_004457 |
| Hs.61481 \|\| \|\| **Transcribed locus (1) | Hs.61481 | 0.79 | 1.67 | | BX113895 |
| Hs.83731 \|\| CD33 \|\| CD33 molecule (1) | Hs.83731 | 0.79 | 0.59 | 945 | BC028152 |
| Hs.430742 \|\| PFTK1 \|\| PFTAIRE protein kinase 1 (1) | Hs.430742 | 0.79 | 0.59 | 5218 | BC167152 |
| Hs.593027 \|\| \|\| CDNA FLJ26120 fis, clone SYN00419 (1) | Hs.593027 | 0.79 | 3.52 | | AK129631 |
| Hs.599934 \|\| \|\| Transcribed locus (1) | Hs.599934 | 0.79 | 1.43 | | AI807147 |
| Hs.591293 \|\| ALCAM \|\| Activated leukocyte cell adhesion molecule (1) | Hs.591293 | 0.79 | 1.24 | 214 | NM_001627 |
| Hs.177744 \|\| FLJ32569 \|\| Hypothetical protein FLJ32569 (1) | Hs.177744 | 0.79 | 0.59 | 148811 | BC063477 |
| Hs.298990 \|\| SMARCA2 \|\| SWI/SNF related, matrix associated, actin dependent regulator of chromatin, subfamily a, member 2 (1) | Hs.298990 | 0.79 | 1.67 | 6595 | X72889 |
| Hs.306769 \|\| RUFY1 \|\| RUN and FYVE domain containing 1 (1) | Hs.306769 | 0.79 | 1.43 | 80230 | NM_025158 |
| Hs.494457 \|\| NINJ1 \|\| Ninjurin 1 (1) | Hs.494457 | 0.79 | 1.67 | 4814 | BQ073581 |
| Hs.150557 \|\| KLF9 \|\| Kruppel-like factor 9 (1) | Hs.150557 | 0.79 | 1.43 | 687 | NM_001206 |
| Hs.591227 \|\| PARP4 \|\| Poly (ADP-ribose) polymerase family, member 4 (1) | Hs.591227 | 0.79 | 0.59 | 143 | AF158255 |
| Hs.494192 \|\| OSTF1 \|\| Osteoclast stimulating factor 1 (2) | Hs.494192 | 0.79 | 0.98 | 26578 | AK091034 |
| Hs.153563 \|\| LY75 \|\| Lymphocyte antigen 75 (1) | Hs.153563 | 0.79 | 0.59 | 4065 | AF011333 |
| Hs.280342 \|\| PRKAR1A \|\| Protein kinase, cAMP-dependent, regulatory, type I, alpha (tissue specific extinguisher 1) (3) | Hs.280342 | 0.80 | 1.75 | 5573 | CR749311 |
| Hs.414183 \|\| LOC349114 \|\| Hypothetical LOC349114 (2) | Hs.414183 | 0.80 | 1.43 | | CN280709 |
| Hs.570423 \|\| SAMSN1 \|\| SAM domain, SH3 domain and nuclear localization signals 1 (2) | Hs.570423 | 0.80 | 1.24 | 64092 | AF218085 |
| Hs.110488 \|\| CHSY1 \|\| Carbohydrate (chondroitin) synthase 1 (1) | Hs.110488 | 0.80 | 2.02 | 22856 | BC046247 |
| Hs.632707 \|\| MARS \|\| Methionyl-tRNA synthetase (1) | Hs.632707 | 0.80 | 0.89 | 4141 | AK122956 |
| Hs.655392 \|\| SAFB2 \|\| Scaffold attachment factor B2 (2) | Hs.655392 | 0.80 | 0.98 | 9667 | NM_014649 |
| Hs.377894 \|\| GCA \|\| Grancalcin, EF-hand calcium binding protein (1) | Hs.377894 | 0.80 | 0.98 | 25801 | BC005214 |
| Hs.473254 \|\| OSBPL2 \|\| Oxysterol binding protein-like 2 (2) | Hs.473254 | 0.80 | 1.24 | 9885 | NM_144498 |
| Hs.465337 \|\| PHLPP \|\| PH domain and leucine rich repeat protein phosphatase (1) | Hs.465337 | 0.80 | 1.24 | 23239 | AB011178 |
| Hs.195080 \|\| ECE1 \|\| Endothelin converting enzyme 1 (2) | Hs.195080 | 0.80 | 0.98 | 1889 | NM_00111334 7 |
| Hs.507680 \|\| PFAAP5 \|\| Phosphonoformate immuno-associated protein 5 (3) | Hs.507680 | 0.80 | 1.24 | 10443 | NM_014887 |
| Hs.467587 \|\| NBPF1 \|\| Neuroblastoma breakpoint family, member 1 (2) | Hs.467587 | 0.80 | 1.43 | 55672 | AY894575 |
| Hs.75277 \|\| RMND5A \|\| Required for meiotic nuclear division 5 homolog A (S. cerevisiae) (2) | Hs.75277 | 0.80 | 0.72 | 64795 | AL832022 |
| \|\| \|\| \|\| \|\| AW005212 \|\| \|\| \|\| \|\| 330971 | 2565546 | 0.80 | 0.98 | | AW005212 |
| Hs.591273 \|\| FNIP1 \|\| Folliculin interacting protein 1 (1) | Hs.591273 | 0.80 | 2.02 | 96459 | NM_133372 |
| Hs.169054 \|\| \|\| CDNA FLJ43532 fis, clone PLACE7001022 (1) | Hs.169054 | 0.80 | 1.67 | 100129380 | AK125520 |
| Hs.513683 \|\| PSKH1 \|\| Protein serine kinase H1 (1) | Hs.513683 | 0.80 | 1.43 | 5681 | BC062616 |
| Hs.195403 \|\| DOCK5 \|\| Dedicator of cytokinesis 5 (2) | Hs.195403 | 0.80 | 1.24 | 80005 | NM_024940 |
| Hs.34341 \|\| CD58 \|\| CD58 molecule (1) | Hs.34341 | 0.80 | 0.98 | 965 | AK125710 |
| Hs.655286 \|\| ZBTB11 \|\| Zinc finger and BTB domain containing 11 (1) | Hs.655286 | 0.80 | 2.98 | | BC028062 |
| Hs.82028 \|\| TGFBR2 \|\| Transforming growth factor, beta receptor II (70/80kDa) (1) | Hs.82028 | 0.80 | 2.02 | 7048 | BX648313 |
| Hs.1239 \|\| ANPEP \|\| Alanyl (membrane) aminopeptidase (aminopeptidase N, aminopeptidase M, microsomal aminopeptidase, CD13, p150) (1) | Hs.1239 | 0.80 | 1.43 | 290 | NM_001150 |
| Hs.499984 \|\| SGPL1 \|\| Sphingosine-1-phosphate lyase 1 (2) | Hs.499984 | 0.80 | 0.59 | 8879 | AB033078 |
| Hs.474596 \|\| LIMK2 \|\| LIM domain kinase 2 (1) | Hs.474596 | 0.80 | 2.61 | 3985 | NM_016733 |
| Hs.75275 \|\| UBE4A \|\| Ubiquitination factor E4A (UFD2 homolog, yeast) (1) | Hs.75275 | 0.80 | 1.67 | 9354 | NM_004788 |
| \|\| \|\| \|\| \|\| R36464 \|\| \|\| \|\| \|\| 110824 | 136819 | 0.80 | 0.45 | | R36464 |
| Hs.497417 \|\| KIAA0317 \|\| KIAA0317 (1) | Hs.497417 | 0.80 | 1.43 | 9870 | NM_00103947 9 |
| Hs.105636 Transcribed locus (2) | Hs.105636 | 0.80 | 1.24 | | BX417162 |
| Hs.321541 \|\| RAB11A \|\| RAB11A, member RAS oncogene family (1) | Hs.321541 | 0.80 | 1.75 | 8766 | BC013348 |
| Hs.19385 \|\| ABHD5 \|\| Abhydrolase domain containing 5 (1) | Hs.19385 | 0.80 | 2.02 | 51099 | AK022457 |
| \|\| \|\| \|\| \|\| R16566 \|\| \|\| \|\| \|\| 98710 | 129629 | 0.80 | 0.89 | | R16566 |
| Hs.632137 \|\| CUGBP1 \|\| CUG triplet repeat, RNA binding protein 1 (1) | Hs.632137 | 0.80 | 1.67 | 10658 | AB210019 |
| Hs.591947 \|\| ST3GAL4 \|\| ST3 beta-galactoside alpha-2,3-sialyltransferase 4 (1) | Hs.591947 | 0.80 | 1.43 | 6484 | AK128605 |
| Hs.682406 \|\| \|\| Transcribed locus (1) | Hs.682406 | 0.80 | 0.98 | | CD252193 |
| Hs.434951 \|\| USP15 \|\| Ubiquitin specific peptidase 15 (1) | Hs.434951 | 0.80 | 4.44 | 9958 | AF106069 |
| Hs.478588 \|\| BCL6 \|\| B-cell CLL/lymphoma 6 (zinc finger protein 51) (3) | Hs.478588 | 0.80 | 1.24 | 604 | NM_001706 |
| Hs.279245 \|\| TACC1 \|\| Transforming, acidic coiled-coil containing protein 1 (2) | Hs.279245 | 0.80 | 1.67 | 6867 | CR933618 |
| Hs.380774 \|\| DDX3X \|\| DEAD (Asp-Glu-Ala-Asp) box polypeptide 3, X-linked (2) | Hs.380774 | 0.80 | 2.61 | 1654 | NM_001356 |
| Hs.94499 \|\| \|\| Transcribed locus (1) | Hs.94499 | 0.80 | 2.98 | | BF683837 |
| Hs.269988 \|\| ROD1 \|\| ROD1 regulator of differentiation 1 (S. pombe) (2) | Hs.269988 | 0.80 | 1.75 | 9991 | NM-005156 |
| Hs.653460 \|\| \|\| **Transcribed locus (1) | Hs.653460 | 0.80 | 2.02 | | |
| Hs.660872 \|\| \|\| Transcribed locus (1) | Hs.660872 | 0.80 | 2.02 | | CB163724 |
| Hs.518530 \|\| PAK2 \|\| P21 (CDKN1A)-activated kinase 2 (4) | Hs.518530 | 0.80 | 0.98 | 5062 | NM_002577 |
| Hs.60371 \|\| \|\| Transcribed locus (1) | Hs.60371 | 0.80 | 0.98 | | CA439310 |
| Hs.25245 \|\| Clorf103 \|\| Chromosome 1 open reading frame 103 (1) | Hs.25245 | 0.80 | 2.02 | 55791 | NM_018372 |
| Hs.666614 \|\| \|\| Transcribed locus (1) | Hs.666614 | 0.80 | 1.43 | | AI014709 |
| Hs.81134 \|\| IL1RN \|\| Interleukin 1 receptor antagonist (1) | Hs.81134 | 0.80 | 1.75 | 3557 | BC068441 |
| \|\| \|\| \|\| \|\| W86423 \|\| \|\| \|\| \|\| 98762 | 416539 | 0.80 | 0.98 | | W86423 |
| Hs.590886 \|\| RXRA \|\| Retinoid X receptor, alpha (1) | Hs.590886 | 0.80 | 1.43 | 6256 | AK131081 |
| Hs.440219 \|\| UBN1 \|\| Ubinuclein 1 (2) | Hs.440219 | 0.80 | 1.75 | 29855 | NM_016936 |
| Hs.594448 \|\| \|\| Transcribed locus, moderately similar to XP_001091341.1 FAT tumor suppressor 1, partial [Macaca mulatta] (2) | Hs.594448 | 0.80 | 1.43 | | CR748750 |
| Hs.659124 \|\| \|\| **Full-length cDNA clone CS0DC002YO07 of Neuroblastoma Cot 25-normalized of Homo sapiens (human) (1) | Hs.659124 | 0.80 | 2.61 | | BM804445 |
| Hs.560 \|\| APOBEC1 \|\| Apolipoprotein B mRNA editing enzyme, catalytic polypeptide 1 (1) | Hs.560 | 0.80 | 0.89 | 339 | NM_001644 |
| Hs.416769 \|\| PPM1B \|\| Protein phosphatase 1B (formerly 2C), magnesium-dependent, beta isoform (2) | Hs.416769 | 0.81 | 2.15 | 5495 | NM_177968 |
| Hs.158932 \|\| APC \|\| Adenomatosis polyposis coli (2) | Hs.158932 | 0.81 | 0.98 | 324 | NM_00112751 1 |
| Hs.594613 \|\| Transcribed locus (1) | Hs.594613 | 0.81 | 1.24 | | |
| Hs.499833 \|\| REEP3 \|\| Receptor accessory protein 3 (1) | Hs.499833 | 0.81 | 2.15 | 221035 | CR936686 |
| Hs.479451 \|\| CENTD1 \|\| Centaurin, delta 1 (1) | Hs.479451 | 0.81 | 1.67 | 116984 | NM_015230 |
| Hs.196384 \|\| PTGS2 \|\| Prostaglandin-endoperoxide synthase 2 (prostaglandin G/H synthase and cyclooxygenase) (3) | Hs.196384 | 0.81 | 2.98 | 5743 | NM_000963 |
| Hs.128841 \|\| \|\| CDNA FLJ41537 fis, clone BRTHA2017985 (1) | Hs.128841 | 0.81 | 2.15 | | AK123531 |
| Hs.77578 \|\| USP9X \|\| Ubiquitin specific peptidase 9, X-linked (5) | Hs.77578 | 0.81 | 0.59 | 8239 | NM_00103959 0 |
| Hs.300863 \|\| SGK2 \|\| Serum/glucocorticoid regulated kinase 2 (1) | Hs.300863 | 0.81 | 4.44 | 10110 | BC006523 |
| Hs.499209 \|\| SVIL \|\| Supervillin (2) | Hs.499209 | 0.81 | 2.61 | 6840 | NM_021738 |
| Hs.633061 \|\| \|\| Transcribed locus (1) | Hs.633061 | 0.81 | 2.61 | | BQ719750 |
| Hs.659469 \|\| \|\| Transcribed locus (1) | Hs.659469 | 0.81 | 0.98 | | BE072767 |
| Hs.120998 \|\| \|\| NPP5E \|\| Inositol polyphosphate-5-phosphatase, 72 kDa (1) | Hs.120998 | 0.81 | 2.61 | 56623 | BC028032 |
| Hs.655067 \|\| FAM128A \|\| Family with sequence similarity 128, member A (1) | Hs.655067 | 0.81 | 4.44 | 653784 | AK090531 |
| Hs.658543 \|\| \|\| Transcribed locus (1) | Hs.658543 | 0.81 | 2.61 | | |
| Hs.182626 \|\| C22orf5 \|\| Chromosome 22 open reading frame 5 (2) | Hs.182626 | 0.81 | 2.15 | 25829 | CR627445 |
| Hs.446079 \|\| CPD \|\| Carboxypeptidase D (3) | Hs.446079 | 0.81 | 0.98 | 1362 | NM_001304 |
| \|\| \|\| \|\| \|\| H90767 \|\| \|\| \|\| \|\| 109297 | 240509 | 0.81 | 2.02 | | H90767 |
| Hs.34494 \|\| AGXT2 \|\| Alanine-glyoxylate aminotransferase 2 (1) | Hs.34494 | 0.81 | 1.67 | 64902 | AK223144 |
| Hs.591751 \|\| FAM105A \|\| Family with sequence similarity 105, member A (1) | Hs.591751 | 0.81 | 1.67 | 54491 | AK001989 |
| Hs.91393 \|\| LIN7C \|\| Lin-7 homolog C (C. elegans) (1) | Hs.91393 | 0.81 | 1.43 | 55327 | BC053907 |
| Hs.26837 \|\| TRIM33 \|\| Tripartite motif-containing 33 (3) | Hs.26837 | 0.81 | 0.59 | 51592 | NM_015906 |
| Hs.654980 \|\| \|\| CDNA FLJ33220 fis, clone ASTR02000482 (1) | Hs.654980 | 0.81 | 1.43 | 100129034 | XM_001726040 |
| Hs.434401 \|\| ZNF638 \|\| Zinc finger protein 638 (2) | Hs.434401 | 0.81 | 1.24 | 27332 | CR749322 |
| Hs.525358 \|\| \|\| Transcribed locus (1) | Hs.525358 | 0.81 | 4.01 | | AI655822 |
| Hs.1867 \|\| PGC \|\| Progastricsin (pepsinogen C) (1) | Hs.1867 | 0.81 | 0.72 | 5225 | BQ067503 |
| Hs.516830 \|\| SRXN1 \|\| Sulfiredoxin 1 homolog (S. cerevisiae) (1) | Hs.516830 | 0.81 | 1.75 | 140809 | AK125343 |
| Hs.155396 \|\| NFE2L2 \|\| Nuclear factor (erythroid-derived 2)-like 2 (2) | Hs.155396 | 0.81 | 2.61 | 4780 | NM_006164 |
| Hs.344400 \|\| MPHOSPH6 \|\| M-phase phosphoprotein 6 (2) | Hs.344400 | 0.81 | 1.24 | 10200 | BX537773 |
| Hs.664595 \|\| \|\| Transcribed locus (1) | Hs.664595 | 0.81 | 4.01 | | AI760944 |
| Hs.27695 \|\| MID \|\| Midline 1 (Opitz/BBB syndrome) (1) | Hs.27695 | 0.81 | 1.43 | 4281 | NM_000381 |
| Hs.516522 \|\| INTS3 \|\| Integrator complex subunit 3 (1) | Hs.516522 | 0.81 | 0.98 | 65123 | CR749376 |
| Hs.172631 \|\| ITGAM \|\| Integrin, alpha M (complement component 3 receptor 3 subunit) (2) | Hs.172631 | 0.81 | 1.43 | 3684 | NM_000632 |
| Hs.299878 \|\| DEGS1 \|\| Degenerative spermatocyte homolog 1, lipid desaturase (Drosophila) (2) | Hs.299878 | 0.81 | 0.72 | 8560 | NM_003676 |
| Hs.603732 \|\| NCKAP1 \|\| NCK-associated protein 1 (1) | Hs.603732 | 0.81 | 3.52 | 10787 | AB011159 |
| \|\| \|\| \|\| \|\| W84667 \|\| \|\| \|\| \|\| 100587 | 415715 | 0.81 | 1.75 | | BX099504 |
| Hs.496684 \|\| LAMP2 \|\| Lysosomal-associated membrane protein 2 (1) | Hs.496684 | 0.81 | 2.15 | 3920 | NM_002294 |
| Hs.131226 \|\| BNIP3L \|\| BCL2/adenovirus E1B 19kDa interacting protein 3-like (2) | Hs.131226 | 0.81 | 1.67 | 665 | AF370457 |
| Hs.365286 \|\| FAM21C \|\| Family with sequence similarity 21, member C (1) | Hs.365286 | 0.81 | 2.98 | 253725 | AB011164 |
| Hs.370247 \|\| APLP2 \|\| Amyloid beta (A4) precursor-like protein 2 (1) | Hs.370247 | 0.81 | 2.15 | | BF208895 |
| Hs.555996 \|\| HIATL1 \|\| Hippocampus abundant transcript-like 1 (2) | Hs.555996 | 0.81 | 2.02 | | AF130099 |
| Hs.356055 \|\| \|\| CDNA FLJ35491 fis, clone SMINT2008625, moderately similar to GLYCINE CLEAVAGE SYSTEM H PROTEIN PRECURSOR (1) | Hs.356055 | 0.81 | 2.61 | | AK092810 |
| Hs.146551 BMP2K \|\| BMP2 inducible kinase (2) | Hs.146551 | 0.81 | 0.59 | 55589 | BX641162 |
| \|\| \|\| \|\| \|\| AA683336 \|\| \|\| \|\| \|\| 221963 | 1293010 | 0.81 | 4.44 | | AA776723 |
| Hs.647069 \|\| SLC4A2 \|\| Solute carrier family 4, anion exchanger, member 2 (erythrocyte membrane protein band 3-like 1) (1) | Hs.647069 | 0.81 | 0.72 | 6522 | AB209158 |
| Hs.396189 \|\| FLJ39575 \|\| Hypothetical protein FLJ39575 (1) | Hs.396189 | 0.81 | 1.75 | 286006 | NM_182597 |
| Hs.419859 \|\| LOC162632 \|\| TL132 pseudogene (1) | Hs.419859 | 0.81 | 0.98 | 284047 | EF553518 |
| Hs.591785 \|\| IER3 \|\| Immediate early response 3 (1) | Hs.591785 | 0.81 | 1.75 | 8870 | BM906591 |
| Hs.659565 \|\| LOC128977 \|\| Hypothetical protein LOC128977 (1) | Hs.659565 | 0.81 | 2.15 | 128977 | AK095271 |
| Hs.207763 \|\| MAPK8IP3 \|\| Mitogen-activated protein kinase 8 interacting protein 3 (1) | Hs.207763 | 0.81 | 1.67 | 23162 | NM_015133 |
| Hs.645283 \|\| RTN4 \|\| Reticulon 4 (1) | Hs.645283 | 0.81 | 3.52 | 57142 | NM_020532 |
| Hs.584996 \|\| FAM40A \|\| Family with sequence similarity 40, member A (1) | Hs.584996 | 0.81 | 2.02 | 85369 | AL834196 |
| Hs.386168 \|\| IHPK1 \|\| Inositol hexaphosphate kinase 1 (1) | Hs.386168 | 0.81 | 1.43 | 9807 | NM_153273 |
| \|\| \|\| \|\| \|\| AA609715 \|\| \|\| \|\| \|\| 308183 | 1031865 | 0.81 | 0.89 | | AA609715 |
| Hs.205558 \|\| GLIPR1 \|\| GLI pathogenesis-related 1 (glioma) (1) | Hs.205558 | 0.81 | 2.61 | 11010 | NM_006851 |
| \|\| \|\| \|\| \|\| R06362 \|\| \|\| \|\| \|\| 115625 | 126234 | 0.81 | 1.75 | | R06362 |
| Hs.440025 \|\| KIAA0247 \|\| KIAA0247 (2) | Hs.440025 | 0.81 | 0.98 | 9766 | BC064697 |
| Hs.501452 \|\| EBI3 \|\| Epstein-Barr virus induced gene 3 (1) | Hs.501452 | 0.81 | 1.43 | 10148 | BM919170 |
| Hs.632267 \|\| SDC4 \|\| Syndecan 4 (1) | Hs.632267 | 0.81 | 1.75 | 6385 | NM_002999 |
| Hs.126057 \|\| FRAT1 \|\| Frequently rearranged in advanced T-cell lymphomas (1) | Hs.126057 | 0.81 | 1.24 | 10023 | NM_005479 |
| Hs.593995 \|\| TCF7L2 \|\| Transcription factor 7-like 2 (T-cell specific, HMG-box) (1) | Hs.593995 | 0.81 | 2.61 | 6934 | BX648364 |
| Hs.654830 \|\| \|\| MRNA; cDNA DKFZp451O1818 (from clone DKFZp451O1818) (3) | Hs.654830 | 0.81 | 1.75 | | AL832650 |
| Hs.267120 \|\| RNF146 \|\| Ring finger protein 146 (1) | Hs.267120 | 0.81 | 0.98 | 81847 | AL136829 |
| Hs.487994 \|\| KIAA1706 \|\| KIAA1706 protein (1) | Hs.487994 | 0.81 | 1.67 | 80820 | NM_030636 |
| Hs.173288 \|\| CTR9 \|\| Ctr9, Pafl/RNA polymerase II complex component, homolog (S. cerevisiae) (2) | Hs.173288 | 0.81 | 2.98 | | BF339109 |
| Hs.592035 \|\| LOC647057 \|\| Similar to carbonic anhydrase VA, mitochondrial precursor (1) | Hs.592035 | 0.81 | 1.24 | | AA699469 |
| Hs.25829 \|\| RASD1 \|\| RAS, dexamethasone-induced 1 (1) | Hs.25829 | 0.81 | 0.98 | 51655 | BC042688 |
| \|\| \|\| \|\| \|\| R08184 \|\| \|\| \|\| \|\| 106980 | 127230 | 0.81 | 0.98 | | BM679514 |
| Hs.85658 \|\| ZNF768 \|\| Zinc finger protein 768 (1) | Hs.85658 | 0.82 | 1.43 | 79724 | NM-024671 |
| Hs.591602 \|\| SSFA2 \|\| Sperm specific antigen 2 (1) | Hs.591602 | 0.82 | 4.44 | 6744 | BX648182 |
| Hs.193784 \|\| \|\| MRNA; cDNA DKFZp586K1922 (from clone DKFZp586K1922) (1) | Hs.193784 | 0.82 | 4.44 | | AL110204 |
| Hs.789 \|\| CXCL1 \|\| Chemokine (C-X-C motif) ligand 1 (melanoma growth stimulating activity, alpha) (2) | Hs.789 | 0.82 | 2.15 | 2919 | BF032655 |
| Hs.529488 \|\| SLC6A6 \|\| Solute carrier family 6 (neurotransmitter transporter, taurine), member 6 (1) | Hs.529488 | 0.82 | 0.98 | 6533 | NM_003043 |
| Hs.654939 \|\| KIAA0892 \|\| KIAA0892 (1) | Hs.654939 | 0.82 | 1.67 | 23383 | NM_015329 |
| Hs.444870 \|\| ORC2L \|\| Origin recognition complex, subunit 2-like (yeast) (2) | Hs.444870 | 0.82 | 0.72 | 4999 | NM_006190 |
| Hs.118400 \|\| FSCN1 \|\| Fascin homolog 1, actin-bundling protein (Strongylocentrotus purpuratus) (1) | Hs.118400 | 0.82 | 2.15 | 6624 | NM_003088 |
| Hs.508148 \|\| ABI1 \|\| Abl-interactor 1 (1) | Hs.508148 | 0.82 | 2.98 | 10006 | NM_005470 |
| Hs.467769 \|\| FAM49A \|\| Family with sequence similarity 49, member A (1) | Hs.467769 | 0.82 | 1.67 | 81553 | AK055334 |
| Hs.73799 \|\| GNAI3 \|\| Guanine nucleotide binding protein (G protein), alpha inhibiting activity polypeptide 3 (1) | Hs.73799 | 0.82 | 1.43 | 2773 | NM_006496 |
| Hs.694759 \|\| GTF2IP1 \|\| General transcription factor II, i, pseudogene 1 (2) | Hs.694759 | 0.82 | 3.52 | | BC045632 |
| Hs.153381 \|\| DARC \|\| Duffy blood group, chemokine receptor (1) | Hs.153381 | 0.82 | 1.75 | 2532 | AK291593 |
| Hs.6551 \|\| ATP6AP1 \|\| ATPase, H+ transporting, lysosomal accessory protein 1 (1) | Hs.6551 | 0.82 | 2.61 | 537 | NM_001183 |
| Hs.662446 \|\| \|\| Transcribed locus (1) | Hs.662446 | 0.82 | 1.75 | | AA416843 |
| Hs.519294 \|\| FBN2 \|\| Fibrillin 2 (congenital contractural arachnodactyly) (1) | Hs.519294 | 0.82 | 3.52 | 2201 | NM_001999 |
| Hs.104661 \|\| C19orf7 \|\| Chromosome 19 open reading frame 7 (1) | Hs.104661 | 0.82 | 1.67 | 23211 | NM_015168 |
| Hs.159430 \|\| FNDC3B \|\| Fibronectin type III domain containing 3B (5) | Hs.159430 | 0.82 | 2.98 | 64778 | NM_022763 |
| Hs.117030 \|\| LOC646708 \|\| Similar to cysteine string protein (1) | Hs.117030 | 0.82 | 2.15 | | BX102278 |
| Hs.308480 \|\| PCMTD1 \|\| Protein-L-isoaspartate (D-aspartate) O-methyltransferase domain containing 1 (1) | Hs.308480 | 0.82 | 2.15 | 115294 | BC032670 |
| Hs.861 \|\| MAPK3 \|\| Mitogen-activated protein kinase 3 (1) | Hs.861 | 0.82 | 2.61 | 5595 | BX537897 |
| Hs.556108 \|\| \|\| MRNA; cDNA DKFZp547K189 (from clone DKFZp547K189) (1) | Hs.556108 | 0.82 | 2.61 | | AK091492 |
| Hs.657672 \|\| \|\| Transcribed locus (1) | Hs.657672 | 0.82 | 1.43 | | |
| Hs.472027 \|\| CDS2 \|\| CDP-diacylglycerol synthase (phosphatidate cytidylyltransferase) 2 (1) | Hs.472027 | 0.82 | 2.98 | | AI681872 |
| Hs.406758 \|\| HIBADH \|\| 3-hydroxyisobutyrate dehydrogenase (1) | Hs.406758 | 0.82 | 1.24 | 11112 | AK025558 |
| Hs.632918 \|\| PGAM1 \|\| Phosphoglycerate mutase 1 (brain) (1) | Hs.632918 | 0.82 | 2.61 | 5223 | BF312409 |
| Hs.693916 \|\| \|\| Transcribed locus (1) | Hs.693916 | 0.82 | 2.02 | | |
| Hs.422901 \|\| LOC730394 \|\| General transcription factor IIH, polypeptide 2, 44kDa-like (1) | Hs.422901 | 0.82 | 3.52 | 728340 | NM_00109872 8 |
| Hs.176503 \|\| TBC1D1 \|\| TBC1 (tre-2/USP6, BUB2, cdc 16) domain family, member 1 (1) | Hs.176503 | 0.82 | 0.98 | 23216 | NM_015173 |
| Hs.527874 \|\| CCDC131 \|\| Coiled-coil domain containing 131 (1) | Hs.527874 | 0.82 | 2.61 | 196441 | NM_144982 |
| Hs.480597 \|\| USP33 \|\| Ubiquitin specific peptidase 33 (1) | Hs.480597 | 0.82 | 1.75 | 23032 | NM_015017 |
| Hs.485915 \|\| C60rf166 \|\| Chromosome 6 open reading frame 166 (2) | Hs.485915 | 0.82 | 1.67 | 55122 | CR599380 |
| Hs.287362 \|\| TLE3 \|\| Transducin-like enhancer of split 3 (E(sp1) homolog, Drosophila) (4) | Hs.287362 | 0.82 | 3.52 | | BM473090 |
| Hs.202023 \|\| RBM22 \|\| RNA binding motif protein 22(1) | Hs.202023 | 0.82 | 1.43 | | D20052 |
| Hs.655183 \|\| \|\| Full-length cDNA clone CS0DM003YK11 of Fetal liver of Homo sapiens (human) (1) | Hs.655183 | 0.82 | 2.02 | | |
| Hs.422986 \|\| ANXA4 \|\| Annexin A4 (1) | Hs.422986 | 0.82 | 1.67 | 307 | BX641114 |
| Hs.199763 \|\| SSH1 \|\| Slingshot homolog 1 (Drosophila) (1) | Hs.199763 | 0.82 | 1.67 | 54434 | AB037719 |
| Hs.130759 \|\| PLSCR1 \|\| Phospholipid scramblase 1 (1) | Hs.130759 | 0.82 | 4.44 | 5359 | NM-021105 |
| Hs.522147 \|\| ZNF658 \|\| Zinc finger protein 658 (1) | Hs.522147 | 0.82 | 0.89 | 26149 | NM_033160 |
| Hs.167535 \|\| SRP54 \|\| Signal recognition particle 54kDa (1) | Hs.167535 | 0.82 | 2.61 | 6729 | BC000652 |
| Hs.405046 \|\| CBX4 \|\| Chromobox homolog 4 (Pc class homolog, Drosophila) (1) | Hs.405046 | 0.82 | 2.61 | | BI334851 |
| Hs.516617 \|\| SATB2 \|\| SATB homeobox 2 (1) | Hs.516617 | 0.82 | 1.24 | 23314 | AB209376 |
| Hs.592711 \|\| \|\| Transcribed locus (1) | Hs.592711 | 0.82 | 1.24 | | |
| Hs.654978 \|\| RAB27A \|\| RAB27A, member RAS oncogene family (6) | Hs.654978 | 0.82 | 2.98 | 5873 | NM_004580 |
| Hs.399719 \|\| FLJ22536 \|\| Hypothetical locus LOC401237 (1) | Hs.399719 | 0.82 | 2.15 | 401237 | AK094718 |
| Hs.465818 \|\| MYO1F \|\| Myosin IF (1) | Hs.465818 | 0.82 | 4.01 | 4542 | AB290178 |
| Hs.500897 \|\| Cl0orf26 \|\| Chromosome 10 open reading frame 26 (1) | Hs.500897 | 0.82 | 1.67 | 54838 | NM_017787 |
| Hs.483036 \|\| PJA2 \|\| Praja 2, RING-H2 motif containing (1) | Hs.483036 | 0.82 | 2.98 | 9867 | NM_014819 |
| Hs.533013 \|\| CBS \|\| Cystathionine-beta-synthase (2) | Hs.533013 | 0.82 | 1.67 | 875 | L00972 |
| Hs.212332 \|\| CAV2 \|\| Caveolin 2 (2) | Hs.212332 | 0.82 | 1.75 | 858 | NM_001233 |
| Hs.620701 \|\| KIAA0701 \|\| KIAA0701 protein (3) | Hs.620701 | 0.82 | 2.15 | 23074 | NM_015054 |
| Hs.514373 \|\| MTMR4 \|\| Myotubularin related protein 4 (2) | Hs.514373 | 0.82 | 2.15 | 9110 | NM_004687 |
| Hs.656902 \|\| ARFGEF1 \|\| ADP-ribosylation factor guanine nucleotide-exchange factor 1(brefeldin A-inhibited) (2) | Hs.656902 | 0.82 | 4.44 | 10565 | NM_006421 |
| \|\| \|\| \|\| \|\| AI968640 \|\| \|\| \|\| \|\| 331174 | 2514804 | 0.82 | 2.61 | | AI968640 |
| Hs.370854 \|\| TSC1 \|\| Tuberous sclerosis 1 (1) | Hs.370854 | 0.82 | 4.44 | 7248 | NM_000368 |
| Hs.567381 \|\| GYG2 \|\| Glycogenin 2 (1) | Hs.567381 | 0.82 | 4.01 | 8908 | NM_003918 |
| Hs.276252 \|\| ATRN \|\| Attractin (2) | Hs.276252 | 0.82 | 2.15 | 8455 | NM_139321 |
| Hs.558440 \|\| tcag7.1310 \|\| KIAA0415 protein (1) | Hs.558440 | 0.82 | 3.52 | 9907 | AB007875 |
| Hs.80132 \|\| SNX15 \|\| Sorting nexin 15 (1) | Hs.80132 | 0.82 | 4.44 | 29907 | AB209439 |
| \|\| \|\| \|\| \|\| H81309 \|\| \|\| \|\| \|\| 102293 | 239580 | 0.82 | 2.98 | | CA776574 |
| Hs.473632 \|\| SYNJ1 \|\| Synaptojanin 1 (1) | Hs.473632 | 0.82 | 4.01 | 8867 | NM_203446 |
| Hs.458657 \|\| NEFM \|\| Neurofilament, medium polypeptide 150kDa (1) | Hs.458657 | 0.82 | 0.89 | 4741 | BC096757 |
| Hs.506748 \|\| HDGF \|\| Hepatoma-derived growth factor (high-mobility group protein 1-like) (2) | Hs.506748 | 0.82 | 4.01 | 3068 | NM_004494 |
| Hs.369356 \|\| \|\| CDNA clone 5302680 (1) | Hs.369356 | 0.82 | 0.98 | | AK123595 |
| Hs.656805 \|\| ALPK2 \|\| Alpha-kinase 2 (2) | Hs.656805 | 0.82 | 1.75 | 115701 | BX647796 |
| Hs.651212 \|\| YWHAB \|\| Tyrosine 3-monooxygenase/tryptophan 5-monooxygenase activation protein, beta polypeptide (1) | Hs.651212 | 0.82 | 3.52 | 7529 | AB209759 |
| Hs.300887 \|\| AHCTF1 \|\| AT hook containing transcription factor 1 (2) | Hs.300887 | 0.82 | 2.61 | 25909 | NM_015446 |
| Hs.594695 \|\| \|\| Transcribed locus (1) | Hs.594695 | 0.82 | 4.88 | | BX397522 |
| Hs.155040 \|\| ZNF217 \|\| Zinc finger protein 217 (4) | Hs.155040 | 0.82 | 4.44 | 7764 | NM_006526 |
| Hs.167679 \|\| SH3BP2 \|\| SH3-domain binding protein 2 (2) | Hs.167679 | 0.82 | 2.15 | 6452 | NM_003023 |
| Hs.532411 \|\| LYST \|\| Lysosomal trafficking regulator (1) | Hs.532411 | 0.82 | 1.75 | 1130 | NM_000081 |
| Hs.585357 \|\| HBZ \|\| Hemoglobin, zeta (1) | Hs.585357 | 0.82 | 1.67 | 3050 | BI833939 |
| Hs.509499 \|\| C14orf135 \|\| Chromosome 14 open reading frame 135 (1) | Hs.509499 | 0.82 | 4.44 | 64430 | NM_022495 |
| Hs.406096 \|\| ZFAND5 \|\| Zinc finger, AN1-type domain 5 (1) | Hs.406096 | 0.82 | 2.61 | 7763 | NM_00110242 0 |
| Hs.661635 \|\| \|\| **Transcribed locus, strongly similar to XP_001108973.1 similar to zinc finger protein 258 isoform 1 [Macaca mulatta] (1) | Hs.661635 | 0.82 | 4.44 | | |
| Hs.516866 \|\| SLC23A2 \|\| Solute carrier family 23 (nucleobase transporters), member 2 (1) | Hs.516866 | 0.82 | 4.44 | 9962 | NM_203327 |
| Hs.489615 \|\| PBEF1 \|\| Pre-B-cell colony enhancing factor 1 (2) | Hs.489615 | 0.83 | 4.44 | 10135 | NM_005746 |
| \|\| \|\| \|\| \|\| AI214272 \|\| \|\| \|\| \|\| 317600 | 1942166 | 0.83 | 1.67 | | AI214272 |
| Hs.632256 \|\| STATSB \|\| Signal transducer and activator of transcription 5B (3) | Hs.632256 | 0.83 | 2.98 | | BM918015 |
| Hs.179309 \|\| UBQLN2 \|\| Ubiquilin 2 (2) | Hs.179309 | 0.83 | 4.88 | 29978 | AF189009 |
| Hs.628957 \|\| hCG_1757335 \|\| HCG1757335 (1) | Hs.628957 | 0.83 | 3.52 | 643752 | NM_00108970 4 |
| Hs.597551 \|\| \|\| Transcribed locus (1) | Hs.597551 | 0.83 | 1.43 | | DB280383 |
| Hs.239818 \|\| PIK3CB \|\| Phosphoinositide-3-kinase, catalytic, beta polypeptide (2) | Hs.239818 | 0.83 | 2.61 | 5291 | CR749357 |
| Hs.507080 \|\| APCS \|\| Amyloid P component, serum (1) | Hs.507080 | 0.83 | 2.15 | 325 | BG533315 |
| Hs.492280 \|\| ATP7B \|\| ATPase, Cu++ transporting, beta polypeptide (1) | Hs.492280 | 0.83 | 1.67 | 540 | NM_000053 |
| \|\| \|\| \|\| \|\| AA909984 \|\| \|\| \|\| \|\| 310776 | 1523144 | 0.83 | 1.67 | | AA909984 |
| Hs.635133 \|\| \|\| Transcribed locus, strongly similar to XP_001105453.1 hypothetical protein [Macaca mulatta] (1) | Hs.635133 | 0.83 | 0.98 | | AI208158 |
| Hs.480938 \|\| LRBA \|\| **LPS-responsive vesicle trafficking, beach and anchor containing (1) | Hs.480938 | 0.83 | 4.44 | 987 | NM_006726 |
| Hs.593620 \|\| SPAG9 \|\| Sperm associated antigen 9 (1) | Hs.593620 | 0.83 | 3.52 | 9043 | AK129652 |
| Hs.236030 \|\| SMARCC2 \|\| SWI/SNF related, matrix associated, actin dependent regulator of chromatin, subfamily c, member 2 (2) | Hs.236030 | 0.83 | 2.98 | 6601 | AB209006 |
| Hs.656252 \|\| \|\| \|\| CDNA FLJ31688 fis, clone NT2RI2005520 (1) | Hs.656252 | 0.83 | 2.61 | | AK056250 |
| Hs.445129 \|\| C12orf35 \|\| Chromosome 12 open reading frame 35 (1) | Hs.445129 | 0.83 | 4.44 | 55196 | NM_018169 |
| Hs.1466 \|\| GK \|\| Glycerol kinase (1) | Hs.1466 | 0.83 | 2.61 | 2710 | BC042421 |
| Hs.81848 \|\| RAD21 \|\| RAD21 homolog (S. pombe) (1) | Hs.81848 | 0.83 | 3.52 | 5885 | NM_006265 |
| Hs.124246 \|\| C10orf119 \|\| Chromosome 10 open reading frame 119 (1) | Hs.124246 | 0.83 | 4.01 | 79892 | NM_024834 |
| \|\| \|\| \|\| \|\| H40351 \|\| \|\| \|\| \|\| 103056 | 191868 | 0.83 | 0.98 | | H40351 |
| Hs.180711 \|\| STX3 \|\| Syntaxin 3 (1) | Hs.180711 | 0.83 | 4.01 | 6809 | BX538158 |
| Hs.642906 \|\| \|\| Transcribed locus (1) | Hs.642906 | 0.83 | 2.98 | | |
| \|\| \|\| \|\| \|\| AA677043 \|\| \|\| \|\| \|\| 225504 | 454592 | 0.83 | 3.52 | | AA677043 |
| Hs.104519 \|\| PLD2 \|\| Phospholipase D2 (1) | Hs.104519 | 0.83 | 1.43 | 5338 | AB209374 |
| Hs.97174 \|\| DKFZP566E164 \|\| DKFZP566E164 protein (1) | Hs.97174 | 0.83 | 0.89 | 25858 | BM563999 |
| Hs.122926 \|\| NR3C1 \|\| Nuclear receptor subfamily 3, group C, member 1 (glucocorticoid receptor) (2) | Hs.122926 | 0.83 | 2.02 | 2908 | NM_00101807 7 |
| \|\| \|\| \|\| \|\| N75718 \|\| \|\| \|\| \|\| 101239 | 244305 | 0.83 | 2.15 | | BX093179 |
| Hs.33446 \|\| NR5A2 \|\| Nuclear receptor subfamily 5, group A, member 2 (1) | Hs.33446 | 0.83 | 2.98 | 2494 | NM_205860 |
| Hs.298023 \|\| AQP5 \|\| Aquaporin 5 (1) | Hs.298023 | 0.83 | 2.98 | 362 | NM_001651 |
| Hs.408708 \|\| SBN02 \|\| Strawberry notch homolog 2 (Drosophila) (3) | Hs.408708 | 0.83 | 2.61 | 22904 | NM_014963 |
| Hs.58612 \|\| \|\| Transcribed locus (1) | Hs.58612 | 0.83 | 2.98 | | BM542225 |
| Hs.207459 \|\| ST6GAL1 \|\| ST6 beta-galactosamide alpha-2,6-sialyltranferase 1 (1) | Hs.207459 | 0.83 | 2.98 | 6480 | AK128726 |
| Hs.593413 \|\| CXCR4 \|\| Chemokine (C-X-C motif) receptor 4 (1) | Hs.593413 | 0.83 | 2.98 | 7852 | AF147204 |
| Hs.498661 \|\| USP6NL \|\| USP6 N-terminal like (1) | Hs.498661 | 0.83 | 1.75 | 9712 | D 13644 |
| Hs.168073 \|\| TRPC4AP \|\| Transient receptor potential cation channel, subfamily C, member 4 associated protein (1) | Hs.168073 | 0.83 | 2.98 | 26133 | AL096738 |
| Hs.481927 \|\| NIPBL \|\| Nipped-B homolog (Drosophila) (2) | Hs.481927 | 0.83 | 4.44 | 25836 | AJ627032 |
| Hs.14611 \|\| DUSP11 \|\| Dual specificity phosphatase 11 (RNA/RNP complex 1-interacting) (1) | Hs.14611 | 0.83 | 1.43 | 8446 | CR627368 |
| Hs.666293 \|\| \|\| Transcribed locus (1) | Hs.666293 | 0.83 | 2.98 | | AA935315 |
| Hs.603099 \|\| \|\| Transcribed locus (1) | Hs.603099 | 0.83 | 0.98 | | AI088001 |
| \|\| \|\| \|\| \|\| AA701655 \|\| \|\| \|\| \|\| 104350 | 433573 | 0.83 | 4.88 | | AA701655 |
| Hs.135646 \|\| ELF1 \|\| E74-like factor 1 (ets domain transcription factor) (2) | Hs.135646 | 0.83 | 4.44 | 1997 | BC010575 |
| Hs.44693 \|\| MKLN1 \|\| Muskelin 1, intracellular mediator containing kelch motifs (3) | Hs.44693 | 0.83 | 2.61 | | BX648653 |
| Hs.382176 \|\| MTRF1 \|\| Mitochondrial translational release factor 1 (1) | Hs.382176 | 0.83 | 2.61 | 9617 | BC038427 |
| Hs.177633 \|\| ZBTB34 \|\| Zinc finger and BTB domain containing 34 (1) | Hs.177633 | 0.83 | 2.61 | 403341 | NM_00109927 0 |
| \|\| \|\| \|\| \|\| AA706818 \|\| \|\| \|\| \|\| 221196 | 451804 | 0.83 | 4.01 | | AA706818 |
| Hs.193043 \|\| CLCN6 \|\| Chloride channel 6 (1) | Hs.193043 | 0.83 | 2.02 | 1185 | NM_001286 |
| Hs.463059 \|\| STAT3 \|\| Signal transducer and activator of transcription 3 (acute-phase response factor) (3) | Hs.463059 | 0.83 | 1.75 | 6774 | NM_139276 |
| Hs.211602 \|\| SMCLA \|\| Structural maintenance of chromosomes 1A (1) | Hs.211602 | 0.83 | 3.52 | 8243 | NM_006306 |
| Hs.591633 \|\| TRIP12 \|\| Thyroid hormone receptor interactor 12 (2) | Hs.591633 | 0.83 | 1.24 | 9320 | NM_004238 |
| Hs.515478 \|\| LOC339344 \|\| Hypothetical protein LOC339344 (1) | Hs.515478 | 0.83 | 2.15 | 339344 | BC044311 |
| Hs.476280 \|\| ZYG11B \|\| Zyg-11 homolog B (C. elegans) (1) | Hs.476280 | 0.83 | 2.61 | 79699 | NM_024646 |
| Hs.29189 \|\| ATP11A \|\| ATPase, Class VI, type 11A (1) | Hs.29189 | 0.83 | 4.44 | 23250 | NM_015205 |
| Hs.517262 \|\| SON \|\| SON DNA binding protein (1) | Hs.517262 | 0.83 | 4.44 | 6651 | NM_138927 |
| Hs.492333 \|\| STK3 \|\| Serine/threonine kinase 3 (STE20 homolog, yeast) (2) | Hs.492333 | 0.83 | 2.98 | 6788 | CR627416 |
| Hs.288487 \|\| SMS \|\| Spermine synthase (1) | Hs.288487 | 0.83 | 4.88 | | BX356206 |
| Hs.201034 \|\| NTN4 \|\| Netrin 4 (1) | Hs.201034 | 0.83 | 4.01 | 59277 | NM_021229 |
| Hs.598011 \|\| \|\| Transcribed locus (1) | Hs.598011 | 0.83 | 4.88 | | DB315367 |
| Hs.432755 \|\| SNAG1 \|\| Sorting nexin associated golgi protein 1 (2) | Hs.432755 | 0.83 | 4.01 | 112574 | NM_00110257 5 |
| Hs.594481 \|\| SERPINB2 \|\| Serpin peptidase inhibitor, clade B (ovalbumin), member 2 (1) | Hs.594481 | 0.83 | 4.44 | 5055 | BC012609 |
| Hs.588289 \|\| MAPK14 \|\| Mitogen-activated protein kinase 14 (1) | Hs.588289 | 0.83 | 4.01 | 1432 | NM_001315 |
| Hs.4014 \|\| ZDHHC17 \|\| Zinc finger, DHHC-type containing 17 (1) | Hs.4014 | 0.83 | 4.01 | 23390 | AB024494 |
| Hs.198072 \|\| PDE4B \|\| Phosphodiesterase 4B, cAMP-specific (phosphodiesterase E4 dunce homolog, Drosophila) (2) | Hs.198072 | 0.83 | 4.88 | 5142 | NM_00103734 1 |
| Hs.267659 \|\| VAV3 \|\| Vav 3 oncogene (1) | Hs.267659 | 0.83 | 1.67 | 10451 | NM_006113 |
| Hs.591866 \|\| SPAG1 \|\| Sperm associated antigen 1 (1) | Hs.591866 | 0.83 | 1.75 | 6674 | NM_003114 |
| Hs.632864 \|\| \|\| CDNA clone 4801326 (1) | Hs.632864 | 0.83 | 1.43 | | CD245238 |
| \|\| \|\| \|\| \|\| AA489670 \|\| \|\| \|\| \|\| 222734 | 824329 | 0.83 | 1.75 | | AA489670 |
| Hs.653674 \|\| \|\| Transcribed locus (1) | Hs.653674 | 0.83 | 3.52 | | |
| Hs.600583 \|\| \|\| Transcribed locus (1) | Hs.600583 | 0.83 | 4.01 | | AA609891 |
| Hs.536158 \|\| PARG \|\| Poly (ADP-ribose) glycohydrolase (1) | Hs.536158 | 0.83 | 2.15 | | AK098044 |
| Hs.301696 \|\| KIAA1539 \|\| KIAA1539 (2) | Hs.301696 | 0.83 | 2.15 | 80256 | NM_025182 |
| Hs.372208 \|\| HSPC159 \|\| Galectin-related protein (1) | Hs.372208 | 0.83 | 2.98 | 29094 | N_014181 |
| Hs.275775 \|\| SEPP1 \|\| Selenoprotein P, plasma, 1 (1) | Hs.275775 | 0.83 | 3.52 | 6414 | BC030009 |
| Hs.594338 \|\| C14orf32 \|\| Chromosome 14 open reading frame 32 (4) | Hs.594338 | 0.83 | 4.01 | 93487 | NM_144578 |
| Hs.72071 \|\| KCTD9 \|\| Potassium channel tetramerisation domain containing 9 (1) | Hs.72071 | 0.83 | 3.52 | 54793 | AL117436 |
| Hs.291030 \|\| QGAP2 \|\| IQ motif containing GTPase activating protein 2 (1) | Hs.291030 | 0.83 | 3.52 | 10788 | NM_006633 |
| Hs.591600 \|\| ROCK2 \|\| Rho-associated, coiled-coil containing protein kinase 2 (2) | Hs.591600 | 0.83 | 0.98 | 9475 | AB014519 |
| Hs.552608 \|\| C1orf58 \|\| Chromosome 1 open reading frame 58 (1) | Hs.552608 | 0.83 | 2.98 | 148362 | AK094916 |
| Hs.248153 \|\| CAPN5 \|\| Calpain 5 (1) | Hs.248153 | 0.83 | 2.61 | 726 | NM_004055 |
| Hs.235935 \|\| NOV \|\| Nephroblastoma overexpressed gene (2) | Hs.235935 | 0.83 | 3.52 | 4856 | NM_002514 |
| Hs.631841 \|\| PALM \|\| Paralemmin (1) | Hs.631841 | 0.83 | 4.01 | 5064 | NM_002579 |
| Hs.280388 \|\| NPHP1 \|\| Nephronophthisis 1 (juvenile) (1) | Hs.280388 | 0.83 | 2.02 | 4867 | BX571745 |
| Hs.654691 \|\| THRAP2 \|\| Thyroid hormone receptor associated protein 2 (4) | Hs.654691 | 0.83 | 2.98 | 23389 | NM_015335 |
| Hs.15929 \|\| C6orf211 \|\| Chromosome 6 open reading frame 211 (1) | Hs.15929 | 0.83 | 2.98 | 79624 | AK022972 |
| Hs.208414 \|\| SLC25A40 \|\| Solute carrier family 25, member 40 (2) | Hs.208414 | 0.83 | 2.98 | 55972 | AL110179 |
| Hs.435774 \|\| ZNF33A \|\| Zinc finger protein 33A (1) | Hs.435774 | 0.83 | 1.67 | 7581 | NM_006954 |
| Hs.401843 \|\| STRN3 \|\| Striatin, calmodulin binding protein 3 (1) | Hs.401843 | 0.83 | 4.01 | 29966 | NM_00108389 3 |
| \|\| \|\| \|\| \|\| AA829784 \|\| \|\| \|\| \|\| 102767 | 1415128 | 0.83 | 4.01 | | AA829784 |
| Hs.657789 \|\| \|\| Transcribed locus (1) | Hs.657789 | 0.83 | 1.67 | | |
| Hs.463074 \|\| ATP6VOA1 \|\| ATPase, H+ transporting, lysosomal V0 subunit a1 (1) | Hs.463074 | 0.83 | 2.15 | 535 | AK125927 |
| Hs.351593 \|\| FGA \|\| Fibrinogen alpha chain (1) | Hs.351593 | 0.83 | 1.43 | 2243 | M58569 |
| \|\| \|\| \|\| \|\| R87752 \|\| \|\| \|\| \|\| 307653 | 197631 | 0.83 | 2.02 | | R87752 |
| Hs.656261 \|\| \|\| CDNA FLJ43417 fis, clone OCBBF2026025 (1) | Hs.656261 | 0.83 | 1.24 | 6546 | AK125406 |
| Hs.471119 \|\| BMPR2 \|\| Bone morphogenetic protein receptor, type II (serine/threonine kinase) (1) | Hs.471119 | 0.83 | 1.67 | 659 | NM_001204 |
| Hs.497183 \|\| IVNS1ABP \|\| Influenza virus NS1A binding protein (2) | Hs.497183 | 0.83 | 4.88 | 10625 | NM_006469 |
| \|\| \|\| \|\| \|\| R65610 \|\| \|\| \|\| \|\| 117560 | 140334 | 0.83 | 2.98 | | BX110630 |
| Hs.46446 \|\| LYL1 \|\| Lymphoblastic leukemia derived sequence 1 (1) | Hs.46446 | 0.84 | 4.44 | 4066 | BM924781 |
| Hs.559459 \|\| C6orf32 \|\| Chromosome 6 open reading frame 32 (1) | Hs.559459 | 0.84 | 1.75 | 9750 | NM_014722 |
| Hs.532345 \|\| ZFYVE9 \|\| Zinc finger, FYVE domain containing 9 (1) | Hs.532345 | 0.84 | 4.01 | 9372 | NM_004799 |
| Hs.604001 \|\| HNRPH1 \|\| Heterogeneous nuclear ribonucleoprotein H1 (H) (3) | Hs.604001 | 0.84 | 4.44 | 3187 | BX647205 |
| Hs.479677 \|\| SLAIN2 \|\| SLAIN motif family, member 2 (2) | Hs.479677 | 0.84 | 4.44 | 57606 | NM_020846 |
| Hs.178499 \|\| ZBTB44 \|\| Zinc finger and BTB domain containing 44 (1) | Hs.178499 | 0.84 | 2.98 | 29068 | NM_014155 |
| Hs.658241 \|\| LOC145474 \|\| Hypothetical protein LOC145474 (1) | Hs.658241 | 0.84 | 2.02 | 145474 | AK023718 |
| Hs.657624 \|\| \|\| **Transcribed locus, weakly similar to XP_001110634.1 similar to ELAV-like 4 isoform 5 [Macaca mulatta] (1) | Hs.657624 | 0.84 | 4.88 | | BM922881 |
| Hs.269782 \|\| GNAQ \|\| Guanine nucleotide binding protein (G protein), q polypeptide (2) | Hs.269782 | 0.84 | 3.52 | 2776 | BC057777 |
| Hs.497512 \|\| NUAK2 \|\| NUAK family, SNF1-like kinase, 2 (1) | Hs.497512 | 0.84 | 2.02 | 81788 | CR749209 |
| Hs.469175 \|\| SYTL1 \|\| Synaptotagmin-like 1 (1) | Hs.469175 | 0.84 | 4.44 | 84958 | AK074154 |
| Hs.434447 \|\| \|\| Transcribed locus, weakly similar to XP_001118564.1 similar to ankyrin repeat domain 20 family, member A2, partial [Macaca mulatta] (1) | Hs.434447 | 0.84 | 0.89 | 100132785 | XM_001720214 |
| Hs.591873 \|\| IL7 \|\| Interleukin 7 (1) | Hs.591873 | 0.84 | 0.72 | 3574 | BC047698 |
| Hs.435044 \|\| TBC1D22A \|\| TBC1 domain family, member 22A (2) | Hs.435044 | 0.84 | 1.43 | 25771 | AK125705 |
| Hs.485205 \|\| TEAD3 \|\| TEA domain family member 3 (2) | Hs.485205 | 0.84 | 4.44 | 7005 | AK226144 |
| Hs.2533 \|\| ALDH9A1 \|\| Aldehyde dehydrogenase 9 family, member A1 (1) | Hs.2533 | 0.84 | 4.88 | 223 | U34252 |
| Hs.21016 \|\| COCH \|\| Coagulation factor C homolog, cochlin (Limulus polyphemus) (2) | Hs.21016 | 0.84 | 2.61 | 1690 | AK123362 |
| Hs.533719 \|\| ARHGEF17 \|\| Rho guanine nucleotide exchange factor (GEF) 17 (1) | Hs.533719 | 0.84 | 2.02 | 9828 | NM_014786 |
| Hs.20716 \|\| TIMM17A \|\| Translocase of inner mitochondrial membrane 17 homolog A (yeast) (1) | Hs.20716 | 0.84 | 2.98 | 10440 | AK023063 |
| Hs.8366 \|\| OIT3 \|\| Oncoprotein induced transcript 3 (1) | Hs.8366 | 0.84 | 3.52 | 170392 | AK096435 |
| Hs.514950 \|\| SCPEP1 \|\| Serine carboxypeptidase 1 (1) | Hs.514950 | 0.84 | 2.02 | 59342 | BC010078 |
| Hs.655824 \|\| PHTF1 \|\| Putative homeodomain transcription factor 1 (2) | Hs.655824 | 0.84 | 1.75 | 10745 | AK126773 |
| Hs.664020 \|\| \|\| Transcribed locus (1) | Hs.664020 | 0.84 | 1.75 | | BU960438 |
| Hs.9731 \|\| NFKBIB \|\| Nuclear factor of kappa light polypeptide gene enhancer in B-cells inhibitor, beta (1) | Hs.9731 | 0.84 | 2.98 | 4793 | NM_00100171 6 |
| Hs.202470 \|\| UVRAG \|\| UV radiation resistance associated gene (1) | Hs.202470 | 0.84 | 2.98 | | BF514762 |
| Hs.567769 \|\| SAMD14 \|\| Sterile alpha motif domain containing 14 (1) | Hs.567769 | 0.84 | 4.88 | 201191 | AK094209 |
| Hs.356270 \|\| SDHD \|\| Succinate dehydrogenase complex, subunit D, integral membrane protein (2) | Hs.356270 | 0.84 | 4.01 | 6392 | BF240727 |
| Hs.351665 \|\| TRDMTI \|\| TRNA aspartic acid methyltransferase 1 (1) | Hs.351665 | 0.84 | 4.44 | 1787 | BX537961 |
| Hs.521456 \|\| TNFRSF10B \|\| **Tumor necrosis factor receptor superfamily, member 10b (1) | Hs.521456 | 0.84 | 1.43 | 8795 | NM_003842 |
| Hs.55024 \|\| XKR8 \|\| XK, Kell blood group complex subunit-related family, member 8 (1) | Hs.55024 | 0.84 | 2.98 | 55113 | AK054909 |
| Hs.372123 \|\| NDELI \|\| NudE nuclear distribution gene E homolog (A. nidulans)-like 1 (2) | Hs.372123 | 0.84 | 4.01 | 81565 | AL832648 |
| Hs.657477 \|\| LOC646214 \|\| **Similar to p21-activated kinase 2 (1) | Hs.657477 | 0.84 | 2.98 | 646214 | XR_016124 |
| Hs.478868 \|\| KIAA0226 \|\| KIAA0226 (5) | Hs.478868 | 0.84 | 3.52 | 9711 | NM_014687 |
| Hs.644289 \|\| \|\| Transcribed locus (1) | Hs.644289 | 0.84 | 4.88 | | |
| Hs.685590 \|\| \|\| **Transcribed locus (1) | Hs.685590 | 0.84 | 2.98 | | BG286834 |
| Hs.666843 \|\| \|\| Transcribed locus (1) | Hs.666843 | 0.84 | 2.61 | | BF589268 |
| Hs.658974 \|\| \|\| Transcribed locus (1) | Hs.658974 | 0.84 | 4.44 | | CD722288 |
| Hs.647072 \|\| PRKAG2 \|\| Protein kinase, AMP-activated, gamma 2 non-catalytic subunit (1) | Hs.647072 | 0.84 | 4.01 | 51422 | CR933725 |
| \|\| \|\| \|\| \|\| H56029 \|\| \|\| \|\| \|\| 119345 | 203554 | 0.84 | 1.67 | | H56029 |
| Hs.540696 \|\| SLC6A8 \|\| Solute carrier family 6 (neurotransmitter transporter, creatine), member 8 (1) | Hs.540696 | 0.84 | 3.52 | 6535 | L31409 |
| Hs.591852 \|\| ADAM9 \|\| ADAM metallopeptidase domain 9 (meltrin gamma) (1) | Hs.591852 | 0.84 | 4.44 | 8754 | NM_003816 |
| Hs.644000 \|\| CLINT1 \|\| Clathrin interactor 1 (1) | Hs.644000 | 0.84 | 4.01 | | DB304879 |
| Hs.483811 \|\| CTF1 \|\| Cardiotrophin 1 (1) | Hs.483811 | 0.84 | 4.44 | 1489 | BC036787 |
| Hs.140944 \|\| FAM50B \|\| Family with sequence similarity 50, member B (1) | Hs.140944 | 0.84 | 4.88 | 26240 | BC001261 |
| Hs. 113919 \|\| CCDC23 \|\| Coiled-coil domain containing 23 (1) | Hs.113919 | 0.84 | 2.98 | 374969 | CD243680 |
| Hs.557646 \|\| CDK9 \|\| Cyclin-dependent kinase 9 (CDC2-related kinase) (2) | Hs.557646 | 0.84 | 4.88 | 1025 | NM_001261 |
| Hs.529272 \|\| MARCH7 \|\| Membrane-associated ring finger (C3HC4) 7 (2) | Hs.529272 | 0.84 | 2.61 | 64844 | AK124225 |
| Hs.652394 \|\| SFRS9 \|\| Splicing factor, arginine/serine-rich 9 (1) | Hs.652394 | 0.84 | 4.88 | 8683 | BG107695 |
| Hs.75199 \|\| PPP2R5B \|\| Protein phosphatase 2, regulatory subunit B', beta isoform (1) | Hs.75199 | 0.84 | 4.44 | 5526 | AB209289 |
| Hs.173524 \|\| KIAA0430 \|\| KIAA0430 (1) | Hs.173524 | 0.84 | 4.44 | 9665 | NM_014647 |
| \|\| \|\| \|\| \|\| AA776319 \|\| \|\| \|\| \|\| 223628 | 453717 | 0.84 | 4.44 | | CA407087 |
| Hs.643566 \|\| MAPKAPK2 \|\| Mitogen-activated protein kinase-activated protein kinase 2 (1) | Hs.643566 | 0.84 | 2.98 | 9261 | NM_004759 |
| Hs.661062 \|\| KRT5 \|\| Keratin 5 (epidermolysis bullosa simplex, Dowling-Meara/Kobner/Weber-Cockayne types) (1) | Hs.661062 | 0.84 | 2.98 | 3852 | M21389 |
| \|\| \|\| \|\| \|\| R26404 \|\| \|\| \|\| \|\| 113725 | 132248 | 0.84 | 2.98 | | R26404 |
| Hs.517249 \|\| NCSTN \|\| Nicastrin (1) | Hs.517249 | 0.84 | 2.02 | 23385 | BC047621 |
| Hs.515487 \|\| CALM3 \|\| Calmodulin 3 (phosphorylase kinase, delta) (2) | Hs.515487 | 0.84 | 3.52 | 808 | AK094964 |
| Hs.17529 \|\| \|\| Clone 120631 mRNA sequence (1) | Hs.17529 | 0.84 | 1.75 | | AF143879 |
| Hs.356604 \|\| WNK1 \|\| WNK lysine deficient protein kinase 1 (3) | Hs.356604 | 0.84 | 2.02 | 65125 | NM_018979 |
| Hs.291319 \|\| \|\| MRNA; cDNA DKFZp779M2422 (from clone DKFZp779M2422) (1) | Hs.291319 | 0.84 | 4.01 | | CR627122 |
| Hs.389906 \|\| \|\| Transcribed locus (1) | Hs.389906 | 0.84 | 4.44 | | BM547530 |
| Hs.54457 \|\| CD81 \|\| CD81 molecule (1) | Hs.54457 | 0.84 | 2.61 | 975 | AB209380 |
| Hs.280695 \|\| KIAA0157 \|\| KIAA0157 (1) | Hs.280695 | 0.84 | 3.52 | 23172 | NM_032182 |
| Hs.466436 \|\| GPATCH1 \|\| G patch domain containing 1 (1) | Hs.466436 | 0.84 | 1.67 | 55094 | NM_018025 |
| Hs.536967 \|\| \|\| Transcribed locus (1) | Hs.536967 | 0.84 | 1.75 | 654466 | AY098594 |
| Hs.475103 \|\| NUP50 \|\| Nucleoporin 50kDa (2) | Hs.475103 | 0.84 | 4.01 | 10762 | NM_007172 |
| Hs.659069 \|\| \|\| Transcribed locus (1) | Hs.659069 | 0.84 | 4.01 | | N28399 |
| Hs.435680 \|\| EPYC \|\| Epiphycan (1) | Hs.435680 | 0.84 | 4.44 | 1833 | NM_004950 |
| Hs.475538 \|\| XPC \|\| Xeroderma pigmentosum, complementation group C (1) | Hs.475538 | 0.84 | 4.01 | 7508 | NM_004628 |
| Hs.643801 \|\| VCAN \|\| Versican (5) | Hs.643801 | 0.84 | 4.44 | | CD359929 |
| Hs.372938 \|\| NRXN2 \|\| Neurexin 2 (1) | Hs.372938 | 0.84 | 4.44 | 9379 | NM_015080 |
| Hs.481186 \|\| CLCN3 \|\| Chloride channel 3 (1) | Hs.481186 | 0.84 | 4.88 | 1182 | NM_173872 |
| Hs.334534 \|\| GNS \|\| Glucosamine (N-acetyl)-6-sulfatase (Sanfilippo disease IIID) (2) | Hs.334534 | 0.84 | 4.44 | 2799 | BX537363 |
| \|\| \|\| \|\| \|\| AI675615 \|\| \|\| \|\| \|\| 313611 | 2314036 | 0.84 | 4.44 | | AI675615 |
| Hs.75850 \|\| WASF1 \|\| WAS protein family, member 1 (1) | Hs.75850 | 0.84 | 1.43 | 8936 | NM_003931 |
| Hs.83636 \|\| ADRBK1 \|\| Adrenergic, beta, receptor kinase 1 (1) | Hs.83636 | 0.84 | 2.61 | 156 | NM_001619 |
| Hs.654369 \|\| CDC2L2 \|\| **Cell division cycle 2-like 2 (PITSLRE proteins) (1) | Hs.654369 | 0.84 | 2.98 | 728642 | AB209095 |
| Hs.516978 \|\| C20orf12 \|\| Chromosome 20 open reading frame 112 (1) | Hs.516978 | 0.84 | 4.88 | 140688 | AL122043 |
| Hs.167451 \|\| KIAA0999 \|\| KIAA0999 protein (1) | Hs.167451 | 0.84 | 2.15 | 23387 | AL832068 |
| Hs.654521 \|\| WIPF1 \|\| WAS/WASL interacting protein family, member 1 (3) | Hs.654521 | 0.84 | 1.75 | 7456 | NM_00107726 9 |
| Hs.105547 \|\| NPDC1 \|\| Neural proliferation, differentiation and control, 1 (1) | Hs.105547 | 0.84 | 3.52 | 56654 | AK054950 |
| Hs.19413 \|\| S100A12 \|\| 5100 calcium binding protein A12 (1) | Hs.19413 | 0.84 | 3.52 | 6283 | AV758762 |
| Hs.64016 \|\| PROS1 \|\| Protein S (alpha) (1) | Hs.64016 | 0.84 | 4.88 | 5627 | M14338 |
| \|\| \|\| \|\| \|\| AA233643 \|\| \|\| \|\| \|\| 115149 | 666172 | 0.84 | 4.44 | | AA233643 |
| Hs.268785 \|\| ACSL4 \|\| Acyl-CoA synthetase long-chain family member 4 (1) | Hs.268785 | 0.84 | 4.44 | 2182 | NM_022977 |
| Hs.37288 \|\| NR1D2 \|\| Nuclear receptor subfamily 1, group D, member 2 (1) | Hs.37288 | 0.84 | 3.52 | 9975 | AB209091 |
| Hs.596712 \|\| \|\| Transcribed locus, strongly similar to XP_526942.1 similar to protein kinase, AMP-activated, alpha 1 catalytic subunit isoform 2; AMP-activated protein kinase, catalytic, alpha-1; 5-AMP-activated prot (1) | Hs.596712 | 0.84 | 4.44 | | BQ933774 |
| Hs.82927 \|\| AMPD2 \|\| Adenosine monophosphate deaminase 2 (isoform L) (2) | Hs.82927 | 0.84 | 4.88 | 271 | NM_004037 |
| Hs.512592 \|\| RRM2B \|\| Ribonucleotide reductase M2 B (TP53 inducible) (1) | Hs.512592 | 0.84 | 4.44 | 50484 | NM_015713 |
| \|\| \|\| \|\| \|\| N62132 \|\| \|\| \|\| \|\| 111608 | 287598 | 0.84 | 2.61 | | N62132 |
| Hs.664675 \|\| PAR5 \|\| Prader-Willi/Angelman syndrome-5 (1) | Hs.664675 | 0.84 | 4.88 | 8123 | AF019618 |
| Hs.652402 \|\| CYB5R3 \|\| Cytochrome b5 reductase 3 (1) | Hs.652402 | 0.84 | 4.88 | 1727 | NM_007326 |
| Hs.608404 \|\| \|\| Transcribed locus (2) | Hs.608404 | 0.85 | 4.44 | | BQ421037 |
| Hs.438851 \|\| \|\| Transcribed locus (1) | Hs.438851 | 0.85 | 4.44 | | AA974411 |
| Hs.657607 \|\| \|\| CDNA clone 5265193 (1) | Hs.657607 | 0.85 | 4.01 | | AK123540 |
| \|\| \|\| \|\| \|\| AA677923 \|\| \|\| \|\| \|\| 221558 | 431007 | 0.85 | 4.01 | | AA677923 |
| Hs.559765 \|\| \|\| Transcribed locus (1) | Hs.559765 | 0.85 | 4.44 | | BQ926066 |
| Hs.650059 \|\| RNF44 \|\| Ring finger protein 44 (1) | Hs.650059 | 0.85 | 4.44 | 22838 | BC063297 |
| \|\| \|\| \|\| \|\| A004626 \|\| \|\| \|\| \|\| 115749 | 428267 | 0.85 | 2.61 | | BX091431 |
| Hs.437156 \|\| GGTLA1 \|\| Gamma-glutamyltransferase-like activity 1 (1) | Hs.437156 | 0.85 | 4.01 | 2687 | NM-00109978 1 |
| Hs.413812 \|\| RAC1 \|\| Ras-related C3 botulinum toxin substrate 1 (rho family, small GTP binding protein Rac1) (2) | Hs.413812 | 0.85 | 4.01 | 5879 | AB209410 |
| Hs.666465 \|\| ABHD2 \|\| Abhydrolase domain containing 2 (2) | Hs.666465 | 0.85 | 4.44 | | NM_007011 |
| Hs.611075 \|\| \|\| Transcribed locus (1) | Hs.611075 | 0.85 | 2.02 | | BQ672983 |
| Hs.523252 \|\| KIAA1754 \|\| KIAA1754 (1) | Hs.523252 | 0.85 | 3.52 | 85450 | NM_033397 |
| \|\| \|\| \|\| \|\| AI265991 \|\| \|\| \|\| \|\| 311733 | 1891788 | 0.85 | 1.67 | | AI265991 |
| Hs.643495 \|\| HMGCR \|\| 3-hydroxy-3-methylglutaryl-Coenzyme A reductase (1) | Hs.643495 | 0.85 | 4.44 | | CN278665 |
| Hs.514474 \|\| KIAA0195 \|\| KIAA0195 (1) | Hs.514474 | 0.85 | 4.44 | 9772 | NM_014738 |
| Hs.406460 \|\| CCDC117 \|\| Coiled-coil domain containing 117 (1) | Hs.406460 | 0.85 | 4.44 | 150275 | NM_173510 |
| Hs.117938 \|\| COL17A1 \|\| Collagen, type XVII, alpha 1 (2) | Hs.117938 | 0.85 | 2.61 | 1308 | NM_000494 |
| Hs.173135 \|\| DYRK2 \|\| Dual-specificity tyrosine-(Y)-phosphorylation regulated kinase 2 (1) | Hs.173135 | 0.85 | 4.88 | 8445 | NM_006482 |
| Hs.191540 \|\| VPS13B \|\| Vacuolar protein sorting 13 homolog B (yeast) (1) | Hs.191540 | 0.85 | 2.98 | 157680 | NM_017890 |
| Hs.471675 \|\| DGKD \|\| Diacylglycerol kinase, delta 130kDa (1) | Hs.471675 | 0.85 | 4.44 | 8527 | NM_152879 |
| Hs.524809 \|\| CLIP1 \|\| CAP-GLY domain containing linker protein 1 (1) | Hs.524809 | 0.85 | 4.44 | 6249 | NM_002956 |
| Hs.590937 \|\| DDX52 \|\| DEAD (Asp-Glu-Ala-Asp) box polypeptide 52 (1) | Hs.590937 | 0.85 | 4.01 | 11056 | NM_152300 |
| Hs.490874 \|\| MTX1 \|\| Metaxin 1 (1) | Hs.490874 | 0.85 | 2.61 | 4580 | NM_002455 |
| Hs.441498 \|\| STAM \|\| Signal transducing adaptor molecule (SH3 domain and ITAM motif) 1 (3) | Hs.441498 | 0.85 | 3.52 | 8027 | NM_003473 |
| Hs.644108 \|\| GPC3 \|\| Glypican 3 (1) | Hs.644108 | 0.85 | 4.88 | | BG208757 |
| Hs.319171 \|\| NFKBIZ \|\| Nuclear factor of kappa light polypeptide gene enhancer in B-cells inhibitor, zeta (3) | Hs.319171 | 0.85 | 4.44 | 64332 | NM_031419 |
| Hs.540038 \|\| CTAGE5 \|\| CTAGE family, member 5 (1) | Hs.540038 | 0.85 | 2.98 | 4253 | AF338234 |
| Hs.508010 \|\| FNDC3A \|\| Fibronectin type III domain containing 3A (1) | Hs.508010 | 0.85 | 4.88 | 22862 | NM_00107967 3 |
| Hs.227777 \|\| PTP4A1 \|\| Protein tyrosine phosphatase type IVA, member 1 (2) | Hs.227777 | 0.85 | 4.88 | 7803 | NM_003463 |
| Hs.54642 \|\| MAT2B \|\| Methionine adenosyltransferase II, beta (3) | Hs.54642 | 0.85 | 4.88 | 27430 | AB073390 |
| Hs.309849 \|\| C14orf159 \|\| Chromosome 14 open reading frame 159 (1) | Hs.309849 | 0.85 | 4.01 | 80017 | BX647492 |
| Hs.339024 \|\| MSRB3 \|\| Methionine sulfoxide reductase B3 (1) | Hs.339024 | 0.85 | 4.01 | 253827 | NM_00103167 9 |
| Hs.516539 \|\| HNRPA3 \|\| Heterogeneous nuclear ribonucleoprotein A3 (4) | Hs.516539 | 0.85 | 4.88 | 220988 | NM_194247 |
| Hs.480042 \|\| ANXA3 \|\| Annexin A3 (1) | Hs.480042 | 0.85 | 4.88 | 306 | AB209868 |
| Hs.631725 \|\| CSNK1D \|\| Casein kinase 1, delta (2) | Hs.631725 | 0.85 | 4.01 | 1453 | AB209463 |
| Hs.266826 \|\| ARMC8 \|\| Armadillo repeat containing 8 (2) | Hs.266826 | 0.85 | 3.52 | 25852 | AL096748 |
| Hs.390567 \|\| FYN \|\| FYN oncogene related to SRC, FGR, YES (3) | Hs.390567 | 0.85 | 2.98 | 2534 | BX537571 |
| Hs.593672 \|\| \|\| Transcribed locus (1) | Hs.593672 | 0.85 | 3.52 | | BX106436 |
| Hs.308 \|\| ARR3 \|\| Arrestin 3, retinal (X-arrestin) (1) | Hs.308 | 0.85 | 4.44 | 407 | NM_004312 |
| Hs.300774 \|\| FGB \|\| Fibrinogen beta chain (1) | Hs.300774 | 0.85 | 4.44 | 2244 | NM_005141 |
| Hs.522605 \|\| MID1IP1 \|\| MID1 interacting protein 1 (gastrulation specific G12 homolog (zebrafish)) (1) | Hs.522605 | 0.85 | 4.88 | 58526 | NM_021242 |
| Hs.141883 \|\| \|\| Transcribed locus (1) | Hs.141883 | 0.85 | 2.61 | | BX091613 |
| Hs.656506 \|\| C14orf43 \|\| Chromosome 14 open reading frame 43 (1) | Hs.656506 | 0.85 | 4.01 | 91748 | NM_194278 |
| Hs.28456 \|\| \|\| Transcribed locus (1) | Hs.28456 | 0.85 | 4.01 | | BX107795 |
| Hs.648744 \|\| KIAA1267 \|\| KIAA1267 (1) | Hs.648744 | 0.85 | 1.67 | 284058 | BX538006 |
| Hs.494321 \|\| AGTPBP1 \|\| ATP/GTP binding protein 1 (3) | Hs.494321 | 0.85 | 4.88 | 23287 | NM_015239 |
| Hs.118651 \|\| HHEX \|\| Hematopoietically expressed homeobox (1) | Hs.118651 | 0.85 | 4.88 | 3087 | BC015110 |
| Hs.434996 \|\| GIT2 \|\| G protein-coupled receptor kinase interactor 2 (2) | Hs.434996 | 0.85 | 3.52 | 9815 | NM_057169 |
| Hs.534847 \|\| C4B \|\| Complement component 4B (Childo blood group) (1) | Hs.534847 | 0.85 | 4.88 | 720 | BC151204 |
| Hs.75335 \|\| GATM \|\| Glycine amidinotransferase (L-arginine:glycine amidinotransferase) (1) | Hs.75335 | 0.85 | 4.01 | 2628 | NM_001482 |
| Hs.352614 \|\| AGPAT7 \|\| 1-acylglycerol-3-phosphate O-acyltransferase 7 (lysophosphatidic acid acyltransferase, eta) (2) | Hs.352614 | 0.85 | 3.52 | 254531 | AF542964 |
| Hs.369052 \|\| SELT \|\| Selenoprotein T (2) | Hs.369052 | 0.85 | 4.01 | 51714 | NM_016275 |
| Hs.389103 \|\| GLP1R \|\| Glucagon-like peptide 1 receptor (1) | Hs.389103 | 0.85 | 1.67 | 2740 | NM_002062 |
| \|\| \|\| \|\| \|\| AA452578 \|\| \|\| \|\| \|\| 106523 | 788519 | 0.85 | 2.15 | | BC029441 |
| Hs.595451 \|\| ZNF609 \|\| Zinc finger protein 609 (1) | Hs.595451 | 0.85 | 4.44 | 23060 | BC029441 |
| Hs.445036 \|\| FLJ20309 \|\| Hypothetical protein FLJ20309 (2) | Hs.445036 | 0.85 | 4.88 | 54891 | NM_017759 |
| Hs.632120 \|\| S-LTV420H1 \|\| Suppressor of variegation 4-20 homolog 1 (Drosophila) (1) | Hs.632120 | 0.85 | 4.01 | 51111 | NM_017635 |
| Hs.664823 \|\| \|\| Transcribed locus (1) | Hs.664823 | 0.85 | 4.88 | | DB099181 |
| Hs.98117 \|\| C13orf18 \|\| Chromosome 13 open reading frame 18 (1) | Hs.98117 | 0.85 | 4.44 | 80183 | NM_025113 |
| Hs.44024 \|\| MRPL19 \|\| Mitochondrial ribosomal protein L19 (3) | Hs.44024 | 0.85 | 4.44 | 9801 | NM_014763 |
| Hs.349024 \|\| \|\| Transcribed locus (1) | Hs.349024 | 0.85 | 2.98 | | BX105464 |
| Hs.98475 \|\| PNKD \|\| Paroxysmal nonkinesiogenic dyskinesia (1) | Hs.98475 | 0.85 | 3.52 | 25953 | NM_015488 |
| Hs.648467 \|\| TncRNA \|\| Trophoblast-derived noncoding RNA (2) | Hs.648467 | 0.85 | 3.52 | 283131 | EF177379 |
| Hs.631768 \|\| JARIDLC \|\| Jumonji, AT rich interactive domain 1C (1) | Hs.631768 | 0.85 | 4.01 | 8242 | NM_004187 |
| \|\| \|\| \|\| \|\| AA629838 \|\| \|\| \|\| \|\| 119582 | 884790 | 0.85 | 1.75 | | AA629838 |
| Hs.23119 \|\| TMEM187 \|\| Transmembrane protein 187 (1) | Hs.23119 | 0.85 | 3.52 | 8269 | NM_003492 |
| Hs.644847 \|\| \|\| Transcribed locus, strongly similar to XP_001117375.1 similar to M-phase phosphoprotein, mpp8 [Macaca mulatta] (1) | Hs.644847 | 0.85 | 3.52 | | BU933052 |
| Hs.461896 \|\| \|\| CDNA FLJ38130 fis, clone D6OST2000464 (1) | Hs.461896 | 0.85 | 4.88 | | AK095449 |
| Hs.497626 \|\| PLXNA2 \|\| Plexin A2 (1) | Hs.497626 | 0.85 | 4.01 | 5362 | NM_025179 |
| Hs.539684 \|\| EIF2S3 \|\| Eukaryotic translation initiation factor 2, subunit 3 gamma, 52kDa (4) | Hs.539684 | 0.85 | 4.88 | 1968 | NM_001415 |
| Hs.59214 \|\| LOC144871 \|\| Hypothetical protein LOC144871 (1) | Hs.59214 | 0.85 | 4.44 | | AF339816 |
| Hs.594095 \|\| CMIP \|\| C-Maf-inducing protein (1) | Hs.594095 | 0.85 | 4.88 | | BI755146 |
| Hs.535276 \|\| PTPN22 \|\| Protein tyrosine phosphatase, non-receptor type 22 (lymphoid) (1) | Hs.535276 | 0.85 | 4.88 | 26191 | NM_015967 |
| Hs.513071 \|\| MESDC1 \|\| Mesoderm development candidate 1 (1) | Hs.513071 | 0.85 | 4.88 | 59274 | AY007810 |
| Hs.369606 \|\| CPSF6 \|\| Cleavage and polyadenylation specific factor 6, 68kDa (1) | Hs.369606 | 0.85 | 4.88 | 11052 | NM_007007 |
| Hs.660038 \|\| MGC23985 \|\| Similar to AVLV472 (1) | Hs.660038 | 0.86 | 3.52 | 389336 | CD556191 |
| Hs.694057 \|\| \|\| Transcribed locus (1) | Hs.694057 | 0.86 | 1.43 | | |
| Hs.652268 \|\| hCG_1646491 \|\| Brother of ataxin-1 (2) | Hs.652268 | 0.86 | 4.01 | 342371 | BX537575 |
| Hs.220950 \|\| FOX03 \|\| Forkhead box 03 (3) | Hs.220950 | 0.86 | 4.01 | 2309 | NM_001455 |
| Hs. 163936 \|\| \|\| Transcribed locus (1) | Hs.163936 | 0.86 | 2.61 | | AW813731 |
| Hs.631814 \|\| LARP5 \|\| La ribonucleoprotein domain family, member 5 (2) | Hs.631814 | 0.86 | 4.01 | | BX482823 |
| Hs.440899 \|\| TTYH3 \|\| Tweety homolog 3 (Drosophila) (1) | Hs.440899 | 0.86 | 4.88 | 80727 | BC152447 |
| Hs.270753 \|\| TMEM130 \|\| Transmembrane protein 130 (1) | Hs.270753 | 0.86 | 2.61 | 222865 | AK124634 |
| \|\| \|\| \|\| \|\| AA521416 \|\| \|\| \|\| \|\| 222884 | 826103 | 0.86 | 2.61 | | AA521416 |
| Hs. 522418 \|\| GLE \|\| GLE1 RNA export mediator-like (yeast) (1) | Hs.522418 | 0.86 | 2.98 | 2733 | NM_00100372 2 |
| Hs.282417 \|\| PYGL \|\| Phosphorylase, glycogen; liver (Hers disease, glycogen storage disease type VI) (1) | Hs.282417 | 0.86 | 4.44 | 5836 | BC110791 |
| Hs.529400 \|\| IFNAR1 \|\| Interferon (alpha, beta and omega) receptor 1 (3) | Hs.529400 | 0.86 | 4.01 | 3454 | NM_000629 |
| Hs.598660 \|\| \|\| Transcribed locus (1) | Hs.598660 | 0.86 | 4.44 | | BX110728 |
| Hs.594238 \|\| KPNA2 \|\| Karyopherin alpha 2 (RAG cohort 1, importin alpha 1) (2) | Hs.594238 | 0.86 | 4.88 | 3838 | BC067848 |
| Hs.587979 \|\| PTOV1 \|\| Prostate tumor overexpressed gene 1 (2) | Hs.587979 | 0.86 | 2.61 | 53635 | NM_017432 |
| Hs.580351 \|\| PRKCE \|\| Protein kinase C, epsilon (1) | Hs.580351 | 0.86 | 4.88 | 5581 | NM_005400 |
| Hs.662670 \|\| \|\| Transcribed locus (1) | Hs.662670 | 0.86 | 4.88 | | |
| Hs.413801 \|\| PSME4 \|\| Proteasome (prosome, macropain) activator subunit 4 (3) | Hs.413801 | 0.86 | 4.44 | 23198 | NM_014614 |
| Hs.50550 \|\| KBTBD10 \|\| Kelch repeat and BTB (POZ) domain containing 10 (1) | Hs.50550 | 0.86 | 3.52 | 10324 | NM_006063 |
| Hs.595299 \|\| PTPRJ \|\| Protein tyrosine phosphatase, receptor type, J (1) | Hs.595299 | 0.86 | 4.01 | 5795 | NM_002843 |
| Hs.644065 \|\| TSC22D2 \|\| TSC22 domain family, member 2 (3) | Hs.644065 | 0.86 | 4.01 | | BQ024475 |
| Hs.658986 \|\| \|\| Transcribed locus (1) | Hs.658986 | 0.86 | 2.98 | | BQ934312 |
| Hs.567327 \|\| PEX5 \|\| Peroxisomal biogenesis factor 5 (2) | Hs.567327 | 0.86 | 4.88 | 5830 | NM_000319 |
| Hs.594250 \|\| \|\| **Transcribed locus (1) | Hs.594250 | 0.86 | 2.61 | | BG249238 |
| Hs. 128605 Transcribed locus (1) | Hs.128605 | 0.86 | 1.75 | | BX092410 |
| Hs.455109 \|\| B4GALT7 \|\| Xylosylprotein beta 1,4-galactosyltransferase, polypeptide 7 (galactosyltransferase I) (1) | Hs.455109 | 0.86 | 2.61 | 11285 | AK022566 |
| Hs.213666 \|\| KIAA0460 \|\| KIAA0460 (1) | Hs.213666 | 0.86 | 2.61 | 23248 | NM_015203 |
| Hs.437191 \|\| PTRF \|\| Polymerase I and transcript release factor (1) | Hs.437191 | 0.86 | 2.98 | 284119 | BC066123 |
| Hs.654668 \|\| ARHGAP26 \|\| Rho GTPase activating protein 26 (1) | Hs.654668 | 0.86 | 4.44 | 23092 | NM_015071 |
| \|\| \|\| \|\| \|\| W88420 \|\| \|\| \|\| \|\| 113733 | 417294 | 0.86 | 2.98 | | W88420 |
| Hs.102471 \|\| \|\| Transcribed locus (1) | Hs.102471 | 0.86 | 4.44 | 9749 | NM_00110016 4 |
| Hs.496459 \|\| TOR1AIP1 \|\| Torsin A interacting protein 1 (2) | Hs.496459 | 0.86 | 4.44 | 26092 | NM_015602 |
| Hs.476663 \|\| \|\| Transcribed locus (1) | Hs.476663 | 0.86 | 4.44 | | BX095616 |
| Hs.162757 \|\| LRP1 \|\| Low density lipoprotein-related protein 1 (alpha-2-macroglobulin receptor) (1) | Hs.162757 | 0.86 | 4.88 | 4035 | NM_002332 |
| Hs.588682 \|\| SUMF1 \|\| Sulfatase modifying factor 1 (1) | Hs.588682 | 0.86 | 4.88 | 285362 | AY358092 |
| Hs.465744 \|\| INSR \|\| Insulin receptor (1) | Hs.465744 | 0.86 | 2.98 | 3643 | NM_000208 |
| Hs.182231 \|\| TRH \|\| Thyrotropin-releasing hormone (1) | Hs.182231 | 0.86 | 1.67 | 7200 | NM_007117 |
| Hs.482520 \|\| BRDT \|\| Bromodomain, testis-specific (1) | Hs.482520 | 0.86 | 3.52 | 676 | BX648135 |
| Hs.424184 \|\| LOC285074 \|\| Hypothetical protein LOC285074 (1) | Hs.424184 | 0.86 | 4.01 | 285074 | AK057663 |
| \|\| \|\| \|\| \|\| R06745 \|\| \|\| \|\| \|\| 100277 | 126509 | 0.86 | 4.88 | | BF679939 |
| Hs.250429 \|\| SUPT6H \|\| Suppressor of Ty 6 homolog (S. cerevisiae) (2) | Hs.250429 | 0.86 | 4.44 | 6830 | BC150268 |
| Hs.493804 \|\| KIAA0258 \|\| KIAA0258 (1) | Hs.493804 | 0.86 | 4.44 | 9827 | D87447 |
| Hs.441113 \|\|MAGEA6 \|\| Melanoma antigen family A, 6 (1) | Hs.441113 | 0.86 | 4.44 | 4105 | BX640600 |
| \|\| \|\| \|\| \|\| R92199 \|\| \|\| \|\| \|\| 118503 | 195841 | 0.86 | 1.43 | | R92199 |
| Hs.416630 \|\| SUDS3 \|\| Suppressor of defective silencing 3 homolog (S. cerevisiae) (1) | Hs.416630 | 0.86 | 4.01 | 64426 | NM_022491 |
| Hs.516948 \|\| PRCC \|\| Papillary renal cell carcinoma (translocation-associated) (1) | Hs.516948 | 0.86 | 4.01 | 5546 | NM_005973 |
| \|\| \|\| \|\| \|\| W37107 \|\| \|\| \|\| \|\| 112918 | 321994 | 0.86 | 4.01 | | W37107 |
| Hs.657753 \|\| EBF1 \|\| Early B-cell factor 1 (1) | Hs.657753 | 0.86 | 2.02 | 1879 | NM_024007 |
| Hs.599839 \|\| \|\| Transcribed locus (1) | Hs.599839 | 0.87 | 2.98 | | AA460584 |
| Hs.501239 Transcribed locus (1) | Hs.501239 | 0.87 | 2.98 | | BM926391 |
| Hs.93534 \|\| STXBPS \|\| Syntaxin binding protein 5 (tomosyn) (1) | Hs.93534 | 0.87 | 4.88 | 134957 | BC043645 |
| Hs.6360 \|\| TMCC2 \|\| Transmembrane and coiled-coil domain family 2 (1) | Hs.6360 | 0.87 | 2.98 | 9911 | AY358192 |
| Hs.602394 \|\| \|\| Transcribed locus (1) | Hs.602394 | 0.87 | 4.88 | | AA863116 |
| Hs.658190 \|\| PDK3 \|\| Pyruvate dehydrogenase kinase, isozyme 3 (2) | Hs.658190 | 0.87 | 4.01 | 5165 | CR606226 |
| Hs.591024 \|\| NEUROD4 \|\| Neurogenic differentiation 4 (1) | Hs.591024 | 0.87 | 4.88 | 58158 | NM_021191 |
| Hs.35101 \|\| PRRG2 \|\| Proline rich Gla (G-carboxyglutamic acid) 2 (1) | Hs.35101 | 0.87 | 1.67 | 5639 | BC026032 |
| Hs.6396 \|\| JTB \|\| Jumping translocation breakpoint (1) | Hs.6396 | 0.87 | 4.01 | 10899 | BQ062593 |
| Hs.21454 \|\|TDRD9 \|\| Tudor domain containing 9 (1) | Hs.21454 | 0.87 | 4.44 | 122402 | NM_153046 |
| \|\| \|\| \|\| \|\| AI025177 \|\| \|\| \|\| \|\| 311066 | 1639441 | 0.87 | 3.52 | | AI025177 |
| Hs.115277 \|\| UTY \|\| Ubiquitously transcribed tetratricopeptide repeat gene, Y-linked (1) | Hs.115277 | 0.87 | 2.98 | 7404 | NM_007125 |
| Hs.667982 \|\| LOC653483 \|\| Similar to myeloid/lymphoid or mixed-lineage leukemia (trithorax homolog, Drosophila); translocated to, 4 (1) | Hs.667982 | 0.87 | 4.88 | | |
| Hs.530930 \|\| ZNF423 \|\| Zinc finger protein 423 (1) | Hs.530930 | 0.87 | 3.52 | 23090 | NM_015069 |
| Hs.693665 \|\| GCS1 \|\| Glucosidase I (1) | Hs.693665 | 0.87 | 4.44 | 7841 | NM_006302 |
| Hs.632738 \|\| U1SNRNPBP \|\| U11/U12 snRNP 35K (1) | Hs.632738 | 0.87 | 3.52 | 11066 | NM_180699 |
| Hs.437126 \|\| ARHGAP9 \|\| Rho GTPase activating protein 9 (1) | Hs.437126 | 0.87 | 4.44 | 64333 | BC006107 |
| Hs.374477 \|\| EWSR1 \|\| Ewing sarcoma breakpoint region 1 (3) | Hs.374477 | 0.87 | 4.44 | 2130 | BX648769 |
| Hs.537218 \|\| SEMG2 \|\| Semenogelin II (1) | Hs.537218 | 0.87 | 1.43 | 6407 | BC070306 |
| Hs.49105 \|\| GLMN \|\| Glomulin, FKBP associated protein (1) | Hs.49105 | 0.87 | 4.88 | 11146 | AK130581 |
| Hs.516966 \|\| BCL2L1 \|\| BCL2-like 1 (1) | Hs.516966 | 0.87 | 4.44 | 598 | CR936637 |
| Hs.597303 \|\| \|\| Full length insert cDNA clone ZA04F06 (1) | Hs.597303 | 0.87 | 4.01 | | AK023472 |
| Hs.657659 \|\| EIF2C3 \|\| Eukaryotic translation initiation factor 2C, 3 (1) | Hs.657659 | 0.87 | 2.61 | 192669 | NM_024852 |
| Hs.434948 \|\| \|\| Transcribed locus (1) | Hs.434948 | 0.87 | 4.88 | | CA423842 |
| Hs.120855 \|\| THSD7A \|\| Thrombospondin, type I, domain containing 7A (1) | Hs.120855 | 0.87 | 4.44 | 221981 | NM_015204 |
| Hs.75317 \|\| SLC16A2 \|\| Solute carrier family 16, member 2 (monocarboxylic acid transporter 8) (1) | Hs.75317 | 0.87 | 4.44 | 6567 | NM_006517 |
| Hs.491805 \|\| TOX \|\| Thymocyte selection-associated high mobility group box (2) | Hs.491805 | 0.87 | 2.61 | 9760 | BX647462 |
| Hs.602110 \|\| \|\| Transcribed locus (1) | Hs.602110 | 0.88 | 2.15 | | AA887085 |
| \|\| \|\| \|\| \|\| AI025943 \|\| \|\| \|\| \|\| 307387 | 1646544 | 0.88 | 3.52 | | AI025943 |
| Hs.468280 \|\| \|\| Transcribed locus, moderately similar to XP_001110462.1 hypothetical protein [Macaca mulatta] (1) | Hs.468280 | 0.88 | 4.44 | | BU561227 |
| Hs.433060 \|\| ACPP \|\| Acid phosphatase, prostate (1) | Hs.433060 | 0.88 | 2.98 | 55 | NM_001099 |
| Hs.654611 \|\| CHN2 \|\| Chimerin (chimaerin) 2 (1) | Hs.654611 | 0.88 | 4.44 | 1124 | AK126784 |
| \|\| \|\| \|\| \|\| AA400676 \|\| \|\| \|\| \|\| 106758 | 753113 | 0.88 | 4.01 | | AA400676 |
| Hs.659558 \|\| APEX2 \|\| APEX nuclease (apurinic/apyrimidinic endonuclease) 2 (1) | Hs.659558 | 0.88 | 2.02 | 27301 | NM_014481 |
| Hs.591391 \|\| DEFA4 \|\| Defensin, alpha 4, corticostatin (1) | Hs.591391 | 0.88 | 1.24 | 1669 | BU616655 |
| Hs.593168 \|\| \|\| Homo sapiens, clone 5547644, mRNA (1) | Hs.593168 | 0.88 | 4.88 | | |
| Hs.659532 \|\| \|\| Transcribed locus (1) | Hs.659532 | 0.88 | 4.01 | | CR746211 |
| Hs.658370 \|\| \|\| Transcribed locus (1) | Hs.658370 | 0.88 | 4.44 | | BG539635 |
| Hs.164060 \|\| GRB10 \|\| Growth factor receptor-bound protein 10 (1) | Hs.164060 | 0.88 | 4.44 | 2887 | D86962 |
| Hs.657791 \|\| LOC400960 \|\| Hypothetical gene supported by BC040598 (1) | Hs.657791 | 0.88 | 2.98 | 400960 | AK056822 |
| Hs.236940 \|\| CEP76 \|\| Centrosomal protein 76kDa (2) | Hs.236940 | 0.88 | 2.98 | 79959 | AL833727 |
| Hs.435850 \|\| LYPLA1 \|\| Lysophospholipase I (1) | Hs.435850 | 0.88 | 4.44 | 10434 | NM_006330 |
| \|\| \|\| \|\| \|\| R35649 \|\| \|\| \|\| \|\| 107754 | 136998 | 0.88 | 4.44 | | R35649 |
| Hs.380240 \|\| \|\| Clone HLS_IMAGE_1646649 mRNA sequence (1) | Hs.380240 | 0.88 | 1.43 | 389741 | BC040170 |
| Hs.472566 \|\| HEYL \|\| Hairy/enhancer-of-split related with YRPW motif-like (1) | Hs.472566 | 0.89 | 4.01 | 26508 | NM_014571 |
| Hs.502182 \|\| BDNF \|\| Brain-derived neurotrophic factor (1) | Hs.502182 | 0.89 | 4.44 | 627 | EF689009 |
| Hs.97199 \|\| CD93 \|\| CD93 molecule (2) | Hs.97199 | 0.89 | 3.52 | 22918 | NM_012072 |
| Hs.661689 \|\| \|\| Transcript ch111 [human, RF1,RF48 stomach cancer cell lines, mRNA, 290 nt] (1) | Hs.661689 | 0.89 | 4.01 | | BU632348 |
| Hs.633844 \|\| GZF1 \|\| GDNF-inducible zinc finger protein 1 (2) | Hs.633844 | 0.89 | 4.88 | | CK430798 |
| Hs.651245 \|\| MAEL \|\| Maelstrom homolog (Drosophila) (1) | Hs.651245 | 0.89 | 4.01 | 84944 | BC034310 |
| Hs.485557 \|\| GSTA4 \|\| Glutathione S-transferase A4 (2) | Hs.485557 | 0.89 | 4.01 | 2941 | BC071631 |
| Hs.516114 \|\| WBPl \|\| WW domain binding protein 1 (1) | Hs.516114 | 0.89 | 4.88 | 23559 | BM811212 |
| Hs.63489 \|\| PTPN6 \|\| Protein tyrosine phosphatase, non-receptor type 6 (1) | Hs.63489 | 0.89 | 4.44 | 5777 | NM_080549 |
| Hs.657367 \|\| JPH1 \|\| Junctophilin 1 (1) | Hs.657367 | 0.89 | 4.88 | 56704 | NM_020647 |
| Hs.632804 \|\| SYP \|\| Synaptophysin (1) | Hs.632804 | 0.89 | 3.52 | 6855 | BC064550 |
| Hs.460184 \|\| MCM4 \|\| Minichromosome maintenance complex component 4 (3) | Hs.460184 | 0.89 | 4.88 | 4173 | NM_005914 |
| Hs.377416 \|\| ELM03 \|\| Engulfment and cell motility 3 (1) | Hs.377416 | 0.89 | 4.44 | 79767 | NM_024712 |
| Hs.2780 \|\| JUND \|\| Jun D proto-oncogene (2) | Hs.2780 | 0.89 | 4.88 | 3727 | NM_005354 |
| Hs.475150 \|\| DIP \|\| Death-inducing-protein (1) | Hs.475150 | 0.89 | 3.52 | 23151 | AB018310 |
| Hs.125533 \|\| Transcribed locus (1) | Hs.125533 | 0.89 | 4.01 | | BX105647 |
| Hs.123106 \|\| Clorf178 \|\| Chromosome 1 open reading frame 178 (1) | Hs.123106 | 0.89 | 4.01 | 440603 | NM_00101092 2 |
| Hs. 130639 \|\| \|\| Transcribed locus (1) | Hs.130639 | 0.90 | 3.52 | | BX109627 |
| Hs.23582 \|\| TACSTD2 \|\| Tumor-associated calcium signal transducer 2 (1) | Hs.23582 | 0.90 | 0.89 | 4070 | X77753 |
| Hs. 193115 \|\| LRRCC \|\| \|\| Leucine rich repeat and coiled-coil domain containing 1 (1) | Hs.193115 | 0.90 | 4.44 | 85444 | AB051551 |
| Hs.538411 \|\| \|\| Transcribed locus (1) | Hs.538411 | 0.90 | 4.44 | | BX091779 |
| Hs.108530 \|\| TMEM30A \|\| Transmembrane protein 30A (1) | Hs.108530 | 0.90 | 4.44 | 55754 | NM_018247 |
| Hs.198365 \|\| BPGM \|\| 2,3-bisphosphoglycerate mutase (2) | Hs.198365 | 0.90 | 4.88 | 669 | NM_199186 |
| Hs.323858 \|\| TRIM58 \| Tripartite motif-containing 58 (1) | Hs.323858 | 0.91 | 0.59 | 25893 | NM_015431 |
| Hs.560028 \|\| \|\| Transcribed locus (1) | Hs.560028 | 0.91 | 4.01 | | BQ188778 |
| Hs.595087 \|\| \|\| Transcribed locus (1) | Hs.595087 | 0.92 | 4.44 | | AI246418 |
| Hs.372152 \|\| DTX1 \|\| Deltex homolog 1 (Drosophila) (1) | Hs.372152 | 0.93 | 4.88 | 1840 | NM_004416 |
| Hs.592625 \|\| \|\| CDNA FLJ30478 fis, clone BRAWH1000167 (1) | Hs.592625 | 0.93 | 4.01 | | AK055040 |
| Hs.656435 \|\| \|\| Transcribed locus (1) | Hs.656435 | 0.93 | 4.88 | | BX091995 |
| Hs.47061 \|\| ULK1 \|\| Unc-51-like kinase 1 (C. elegans) (1) | Hs.47061 | 0.95 | 4.44 | 8408 | AF045458 |
| Hs.443948 \|\| SLC4A1 \|\| Solute carrier family 4, anion exchanger, member 1 (erythrocyte membrane protein band 3, Diego blood group) (1) | Hs.443948 | 1.08 | 4.88 | 6521 | NM_000342 |

### References Example 1

(1) Baechler EC, Batliwalla FM, Karypis G, Gaffney PM, Ortmann WA, Espe KJ et al. Interferon-inducible gene expression signature in peripheral blood cells of patients with severe lupus. Proc Natl Acad Sci U S A 2003; 100(5):2610-2615.
(2) Huang X, Yuang J, Goddard A, Foulis A, James RF, Lernmark A et al. Interferon expression in the pancreases of patients with type I diabetes. Diabetes 1995; 44(6):658-664.
(3) Greenberg SA, Pinkus JL, Pinkus GS, Burleson T, Sanoudou D, Tawil R et al. Interferon-alpha/beta-mediated innate immune mechanisms in dermatomyositis. Ann Neurol 2005; 57(5):664-678.
(4) Bave U, Nordmark G, Lovgren T, Ronnelid J, Cajander S, Eloranta ML et al. Activation of the type I interferon system in primary Sjogren's syndrome: a possible etiopathogenic mechanism. Arthritis Rheum 2005; 52 (4):1185-1195.
(5) van Baarsen LG, van der Pouw Kraan TC, Kragt JJ, Baggen JM, Rustenburg F, Hooper T et al. A subtype of multiple sclerosis defined by an activated immune defense program. Genes Immun 2006; 7(6):522-531.
(6) van der Pouw Kraan TC, Wijbrandts CA, van Baarsen LG, Voskuyl AE, Rustenburg F, Baggen JM et al. Rheumatoid arthritis subtypes identified by genomic profiling of peripheral blood cells: assignment of a type I interferon signature in a subpopulation of patients. Ann Rheum Dis 2007; 66(8):1008-1014.
(7) Jego G, Palucka AK, Blanck JP, Chalouni C, Pascual V, Banchereau J. Plasmacytoid dendritic cells induce plasma cell differentiation through type I interferon and interleukin 6. Immunity 2003; 19(2):225-234.
(8) Le BA, Schiavoni G, D'Agostino G, Gresser I, Belardelli F, Tough DF. Type i interferons potently enhance humoral immunity and can promote isotype switching by stimulating dendritic cells in vivo. Immunity 2001; 14(4):461-470.
(9) Nikpour M, Dempsey AA, Urowitz MB, Gladman DD, Barnes DA. Association of a gene expression profile from whole blood with disease activity in systemic lupus erythaematosus. Ann Rheum Dis 2008; 67(8):1069-1075.
(10) Braun D, Geraldes P, Demengeot J. Type I Interferon controls the onset and severity of autoimmune manifestations in lpr mice. J Autoimmun 2003; 20(1):15-25.
(11) Santiago-Raber ML, Baccala R, Haraldsson KM, Choubey D, Stewart TA, Kono DH et al. Type-I interferon receptor deficiency reduces lupus-like disease in NZB mice. J Exp Med 2003; 197(6):777-788.
(12) van HJ, Reedquist K, Sattonet-Roche P, Smeets TJ, Plater-Zyberk C, Vervoordeldonk MJ et al. Treatment with recombinant interferon-beta reduces inflammation and slows cartilage destruction in the collagen-induced arthritis model of rheumatoid arthritis. Arthritis Res Ther 2004; 6(3):R239-R249.
(13) van HJ, Pavelka K, Vencovsky J, Stahl H, Rozman B, Genovese M et al. A multicentre, randomised, double blind, placebo controlled phase II study of subcutaneous interferon beta-1a in the treatment of patients with active rheumatoid arthritis. Ann Rheum Dis 2005; 64(1):64-69.
(14) van der Pouw Kraan TC, van Baarsen LG, Wijbrandts CA, Voskuyl AE, Rustenburg F, Baggen JM et al. Expression of a pathogen-response program in peripheral blood cells defines a subgroup of rheumatoid arthritis patients. Genes Immun 2008; 9(1):16-22.
(15) Charles PJ, Smeenk RJ, De Jong J, Feldmann M, Maini RN. Assessment of antibodies to double-stranded DNA induced in rheumatoid arthritis patients following treatment with infliximab, a monoclonal antibody to tumor necrosis factor alpha: findings in open-label and randomized placebo-controlled trials. Arthritis Rheum 2000; 43(11):2383-2390.
(16) Maini RN, Elliott MJ, Charles PJ, Feldmann M. Immunological intervention reveals reciprocal roles for tumor necrosis factor-alpha and interleukin-10 in rheumatoid arthritis and systemic lupus erythematosus. Springer Semin Immunopathol 1994; 16(2-3):327-336.
(17) Banchereau J, Pascual V, Palucka AK. Autoimmunity through cytokine-induced dendritic cell activation. Immunity 2004; 20(5):539-550.
(18) Palucka AK, Blanck JP, Bennett L, Pascual V, Banchereau J. Cross-regulation of TNF and IFN-alpha in autoimmune diseases. Proc Natl Acad Sci U S A 2005; 102(9):3372-3377.
(19) Mavragani CP, Niewold TB, Moutsopoulos NM, Pillemer SR, Wahl SM, Crow MK. Augmented interferon-alpha pathway activation in patients with Sjogren's syndrome treated with etanercept. Arthritis Rheum 2007; 56(12):3995-4004.
(20) Jacobsen H, Mestan J, Mittnacht S, Dieffenbach CW. Beta interferon subtype 1 induction by tumor necrosis factor. Mol Cell Biol 1989; 9(7):3037-3042.
(21) Mestan J, Digel W, Mittnacht S, Hillen H, Blohm D, Moller A et al. Antiviral effects of recombinant tumour necrosis factor in vitro. Nature 1986; 323(6091):816-819.
(22) Yarilina A, Park-Min KH, Antoniv T, Hu X, Ivashkiv LB. TNF activates an IRF1-dependent autocrine loop leading to sustained expression of chemokines and STAT1-dependent type I interferon-response genes. Nat Immunol 2008; 9(4):378-387.
(23) van Baarsen LG, Vosslamber S, Tijssen M, Baggen JM, van d, V, Killestein J et al. Pharmacogenomics of interferon-beta therapy in multiple sclerosis: baseline IFN signature determines pharmacological differences between patients. PLoS ONE 2008; 3(4):e1927.
(24) van Gestel AM, Prevoo ML, van't Hof MA, van Rijswijk MH, van de Putte LB, van Riel PL. Development and validation of the European League Against Rheumatism response criteria for rheumatoid arthritis. Comparison with the preliminary American College of Rheumatology and the World Health Organization/International League Against Rheumatism Criteria. Arthritis Rheum 1996; 39(1):34-40.
(25) Fransen J, van Riel PL. The Disease Activity Score and the EULAR response criteria. Clin Exp Rheumatol 2005; 23(5 Suppl 39):593-S99.
(26) van Gestel AM, Haagsma CJ, van Riel PL. Validation of rheumatoid arthritis improvement criteria that include simplified joint counts. Arthritis Rheum 1998; 41(10):1845-1850.
(27) van der Pouw Kraan TC, Baarsen EGM, Rustenburg F, Baltus B, Fero M, Verweij CL. Gene expression profiling in rheumatology. In: Cope AP, editor. Arthritis Research. Methods and Protocols, Volume 2 ed. The Humana Press Inc.; 2007.
(28) Demeter J, Beauheim C, Gollub J, Hernandez-Boussard T, Jin H, Maier D et al. The Stanford Microarray Database: implementation of new analysis tools and open source release of software. Nucleic Acids Res 2007; 35(Database issue):D766-D770.
(29) van Baarsen LG, van der Pouw Kraan TC, Kragt JJ, Baggen JM, Rustenburg F, Hooper T et al. A subtype of multiple sclerosis defined by an activated immune defense program. Genes Immun 2006; 7(6):522-531.
(30) Sekiguchi N, Kawauchi S, Furuya T, Inaba N, Matsuda K, Ando S et al. Messenger ribonucleic acid expression profile in peripheral blood cells from RA patients following treatment with an anti-TNF-{alpha} monoclonal antibody, infliximab. Rheumatology (Oxford) 2008.
(31) Bachmann A, Hanke B, Zawatzky R, Soto U, van Riggelen J, zur HH et al. Disturbance of tumor necrosis factor alpha-mediated beta interferon signaling in cervical carcinoma cells. J Virol 2002; 76(1):280-291.
(32) Sigurdsson S, Nordmark G, Goring HH, Lindroos K, Wiman AC, Sturfelt G et al. Polymorphisms in the tyrosine kinase 2 and interferon regulatory factor 5 genes are associated with systemic lupus erythematosus. Am J Hum Genet 2005; 76(3):528-537.
(33) Sigurdsson S, Nordmark G, Garnier S, Grundberg E, Kwan T, Nilsson O et al. A common STAT4 risk haplotype for Systemic Lupus Erythematosus is over-expressed, correlates with anti-dsDNA production and shows additive effects with two IRF5 risk alleles. Hum Mol Genet 2008.
(34) Remmers EF, Plenge RM, Lee AT, Graham RR, Hom G, Behrens TW et al. STAT4 and the risk of rheumatoid arthritis and systemic lupus erythematosus. N Engl J Med 2007; 357(10):977-986.
(35) eguez-Gonzalez R, Calaza M, Perez-Pampin E, de la Serna AR, Fernandez-Gutierrez B, Castaneda S et al. Association of interferon regulatory factor 5 haplotypes, similar to that found in systemic lupus erythematosus, in a large subgroup of patients with rheumatoid arthritis. Arthritis Rheum 2008; 58(5):1264-1274.
(36) Sigurdsson S, Padyukov L, Kurreeman FA, Liljedahl U, Wiman AC, Alfredsson L et al. Association of a haplotype in the promoter region of the interferon regulatory factor 5 gene with rheumatoid arthritis. Arthritis Rheum 2007; 56(7):2202-2210.

### References Example 2

(1) Tracey D, Klareskog L, Sasso EH, Salfeld JG, Tak PP. Tumor necrosis factor antagonist mechanisms of action: a comprehensive review. Pharmacol Ther 2008; 117(2):244-279.
(2) Bazzoni F, Beutler B. The tumor necrosis factor ligand and receptor families. N Engl J Med 1996; 334(26):1717-1725.
(3) Feldmann M, Brennan FM, Maini RN. Rheumatoid arthritis. Cell 1996; 85(3):307-310.
(4) Elliott MJ, Maini RN, Feldmann M, Kalden JR, Antoni C, Smolen JS et al. Randomised double-blind comparison of chimeric monoclonal antibody to tumour necrosis factor alpha (cA2) versus placebo in rheumatoid arthritis. Lancet 1994; 344(8930):1105-1110.
(5) Feldmann M, Maini RN. Anti-TNF alpha therapy of rheumatoid arthritis: what have we learned? Annu Rev Immunol 2001; 19:163-196.
(6) Tak PP, Taylor PC, Breedveld FC, Smeets TJ, Daha MR, Kluin PM et al. Decrease in cellularity and expression of adhesion molecules by anti-tumor necrosis factor alpha monoclonal antibody treatment in patients with rheumatoid arthritis. Arthritis Rheum 1996; 39(7):1077-1081.
(7) Taylor PC, Peters AM, Paleolog E, Chapman PT, Elliott MJ, McCloskey R et al. Reduction of chemokine levels and leukocyte traffic to joints by tumor necrosis factor alpha blockade in patients with rheumatoid arthritis 1. Arthritis Rheum 2000; 43(1):38-47.
(8) Maini RN, Taylor PC, Paleolog E, Charles P, Ballara S, Brennan FM et al. Anti-tumour necrosis factor specific antibody (infliximab) treatment provides insights into the pathophysiology of rheumatoid arthritis. Ann Rheum Dis 1999; 58 Suppl 1:I56-I60.
(9) Polzer K, Baeten D, Soleiman A, Distler J, Gerlag DM, Tak PP et al. TNF blockade increases lymphangiogenesis in murine and human arthritic joints. Ann Rheum Dis 2008.
(10) Davis D, Charles PJ, Potter A, Feldmann M, Maini RN, Elliott MJ. Anaemia of chronic disease in rheumatoid arthritis: in vivo effects of tumour necrosis factor alpha blockade. Br J Rheumatol 1997; 36(9):950-956.
(11) Ignatowski TA, Covey WC, Knight PR, Severin CM, Nickola TJ, Spengler RN. Brain-derived TNFalpha mediates neuropathic pain. Brain Res 1999; 841(1-2):70-77.
(12) Tak PP, Smeets TJ, Daha MR, Kluin PM, Meijers KA, Brand R et al. Analysis of the synovial cell infiltrate in early rheumatoid synovial tissue in relation to local disease activity. Arthritis Rheum 1997; 40(2):217-225.
(13) Ulfgren AK, Grondal L, Lindblad S, Khademi M, Johnell O, Klareskog L et al. Interindividual and intra-articular variation of proinflammatory cytokines in patients with rheumatoid arthritis: potential implications for treatment. Ann Rheum Dis 2000; 59(6):439-447.
(14) Wijbrandts CA, Dijkgraaf MG, Kraan MC, Vinkenoog M, Smeets TJ, Dinant H et al. The clinical response to infliximab in rheumatoid arthritis is in part dependent on pre-treatment TNF{alpha} expression in the synovium. Ann Rheum Dis 2007.
(15) Marotte H, Maslinski W, Miossec P. Circulating tumour necrosis factor-alpha bioactivity in rheumatoid arthritis patients treated with infliximab: link to clinical response. Arthritis Res Ther 2005; 7(1):R149-R155.
(16) Pachot A, Arnaud B, Marrote H, Cazalis MA, Diasparra J, Gouraud A et al. Increased tumor necrosis factor-alpha mRNA expression in whole blood from patients with rheumatoid arthritis: reduction after infliximab treatment does not predict response. J Rheumatol 2007; 34(11):2158-2161.
(17) McInnes IB, Schett G. Cytokines in the pathogenesis of rheumatoid arthritis. Nat Rev Immunol 2007; 7(6):429-442.
(18) Kollias G, Douni E, Kassiotis G, Kontoyiannis D. On the role of tumor necrosis factor and receptors in models of multiorgan failure, rheumatoid arthritis, multiple sclerosis and inflammatory bowel disease. Immunol Rev 1999; 169:175-194.
(19) Grell M, Wajant H, Zimmermann G, Scheurich P. The type 1 receptor (CD120a) is the high-affinity receptor for soluble tumor necrosis factor. Proc Natl Acad Sci U S A 1998; 95(2):570-575.
(20) Grell M, Douni E, Wajant H, Lohden M, Clauss M, Maxeiner B et al. The transmembrane form of tumor necrosis factor is the prime activating ligand of the 80 kDa tumor necrosis factor receptor. Cell 1995; 83(5):793-802.
(21) Pocsik E, Duda E, Wallach D. Phosphorylation of the 26 kDa TNF precursor in monocytic cells and in transfected HeLa cells. J Inflamm 1995; 45(3):152-160.
(22) van Gestel AM, Prevoo ML, van't Hof MA, van Rijswijk MH, van de Putte LB, van Riel PL. Development and validation of the European League Against Rheumatism response criteria for rheumatoid arthritis. Comparison with the preliminary American College of Rheumatology and the World Health Organization/International League Against Rheumatism Criteria. Arthritis Rheum 1996; 39(1):34-40.
(23) Fransen J, van Riel PL. The Disease Activity Score and the EULAR response criteria. Clin Exp Rheumatol 2005; 23(5 Suppl 39):S93-S99.
(24) van Gestel AM, Haagsma CJ, van Riel PL. Validation of rheumatoid arthritis improvement criteria that include simplified joint counts. Arthritis Rheum 1998; 41(10):1845-1850.
(25) van der Pouw Kraan TC, van Baarsen LG, Rustenburg F, Baltus B, Fero M, Verweij CL. Gene expression profiling in rheumatology. Methods Mol Med 2007; 136:305-327.
(26) Demeter J, Beauheim C, Gollub J, Hernandez-Boussard T, Jin H, Maier D et al. The Stanford Microarray Database: implementation of new analysis tools and open source release of software. Nucleic Acids Res 2007; 35(Database issue):D766-D770.
(27) van Baarsen LG, van der Pouw Kraan TC, Kragt JJ, Baggen JM, Rustenburg F, Hooper T et al. A subtype of multiple sclerosis defined by an activated immune defense program. Genes Immun 2006; 7(6):522-531.
(28) Tusher VG, Tibshirani R, Chu G. Significance analysis of microarrays applied to the ionizing radiation response. Proc Natl Acad Sci U S A 2001; 98(9):5116-5121.
(29) Eisen MB, Spellman PT, Brown PO, Botstein D. Cluster analysis and display of genome-wide expression patterns. Proc Natl Acad Sci U S A 1998; 95(25):14863-14868.
(30) Thomas PD, Campbell MJ, Kejariwal A, Mi H, Karlak B, Daverman R et al. PANTHER: a library of protein families and subfamilies indexed by function. Genome Res 2003; 13(9):2129-2141.
(31) Thomas PD, Kejariwal A, Guo N, Mi H, Campbell MJ, Muruganujan A et al. Applications for protein sequence-function evolution data: mRNA/protein expression analysis and coding SNP scoring tools. Nucleic Acids Res 2006; 34(Web Server issue):W645-W650.
(32) Farr M, Scott DL, Constable TJ, Hawker RJ, Hawkins CF, Stuart J. Thrombocytosis of active rheumatoid disease. Ann Rheum Dis 1983; 42(5):545-549.
(33) Danhof M, de Lange EC, la Pasqua OE, Ploeger BA, Voskuyl RA. Mechanism-based pharmacokinetic-pharmacodynamic (PK-PD) modeling in translational drug research. Trends Pharmacol Sci 2008; 29(4):186-191.
(34) Workman P. How much gets there and what does it do?: The need for better pharmacokinetic and pharmacodynamic endpoints in contemporary drug discovery and development. Curr Pharm Des 2003; 9(11):891-902.
(35) Charles P, Elliott MJ, Davis D, Potter A, Kalden JR, Antoni C et al. Regulation of cytokines, cytokine inhibitors, and acute-phase proteins following anti-TNF-alpha therapy in rheumatoid arthritis. J Immunol 1999; 163(3):1521-1528.
(36) Feldmann M, Brennan FM, Maini RN. Role of cytokines in rheumatoid arthritis. Annu Rev Immunol 1996; 14:397-440.
(37) Smolen JS, Han C, Bala M, Maini RN, Kalden JR, van der HD et al. Evidence of radiographic benefit of treatment with infliximab plus methotrexate in rheumatoid arthritis patients who had no clinical improvement: a detailed subanalysis of data from the anti-tumor necrosis factor trial in rheumatoid arthritis with concomitant therapy study. Arthritis Rheum 2005; 52(4):1020-1030.
(38) Pincus T, Yazici Y, Yazici H, Kavanaugh AF, Kremer JM, Wolfe F. Radiographic benefit without clinical improvement in infliximab-treated patients with rheumatoid arthritis: comment on the article by Smolen et al. Arthritis Rheum 2005; 52(12):4044-4045.
(39) van den Berg WB, van Riel PL. Uncoupling of inflammation and destruction in rheumatoid arthritis: myth or reality? Arthritis Rheum 2005; 52(4):995-999.

## Claims

1. A method for prognosticating a clinical reponse of a patient to a treatment with a soluble TNF antagonist, TNF or a TNF receptor agonist, said method comprising determining the level of an IFN-I type response in at least two samples of said individual, wherein a first of said samples has not been exposed to a soluble TNF antagonist, TNF or a TNF receptor agonist and wherein at least a second of said samples has been exposed to said soluble TNF antagonist, TNF or TNF receptor agonist prior to determining said level, said method further comprising comparing said levels and prognosticating said clinical reponse from said comparison.

2. A method according to claim 1 wherein said IFN-I type response level is determined by determining in said at least two samples the level of an expression product of at least one gene of table 2.

3. A method according to any of claims 1-3, wherein one level of said IFN-I type response is determined by determining the level of an expression product of at least OAS1, LGALS3BP, RSAD2, IFI44L, MX2, OAS2 and/or SERPING1.

4. A method according to any of claims 1-3, wherein one level of said IFN-I type response is determined by determining the level of an expression product of at least OAS1 and LGALS3BP.

5. A method according to any of claims 1-4, wherein the level of said IFN-I type response is determined by determining the level of an expression product of at least OAS1, RSAD2 and IFI44L.

6. A method according to claim 4 or 5, wherein said IFN-I type response is determined by further determining the level of an expression product of at least MX2, OAS2 and/or SERPING1.

7. A method according to any of claims 1-6, wherein said IFN-I type response level is determined by determining the level of an expression product of at least the 15 validation genes listed in Table 2.

8. A method according to any of claims 1-7, wherein said IFN-I type response level is determined by determining the level of an expression product of at least the 34 genes listed in Table 2.

9. A method for prognosticating a clinical reponse of a patient to a treatment with a soluble TNF antagonist, TNF or a TNF receptor agonist, said method comprising determining the level of the expression products of the genes listed in Table 6 in at least two samples of said individual, wherein a first of said samples has not been exposed to a soluble TNF antagonist, TNF or a TNF receptor agonist and wherein at least a second of said samples has been exposed to said soluble TNF antagonist, TNF or TNF receptor agonist prior to determining said level, said method further comprising comparing said levels and prognosticating said clinical reponse from said comparison.

10. A method for prognosticating a clinical reponse of a patient to a treatment with a soluble TNF antagonist, TNF or a TNF receptor agonist, said method comprising determining the level of the expression products at least one gene of Table 6 in at least two samples of said individual, wherein a first of said samples has not been exposed to a soluble TNF antagonist, TNF or a TNF receptor agonist and wherein at least a second of said samples has been exposed to said soluble TNF antagonist, TNF or TNF receptor agonist prior to determining said level, said method further comprising comparing said levels and prognosticating said clinical reponse from said comparison.

11. A method according to anyone of claims 1-10, wherein said at least two samples comprise cell samples.

12. A method according to any of claims 1-11, wherein said second sample is of an individual between 1 and 4 months after the first exposure of said individual to said soluble TNF antagonist, TNF or TNF receptor agonist.

13. A method to any of claims 1-12, wherein said second sample has been exposed in vitro to said soluble TNF antagonist, TNF or TNF receptor agonist.

14. A method for treatment of an individual suffering from or at risk of suffering from a TNF related disease with a soluble TNF antagonist, TNF or a TNF receptor agonist, comprising determining a prognosis for a clinical reponse to a treatment with said soluble TNF antagonist, TNF or TNF receptor agonist according to any one of claims 1-13 further comprising treating said individual with said soluble TNF antagonist, TNF or TNF receptor agonist, if said individual has been prognosticated as a good responder.

15. Use of a soluble TNF antagonist, TNF or a TNF receptor agonist for the preparation of a medicament for the treatment of an individual suffering from or at risk of suffering from a TNF related disease, wherein prior to said treatment a prognosis for a clinical reponse to said soluble TNF antagonist, TNF or TNF receptor agonist was determined for said individual with a method according to any one of claims 1-13.

16. A method for evaluating a pharmacological effect of a treatment of a patient with a soluble TNF antagonist, TNF or a TNF receptor agonist said method comprising determining the level of an expression product of at least one gene of Table S2 in at least two samples of said individual, wherein a first of said samples has not been exposed to a soluble TNF antagonist, TNF or a TNF receptor agonist and wherein at least a second of said samples has been exposed to said soluble TNF antagonist, TNF or TNF receptor agonist prior to determining said level.

17. A method according to claim 17, wherein said at least one gene comprises SKALP.

18. A method for treatment according to claim 14, wherein said treatment is based on a method according to claim 17 or 18.

19. A kit suitable for use in the method according to any of claims 1-8, comprising up to 34 reagents, sequence specific oligonucleotides and/or capture agents for detecting up to 34 of the gene products of the genes listed in Table 2.

20. A kit suitable for use in the method according to claims 9 or 10, comprising up to 21 reagents, sequence specific oligonucleotides and/or capture agents for detecting up to 21 of the gene products of the genes listed in Table 6.
